(19) Europäisches Patentamt / European Patent Office / Office européen des brevets

(11) **EP 4 001 431 A1**

(12) **EUROPEAN PATENT APPLICATION**
published in accordance with Art. 153(4) EPC

(43) Date of publication:
**25.05.2022  Bulletin 2022/21**

(21) Application number: **20843654.3**

(22) Date of filing: **16.07.2020**

(51) International Patent Classification (IPC):
***C12Q 1/6837*** (2018.01)     ***C12Q 1/686*** (2018.01)
***C12Q 1/6876*** (2018.01)

(52) Cooperative Patent Classification (CPC):
**C12Q 1/6837; C12Q 1/686; C12Q 1/6876**

(86) International application number:
**PCT/JP2020/027649**

(87) International publication number:
**WO 2021/015084 (28.01.2021 Gazette 2021/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **19.07.2019   JP 2019134181**

(71) Applicant: **Public University Corporation Fukushima Medical University**
**Fukushima City Fukushima, 960-1295 (JP)**

(72) Inventors:
• **WATANABE, Shinya**
  **Fukushima City Fukushima 9601295 (JP)**
• **IMAI, Junichi**
  **Fukushima City Fukushima 9601295 (JP)**
• **ITO, Emi**
  **Fukushima City Fukushima 9601295 (JP)**
• **OTAKE, Toru**
  **Fukushima City Fukushima 9601295 (JP)**
• **ABE, Noriko**
  **Fukushima City Fukushima 9601295 (JP)**
• **TACHIBANA, Kazunoshin**
  **Fukushima City Fukushima 9601295 (JP)**

(74) Representative: **Betten & Resch Patent- und Rechtsanwälte PartGmbB Maximiliansplatz 14 80333 München (DE)**

Remarks:
The complete document including Reference Tables and the Sequence Listing can be downloaded from the EPO website

(54) **DISTINGUISHING MARKER GENE SET, METHOD AND KIT EACH FOR DISTINGUISHING OR CLASSIFYING SUBTYPE OF BREAST CANCER**

(57)  To provide a differentiation marker gene set which, in the differentiation or classification of a breast cancer subtype, can perform differentiation or classification with high reproducibility by gene expression analysis.

The above-described problem is solved by a differentiation marker gene set for differentiating or classifying a subtype of breast cancer. The differentiation marker gene set comprises a combination of genes obtained by selecting at least one gene from each gene group of at least one gene group selected from gene groups composed of groups a to o, the at least one gene group being selected in accordance with a desired subtype to be differentiated or classified.

**EP 4 001 431 A1**

**Description**

Field of the Invention

The present invention relates to a gene marker set, a method, and a kit for differentiating or classifying a subtype of breast cancer.

Description of the Background Art

Breast cancer is not a homogeneous disease and is classified into a plurality of subtypes having various characteristics. A precursor to this subtype classification is "intrinsic subtype" reported by Perou et al. in 2000 (Non-Patent Document 1). The authors selected genes having greater variation in expression before and after doxorubicin treatment of 20 breast cancer tissues and between two different tumors in primary lesions and metastatic lymph nodes, and created an intrinsic gene set of 496 genes. Cluster analysis of 65 cases of breast cancer was conducted using this intrinsic gene set of 496 genes, and the subtypes were classified into ER+/luminal-like with high expressions of ESR1, GATA3, and other luminal genes, basal-like with a high expression of the polymeric cytokeratin (5/6/17) gene, and HER2-enriched with a high expression of the ERBB2 gene.

In 2001, the same group increased the number of target cases to 85 and examined and subclassified the cases into luminal A/B/C, ERBB2, basal-like, and normal breast-like on the basis of biological properties (Non-Patent Document 2). With prognosis and drug sensitivity differing depending on the type, this classification has the advantage of potentially being an indicator of drug therapy selection. Thus, this classification has become a prototype of the subtype classification currently used in clinical practice.

For the "intrinsic subtype," an alternative method using an immunohistochemical technique has been developed to facilitate use in clinical practice. At the 2011 St. Gallen Consensus Conference, the following alternative intrinsic subtype classifications based on ER/PgR/HER2/Ki67 information obtained by general pathological examinations mainly based on immunohistochemical methods were adopted, and basically passed down through the same conference in 2013 and 2015.

　　　(1) Luminal A-like: Produced from lumen (luminal epithelium), is estrogen receptor (ER) positive, and has a high degree of differentiation. Hormone therapy is effective, resulting in a high possibility that chemotherapy is unnecessary.
　　　(2) Luminal B-like: ER positive and seemingly quiet at first, but has a high Ki-67 proliferation marker, includes cases of HER-2 positive, and has high malignancy.
　　　(3) HER-2-enriched: ER negative and HER-2 positive; humanized anti-HER2 monoclonal antibodies and trastuzumab (Herceptin) are highly effective.
　　　(4) Basal-like: A triple negative breast cancer that is ER negative, HER-2 negative, progesterone receptor (PgR) negative, and has a high histological grade; chemotherapy is effective.

If the alternative classifications are used, the convenience of being able to imagine the ER/PgR/HER2/Ki67 state and even the treatment policy has an effect, and alternative classification names such as 'luminal A-like' are very commonly heard in routine medical care, even in Japan. However, essentially the intrinsic subtype is just a classification based on gene expression analysis and does not necessarily match the alternative classification. Further, although both Ki67 and PgR are considered necessary for alternative classification, setting cutoff values thereof at each facility is recommended and, for Ki67, no firm standard has been globally set for the evaluation method itself. Under such circumstances, there are many scenarios in which it is difficult to apply an alternative definition in clinical practice. Although alternative classification names are becoming more common in routine medical care, the medical care actually carried out is basically in consideration of the ER/PgR/HER2 (and in some cases Ki67) state and the risk based on conventional clinicopathological information, and thus there is essentially no significant change from before the introduction of the intrinsic subtype. Further, the intrinsic subtype itself by gene expression analysis is not at the stage that allows use in clinical practice from the standpoint of reproducibility and the like. From these perspectives, when using an intrinsic subtype alternative definition, it is necessary to keep in mind that the definition is convenient and conceptual and, furthermore, to pay close attention to whether this alternative definition will continue to be used in the future.

PRIOR ART DOCUMENTSNon-Patent Documents[0006]

[0006]

　　Non-Patent Document 1: Perou CM et al., Nature 406: 747-752, 2000

Non-Patent Document 2: Sorlie T et al., Proc Natl Acad Sci 98 (19), 10869-74, 2001

SUMMARY OF THE INVENTION

Problems to be Solved by the Invention[0007]

The problem of the present invention is to provide a differentiation marker gene set which, in the differentiation or classification of a breast cancer subtype, can perform differentiation or classification with high reproducibility by gene expression analysis, and a method of differentiating or classifying a breast cancer subtype that uses the gene set.

Means for Solving the Problems

In order to solve the above-described problems, the present inventors acquired the gene expression profiles of 14,400 genes from each specimen of 470 cases including breast cancer tissue (453 cases) and normal mammary gland tissue (17 cases), and succeeded in identifying genes that exhibit characteristic behavior of each breast cancer subtype and can differentiate or classify the breast cancer subtype, leading to the completion of the present invention.

That is, the present invention includes the following aspects.

The present invention, according to one aspect, relates to

[1] a method of differentiating or classifying a subtype of breast cancer in a test sample, the method comprising:

(a) a step of measuring, in the test sample, expression levels of genes included in a differentiation marker gene set for differentiating or classifying a subtype of breast cancer; and

(b) a step of differentiating or classifying whether the test sample is a desired subtype to be differentiated or classified from the expression levels of the genes included in the differentiation marker gene set thus measured,

the differentiation marker gene set including a combination of genes obtained by selecting at least one gene from each gene group of at least one gene group selected from gene groups composed of groups a to o shown in the tables below, and

the at least one gene group being selected in accordance with the desired subtype to be differentiated or classified.

[Table 1A]

| Gene group | Gene symbol | Gene group | Gene symbol | Gene group | Gene symbol |
|---|---|---|---|---|---|
| Group a | KRTDAP | Group e | KRT14 | Group g | ART3 |
| | SERPINB3 | | DST | | EN1 |
| | SPRR2A | | WIF1 | | SPHK1 |
| | SPRR1B | | SYNM | | TRIM47 |
| | KLK13 | | KIT | | COL27A1 |
| | KRT1 | Group f | GABRP | | RFLNA |
| | LGALS7 | | SFRP1 | | RASD2 |
| | PI3 | | ELF5 | | A2ML1 |
| Group b | SERPINH1 | | MIA | | MARCO |
| | SNAI2 | | MMP7 | | TSPYL5 |
| | GPR173 | | FDCSP | | TM4SF1 |
| | HAS2 | Group g | CRABP1 | | FABP5 |
| | PTH1R | | PROM1 | Group h | SPIB |
| | PAGE5 | | KRT23 | | BCL2A1 |
| | ITLN1 | | S100A1 | | MZB1 |
| | SH3PXD2B | | WIPF3 | | KCNK5 |
| Group c | TAP1 | | CYYR1 | | LM04 |
| | FN1 | | TFCP2L1 | | RNF150 |
| | CTHRC1 | | DSC2 | | LYZ |
| | MMP9 | | MFGE8 | Group i | C21orf58 |
| Group d | ADIPOQ | | KLK7 | | ATP13A5 |
| | CD36 | | KLK5 | | NUDT8 |
| | G0S2 | | DSG3 | | HSD17B2 |
| | GPD1 | | TTYH1 | | ABCA12 |
| | LEP | | SCRG1 | | ENPP3 |
| | LIPE | | S100B | | WNT5A |
| | PLIN1 | | ETV6 | | MPP3 |
| | CAVIN2 | | OGFRL1 | | VPS13D |
| | LIFR | | MELTF | | PXMP4 |
| | TGFBR3 | | HORMAD1 | | GGT1 |
| Group e | CAPN6 | | PKP1 | | TRPV6 |
| | PIGR | | FOXC1 | | MAB21L4 |
| | KRT15 | | ITGB8 | | CLDN8 |
| | KRT5 | | VGLL1 | | LBP |

[Table 1B]

| Gene group | Gene symbol | Gene group | Gene symbol | Gene group | Gene symbol |
|---|---|---|---|---|---|
| Group i | SRD5A3 | Group l | THSD4 | Group n | GATA3 |
| | PAPSS2 | | MAPT | | ZG16B |
| | TMEM45B | | L0NRF2 | | KIAA0040 |
| | CLCA2 | | TCEAL3 | | TMC4 |
| | FASN | | DBNDD2 | | AGR2 |
| | MPHOSPH6 | | FGD3 | | TFF3 |
| | NXPH4 | | GFRA1 | | SCGB2A2 |
| | HPGD | | PARD6B | | MUCL1 |
| | KYNU | | STC2 | Group o | DDX11 |
| | GLYATL2 | | SLC39A6 | | ATAD2 |
| | KMO | | ENPP5 | | GGH |
| | SRPK3 | | ZNF703 | | CDCA3 |
| | THRSP | | EVL | | CCNA2 |
| | PLA2G2A | | TBC1D9 | | CCNB2 |
| | TFAP2B | | CHAD | | ANLN |
| | FABP7 | | GREB1 | | UBE2C |
| | SLPI | | HPN | | CKS2 |
| | SERHL2 | | IL6ST | | MKI67 |
| | S100A9 | | GASK1B | | F0XM1 |
| | KRT7 | | CA12 | | UBE2T |
| | TMEM86A | | KCNE4 | | MCM4 |
| | MB0AT1 | | NAT1 | | CKAP2 |
| Group j | PGAP3 | | CYP2B6 (CYP2B7P) | | JPT1 |
| | STARD3 | | ARMT1 | | KPNA2 |
| | ERBB2 | | MAGED2 | | H2AFX |
| | MIEN1 | | CELSR1 | | H2AFZ |
| | GRB7 | | INPP5J | | CDK1 |
| Group k | GSDMB | | PADI2 | | PTTG 1 |
| | 0RMDL3 | | PPP1R1B | | CDC20 |
| | MED24 | Group m | ESR1 | | MYBL2 |
| | MSL1 | Group n | MLPH | | RRM2 |
| | CASC3 | | F0XA1 | | |
| | WIPF2 | | XBP1 | | |

Here, the method of differentiating or classifying a subtype of breast cancer in a test sample of the present invention is, in one embodiment,

[2] the method of differentiating or classifying according to [1] described above, the step (b) being a step of differentiating or classifying a subtype of the test sample by acquiring an expression profile of the differentiation marker gene set from the expression levels of the genes thus measured, and comparing the expression profile thus acquired and an expression profile of a corresponding differentiation marker gene set in a sample derived from a breast cancer patient having the desired subtype to be differentiated or classified.

Further, the method of differentiating or classifying a subtype of breast cancer in a test sample of the present invention is, in one embodiment,

[3] the method of differentiating or classifying according to [2] described above,

in the step (b), the expression profile thus acquired and the expression profile of a corresponding differentiation marker gene set in the sample derived from a breast cancer patient having the desired subtype to be differentiated or classified being compared, and

the test sample being evaluated as being breast cancer of the subtype thus compared when having an expression profile equivalent to the expression profile of the sample thus compared, or being evaluated as not being breast cancer of the subtype thus compared when having an expression profile of genes different from the expression profile of the sample thus compared.

Further, the method of differentiating or classifying a subtype of breast cancer in a test sample of the present invention is, in one embodiment,

[4] the method of differentiating or classifying according to [2] described above, comparison with the expression profile of the corresponding differentiation marker gene set in a sample derived from a breast cancer patient having the desired subtype to be differentiated or classified in the step (b) being performed by cluster analysis.

Further, the method of differentiating or classifying a subtype of breast cancer in a test sample of the present invention is, in one embodiment,

[5] the method of differentiating or classifying according to [2] described above, in the step (b), the differentiating or classifying being performed by comparing the expression profile thus acquired with a predetermined threshold value.

Further, the method of differentiating or classifying a subtype of breast cancer in a test sample of the present invention is, in one embodiment,

[6] the method of differentiating or classifying according to [2] described above, the step (b) being a step of differentiating or classifying whether the test sample is the desired subtype to be differentiated by calculating a subtype differentiation score from the expression levels of the genes included in the gene set thus measured.

Further, the method of differentiating or classifying a subtype of breast cancer in a test sample of the present invention is, in one embodiment,

[7] the method of differentiating or classifying according to [6] described above,

the subtype differentiation score in the step (b) being determined on the basis of the expression levels of genes included in each gene group selected in accordance with the desired subtype to be differentiated, or an average value thereof.

Further, the method of differentiating or classifying a subtype of breast cancer in a test sample of the present invention is, in one embodiment,

[8] the method of differentiating or classifying according to any one of [1] to [7] described above,

the desired subtype being a subtype selected from a group composed of luminal A, luminal B (HER2 positive), luminal B (HER2 negative), HER2 positive, HER2 positive-like, triple negative, phyllodes tumor, squamous cell carcinoma, normal-like, normal, and undeterminable.

Further, the method of differentiating or classifying a subtype of breast cancer in a test sample of the present invention is, in one embodiment,

[9] the method of differentiating or classifying according to any one of [1] to [8] described above,

the at least one gene group selected in accordance with the desired subtype to be differentiated in the step (a) being

(i) the group 1 and the group m for calculating a hormone sensitivity score, the group o for calculating a cell cycle score, and the group j and the group k for calculating a HER2 amplification score when the desired subtype is luminal A,
(ii) the group j and the group k for calculating the HER2 amplification score, and the group 1 and the group m for calculating the hormone sensitivity score when the desired subtype is luminal B (HER2 positive),
(iii) the group j and the group k for calculating the HER2 amplification score, the group 1 and the group m for calculating the hormone sensitivity score, and the group o for calculating the cell cycle score when the desired subtype is luminal B (HER2 negative),
(iv) the group j and the group k for calculating the HER2 amplification score, the group 1 and the group m for calculating the hormone sensitivity score, or the group i for calculating a HER2-like score when the desired subtype is HER2 positive,
(v) the group i for calculating the HER2-like score, and the group j and the group k for calculating the HER2 amplification score when the desired subtype is HER2 positive-like,
(vi) the group f, the group g, the group h, and the group n for calculating a triple negative score when the desired subtype is triple negative,
(vii) the group b for calculating a phyllodes tumor score when the desired subtype is phyllodes tumor,
(viii) the group a for calculating a squamous cell score when the desired subtype is squamous cell carcinoma,
(ix) the group a to the group o for calculating a cancer score and all other scores when the desired subtype is undeterminable,
(x) the group e for calculating a normal-like score, the group o for calculating the cell cycle score, and the group c and the group d for calculating the cancer score when the desired subtype is normal-like, or
(xi) the group c and the group d for calculating the cancer score when the desired subtype is normal.

Further, the method of differentiating or classifying a subtype of breast cancer in a test sample of the present invention is, in one embodiment,

[10] the method of differentiating or classifying according to any one of [1] to [9] described above,

the desired subtypes being luminal A and B, HER2 positive-like, HER2 positive, and triple negative, and
the differentiation marker gene set including a combination of genes obtained by selecting at least one gene from each gene group of the group f, the group g, the group i, the group j, the group k, the group 1, the group m, the group n, and the group o.

Further, the method of differentiating or classifying a subtype of breast cancer in a test sample of the present invention is, in one embodiment,

[11] the method of differentiating or classifying according to any one of [1] to [10] described above, the differentiation marker gene set including all genes included in each gene group of a plurality of the gene groups thus selected.

Further, the method of differentiating or classifying a subtype of breast cancer in a test sample of the present invention is, in one embodiment,

[12] the method of differentiating or classifying according to any one of [1] to [11] described above, the differentiation marker gene set further including at least one gene selected from a control group composed of ABCF3, FBXW5, MLLT1, FAM234A, PITPNM1, WDR1, NDUFS7, and AP2A1.

Further, the present invention, according to another aspect, relates to

[13] a differentiation marker gene set for differentiating or classifying a subtype of breast cancer, the differentiation marker gene set comprising:

a combination of genes obtained by selecting at least one gene from each gene group of at least one gene group selected from gene groups composed of groups a to o shown in the tables below,
the at least one gene group being selected in accordance with a desired subtype to be differentiated or classified.

[Table 2A]

| Gene group | Gene symbol | Gene group | Gene symbol | Gene group | Gene symbol |
|---|---|---|---|---|---|
| Group a | KRTDAP | Group e | KRT14 | Group g | ART3 |
| | SERPINB3 | | DST | | EN1 |
| | SPRR2A | | WIF1 | | SPHK1 |
| | SPRR1B | | SYNM | | TRIM47 |
| | KLK13 | | KIT | | COL27A1 |
| | KRT1 | Group f | GABRP | | RFLNA |
| | LGALS7 | | SFRP1 | | RASD2 |
| | PI3 | | ELF5 | | A2ML1 |
| Group b | SERPINH1 | | MIA | | MARCO |
| | SNAI2 | | MMP7 | | TSPYL5 |
| | GPR173 | | FDCSP | | TM4SF1 |
| | HAS2 | | CRABP1 | | FABP5 |
| | PTH1R | | PR0M1 | Group h | SPIB |
| | PAGE5 | | KRT23 | | BGL2A1 |
| | ITLN1 | | S100A1 | | MZB1 |
| | SH3PXD2B | | WIPF3 | | KCNK5 |
| Group c | TAP1 | | CYYR1 | | LM04 |
| | FN1 | | TFCP2L1 | | RNF150 |
| | CTHRC1 | | DSC2 | | LYZ |
| | MMP9 | | MFGE8 | Group i | C21orf58 |
| Group d | ADIPOQ | Group g | KLK7 | | ATP13A5 |
| | CD36 | | KLK5 | | NUDT8 |
| | GOS2 | | DSG3 | | HSD17B2 |
| | GPD1 | | TTYH1 | | ABCA12 |
| | LEP | | SCRG1 | | ENPP3 |
| | LIPE | | S100B | | WNT5A |
| | PLIN1 | | ETV6 | | MPP3 |
| | CAVIN2 | | 0GFRL1 | | VPS13D |
| | LIFR | | MELTF | | PXMP4 |
| | TGFBR3 | | HORMAD1 | | GGT1 |
| Group e | CAPN6 | | PKP1 | | TRPV6 |
| | PIGR | | FOXC1 | | MAB21L4 |
| | KRT15 | | ITGB8 | | CLDN8 |
| | KRT5 | | VGLL1 | | LBP |

EP 4 001 431 A1

[Table 2B]

| Gene group | Gene symbol | Gene group | Gene symbol | Gene group | Gene symbol |
|---|---|---|---|---|---|
| Group i | SRD5A3 | | THSD4 | Group n | ZG16B |
| | PAPSS2 | | MAPT | | KIAA0040 |
| | TMEM45B | | L0NRF2 | | TMC4 |
| | CLCA2 | | TCEAL3 | | AGR2 |
| | FASN | | DBNDD2 | | TFF3 |
| | MPH0SPH6 | | FGD3 | | SCGB2A2 |
| | NXPH4 | | GFRA1 | | MUCL1 |
| | HPGD | | PARD6B | | |
| | KYNU | Group l | STC2 | Group o | DDX11 |
| | GLYATL2 | | SLC39A6 | | ATAD2 |
| | KMO | | ENPP5 | | GGH |
| | SRPK3 | | ZNF703 | | CDCA3 |
| | THRSP | | EVL | | CCNA2 |
| | PLA2G2A | | TBC1D9 | | CCNB2 |
| | TFAP2B | | CHAD | | ANLN |
| | FABP7 | | GREB1 | | UBE2C |
| | SLPI | | HPN | | CKS2 |
| | SERHL2 | | IL6ST | | MKI67 |
| | S100A9 | | GASK1B | | FOXM1 |
| | KRT7 | | CA12 | | UBE2T |
| | TMEM86A | | KCNE4 | | MCM4 |
| | MBOAT1 | | NAT1 | | CKAP2 |
| Group j | PGAP3 | | CYP2B6 (CYP2B7P) | | JPT1 |
| | STARD3 | | ARMT 1 | | KPNA2 |
| | ERBB2 | | MAGED2 | | H2AFX |
| | MIEN1 | | CELSR1 | | H2AFZ |
| | GRB7 | | INPP5J | | CDK1 |
| Group k | GSDMB | | PADI2 | | PTTG1 |
| | ORMDL3 | | PPP1R1B | | CDC20 |
| | MED24 | Group m | ESR1 | | MYBL2 |
| | MSL1 | Group n | MLPH | | RRM2 |
| | CASC3 | | FOXA1 | | |
| | WIPF2 | | XBP1 | | |

Here, the differentiation marker gene set of the present invention is, in one embodiment,

[14] the differentiation marker gene set according to [13] described above,

the desired subtype being a subtype selected from a group composed of luminal A, luminal B (HER2 positive), luminal B (HER2 negative), HER2 positive, HER2 positive-like, triple negative, phyllodes tumor, squamous cell carcinoma, normal-like, normal, and undeterminable.

Further, the differentiation marker gene set of the present invention is, in one embodiment,

[15] the differentiation marker gene set according to [13] or [14] described above,

the at least one gene group being

(i) the group 1 and the group m for calculating a hormone sensitivity score, the group o for calculating a cell cycle score, and the group j and the group k for calculating a HER2 amplification score when the desired subtype is luminal A,

(ii) the group j and the group k for calculating the HER2 amplification score, and the group 1 and the group m for calculating the hormone sensitivity score when the desired subtype is luminal B (HER2 positive),

(iii) the group j and the group k for calculating the HER2 amplification score, the group 1 and the group m for calculating the hormone sensitivity score, and the group o for calculating the cell cycle score when the desired subtype is luminal B (HER2 negative),

(iv) the group j and the group k for calculating the HER2 amplification score, the group 1 and the group m for calculating the hormone sensitivity score, or the group i for calculating a HER2-like score when the desired subtype is HER2 positive,

(v) the group i for calculating a HER2-like score, and the group j and the group k for calculating the HER2 amplification score when the desired subtype is HER2 positive-like,

(vi) the group f, the group g, the group h, and the group n for calculating a triple negative score when the desired subtype is triple negative,

(vii) the group b for calculating a phyllodes tumor score when the desired subtype is phyllodes tumor,

(viii) the group a for calculating a squamous cell score when the desired subtype is squamous cell carcinoma,

(ix) the group a to the group o for calculating a cancer score and all other scores when the desired subtype is undeterminable,

(x) the group e for calculating a normal-like score, the group o for calculating the cell cycle score, and the group c and the group d for calculating the cancer score when the desired subtype is normal-like, or

(xi) the group c and the group d for calculating the cancer score when the desired subtype is normal.

Further, the differentiation marker gene set of the present invention, in one embodiment, relates to

[16] the differentiation marker gene set according to any one of [13] to [15] described above, the differentiation marker gene set comprising:

a combination of genes obtained by selecting at least one gene from each gene group of nine gene groups composed of the group f, the group g, the group i, the group j, the group k, the group 1, the group m, the group n, and the group o.

Further, the differentiation marker gene set of the present invention is, in one embodiment,

[17] the differentiation marker gene set according to any one of [13] to [15] described above, the differentiation marker gene set comprising:

a combination of genes obtained by selecting at least one gene from each gene group of 15 gene groups composed of the groups a to o.

Further, the differentiation marker gene set of the present invention is, in one embodiment,

[18] the differentiation marker gene set according to any one of [13] to [17] described above, the differentiation marker gene set further comprising:

at least one gene selected from a control group composed of ABCF3, FBXW5, MLLT1, FAM234A, PITPNM1, WDR1, NDUFS7, and AP2A1.

Further, the present invention, according to another aspect, relates to

[19] a kit for differentiating or classifying a subtype of breast cancer in a test sample, the kit comprising:

means for measuring expression levels of genes included in the differentiation marker gene set for differentiating or classifying a subtype of breast cancer according to any one of [13] to [18] described above.

Here, the kit of the present invention is, in one embodiment,

[20] the kit according to [19] described above,

the means for measuring expression levels of genes being at least one means selected from a group composed of a primer or a probe for the genes or markers thereof.

Further, the kit of the present invention is, in one embodiment,

[21] the kit according to [20] described above,

the kit being for a PCR, a microarray, or an RNA sequence.

Effect of the Invention

According to a differentiation marker gene set of the present invention, it is possible to differentiate or classify a subtype of breast cancer with high reproducibility by expression analysis of genes included in the differentiation marker gene set.

BRIEF DESCRIPTION OF THE DRAWINGS

Fig. 1 shows a heat map of results of cluster analysis by a group-average method based on Euclidean distance for 470 cases by using a differentiation marker gene set of 207 kinds of genes indicated in Example 4 below.
Fig. 2 shows a heat map of scoring results of subtype differentiation scores for 470 cases by using a differentiation marker gene set of 207 kinds of genes indicated in Example 5 below.

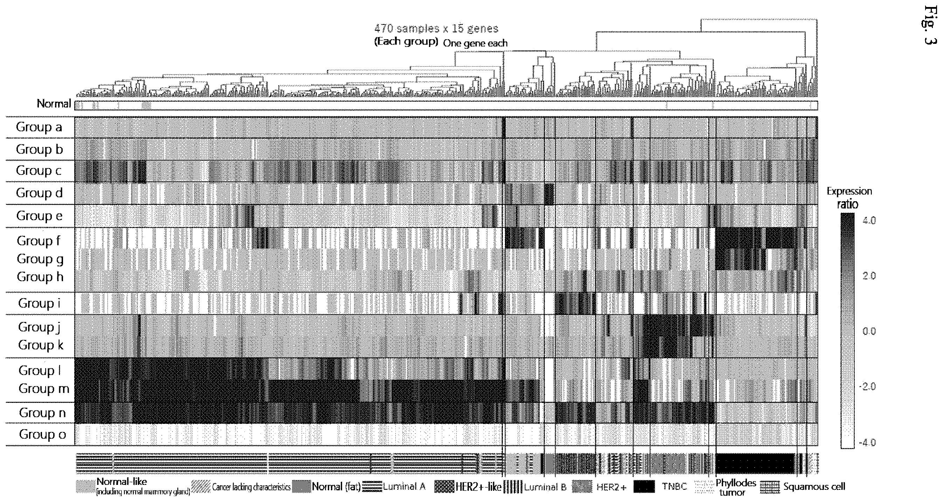

Fig. 3 shows a heat map of results of cluster analysis by a group-average method based on Euclidean distance for 470 cases by using a differentiation marker gene set of 15 kinds of genes indicated in Example 7 below.

Fig. 4 shows a heat map of scoring results of subtype differentiation scores for 470 cases by using a differentiation marker gene set of 15 kinds of genes indicated in Example 8 below. It should be noted that the heat map in the upper section of Fig. 4 shows the heat map of Fig. 2 as a comparison.

Fig. 5 shows a heat map of results of cluster analysis by a group-average method based on Euclidean distance for 470 cases by using a differentiation marker gene set of 161 kinds of genes indicated in Example 9 below.

Fig. 6 shows a heat map of scoring results of subtype differentiation scores for 470 cases by using a differentiation marker gene set of 161 kinds of genes indicated in Example 10 below.

## DESCRIPTION OF THE PREFERRED EMBODIMENTS

1. Differentiation Marker Gene Set for Differentiating or Classifying Subtype of Breast Cancer

1-1. Overview

A first aspect of the present invention is a differentiation marker gene set capable of distinguishing a subtype (histological type) of breast cancer. The differentiation marker gene set of the present invention is constituted by genes selected from a gene group of at least 199 kinds of genes, and makes it possible to classify breast cancer into one of the histological types of luminal A, luminal B (HER2 positive), luminal B (HER2 negative), HER2 positive, HER2 positive-like, triple negative, normal-like, normal, squamous cell carcinoma, phyllodes tumor, and undeterminable by measurement of expression levels of specific genes in the gene group in a sample of a subject.

1-2. Definitions

The term "breast cancer" refers to cancer that usually begins in intraductal tissue such as ducts and lobules. Further, breast cancer refers to any malignant tumor of breast tissue, including carcinoma and sarcoma. Furthermore, breast cancer is a heterogeneous disease and is classified into a plurality of subtypes having various characteristics.

The subtypes of breast cancer are mainly classified into the 11 types of luminal A, luminal B (HER2 positive), luminal B (HER2 negative), HER2 positive, HER2 positive-like, triple negative, phyllodes tumor, squamous cell carcinoma, normal-like, normal, and undeterminable. In one embodiment, luminal A, luminal B (HER2 positive), and luminal B (HER2 negative) can also be classified into the subtype "luminal A + B" as a group.

Here, the term "luminal A" refers to a case that clinicopathologically satisfies all of 1) ER positive and PgR negative, 2) HER2 negative, 3) low Ki67 value, and 4) low recurrence risk in MGEA, but in this specification also includes cases in which the gene expression profile is clinicopathologically similar to those of most cases diagnosed as luminal A.

It should be noted that diagnosis of the clinicopathological subtype is one that mainly confirms the expression of ER, PgR, HER2, and Ki67 by immunohistochemical staining, but is not limited thereto and includes confirmation by gene expression analysis.

The term "luminal B (HER2 positive)" refers to a case that clinicopathologically is ER positive and HER2 positive, but in this specification also includes cases in which the gene expression profile is clinicopathologically similar to those of most cases diagnosed as luminal B (HER2 positive).

The term "luminal B (HER2 negative)" refers to a case that clinicopathologically falls under any of 1) ER positive and HER2 negative, 2) high Ki67 value, 3) negative or low PgR, and 4) high recurrence risk in MGEA, but in this specification also includes cases in which the cell cycle-related gene group is more highly expressed than in other cases in luminal A.

The term "HER2 positive" refers to a case that clinicopathologically is HER2 positive, ER negative, and PgR negative, but in this specification also includes cases in which the gene expression profile is clinicopathologically similar to those of most cases diagnosed as HER2 positive.

The term "HER2 positive-like" refers to a case in which HER2 is negative, but other gene expression profiles are similar to most of the cases clinicopathologically diagnosed as HER2 positive.

The term "triple negative" refers to case that clinicopathologically is ER negative, PgR negative, and HER2 negative, but in this specification also includes cases in which the gene expression profile is clinicopathologically similar to most of the cases diagnosed as triple negative.

The term "squamous cell carcinoma" refers to a cancer produced by the malignant proliferation of cells called epidermal keratinocytes existing in the epidermis, and in this specification refers to cancer originating from the mammary gland.

The term "phyllodes tumor" refers to a tumor that is clinicopathologically similar to fibroadenoma of the mammary gland, but is produced by rapid growth of fibrous stroma and ductal epithelium in contrast to fibroadenoma in which connective tissue within the lobules of the mammary gland proliferates.

The term "undeterminable" refers to a case in which the gene expression profile is not similar to any of those of luminal A, luminal B (HER2 positive), luminal B (HER2 negative), HER2 positive, HER2 positive-like, triple negative, normal-like, normal, squamous cell carcinoma, and phyllodes tumor.

The term "normal-like" refers to a case that is clinicopathologically diagnosed as "cancer," but has a gene expression profile similar to that of normal mammary gland tissue.

The term "normal" refers to normal tissue.

In this specification, the term "differentiation marker gene" is a marker related with a gene included in the "differentiating gene group" and refers to a biomarker capable of differentiating the histological type of breast cancer. In the present invention, the biomarker is particularly a transcription product (mRNA) of each gene included in the differentiating gene group, and is a protein in which the cDNA or each gene is encoded.

In this specification, "gene expression score" is a score determined by the expression level of each gene or a plurality of genes included in the "differentiating gene group." The type and calculation method of the score are not particularly limited, and examples include a score ($-1 \leq n \leq 1$) determined by setting a cutoff value of the expression level for each gene and conducting a comparison with the cutoff values.

In this specification, the "expression level of a gene" means a transcription product amount, an expression intensity, or an expression frequency of a differentiating gene. The expression level of a gene referred to herein is not limited to the expression level of a wild-type gene of the differentiating gene, and may include the expression level of a mutant gene such as a point-mutant gene. Further, the transcription product showing the expression of the differentiating gene may include atypical transcription products (variants) such as splicing variants and fragments thereof. This is because the expression profile of a gene in the present invention can be constructed even with information based on a mutant gene, a transcription product, or a fragment thereof. The expression level of a gene can be obtained as a measured value by measuring the amount of transcription product, that is, mRNA, of the gene groups constituting the differentiation marker gene set, or the like. It should be noted that, in a preferred embodiment, the measurement of the expression level of a gene is the measurement of mRNA.

Further, in this specification, the term "expression profile" refers to information regarding the expression level of each gene, and particularly refers to information regarding the expression levels of a plurality of genes. Further, the expression profile includes a "subtype differentiation score" and a "gene expression score" determined by the expression level of the differentiation marker.

In this specification, the "measured value" is a value obtained by a measurement method of the gene expression level. The measured value may be an absolute value in which the amount of mRNA or the like in the sample is expressed by weight such as ng (nanogram) or μg (microgram), or may be a relative value expressed by absorbance with respect to a control value, a fluorescence intensity of a labeled molecule, or the like.

It should be noted that the measured value of the expression level of each gene depends on the measurement method, but can be calculated as a relative ratio (expression ratio) to a common sample (hereinafter referred to as "common reference"), for example. The common reference when calculating the expression ratio may be any reference as long as the same in the measurement conditions across the samples to be compared. For example, the common reference may be a specific cell line or a mixture of a plurality of cell lines. Alternatively, a commercially available universal reference, a known housekeeping gene, or a combination thereof can be used as the common reference.

In this specification, the "differentiating or classifying" means differentiating or classifying, for a sample derived from a subject who has a history of breast cancer, the subtype to which the breast cancer belongs, or differentiating or classifying the histological type to which there is a high or low possibility of the cancer belonging.

1-3. Configuration

In this specification, "differentiating gene group" is composed of the ABCF3 gene, FBXW5 gene, MLLT1 gene, FAM234A gene, PITPNM1 gene, WDR1 gene, NDUFS7 gene, AP2A1 gene, KRTDAP gene, SERPINB3 gene, SPRR2A gene, SPRR1B gene, KLK13 gene, KRT1 gene, LGALS7 gene, PI3 gene, SERPINH1 gene, SNAI2 gene, GPR173 gene, HAS2 gene, PTH1R gene, PAGE5 gene, ITLN1 gene, SH3PXD2B gene, TAP1 gene, FN1 gene, CTHRC1 gene, MMP9 gene, ADIPOQ gene, CD36 gene, G0S2 gene, GPD1 gene, LEP gene, LIPE gene, PLIN1 gene, CAVIN2 gene, LIFR gene, TGFBR3 gene, CAPN6 gene, PIGR gene, KRT15 gene, KRT5 gene, KRT14 gene, DST gene, WIF1 gene, SYNM gene, KIT gene, GABRP gene, SFRP1 gene, ELF5 gene, MIA gene, MMP7 gene, FDCSP gene, CRABP1 gene, PROM1 gene, KRT23 gene, S100A1 gene, WIPF3 gene, CYYR1 gene, TFCP2L1 gene, DSC2 gene, MFGE8 gene, KLK7 gene, KLK5 gene, DSG3 gene, TTYH1 gene, SCRG1 gene, S100B gene, ETV6 gene, OGFRL1 gene, MELTF gene, HORMAD1 gene, PKP1 gene, FOXC1 gene, ITGB8 gene, VGLL1 gene, ART3 gene, EN1 gene, SPHK1 gene, TRIM47 gene, COL27A1 gene, RFLNA gene, RASD2 gene, A2ML1 gene, MARCO gene, TSPYL5 gene, TM4SF1 gene, FABP5 gene, SPIB gene, BCL2A1 gene, MZB1 gene, KCNK5 gene, LM04 gene, RNF150 gene, LYZ gene, C21orf58 gene, ATP13A5 gene, NUDT8 gene, HSD17B2 gene, ABCA12 gene, ENPP3 gene, WNT5A gene, MPP3 gene, VPS13D gene, PXMP4 gene, GGT1 gene, TRPV6 gene, MAB21L4 gene, CLDN8 gene, LBP gene, SRD5A3 gene, PAPSS2 gene, TMEM45B gene, CLCA2 gene, FASN gene, MPHOSPH6 gene, NXPH4 gene, HPGD gene, KYNU gene, GLYATL2 gene, KMO gene, SRPK3 gene, THRSP gene, PLA2G2A gene, TFAP2B gene, FABP7 gene, SLPI gene, SERHL2 gene, S100A9 gene, KRT7 gene, TMEM86A gene, MBOAT1 gene, PGAP3 gene, STARD3 gene, ERBB2 gene, MIEN1 gene, GRB7 gene, GSDMB gene, ORMDL3 gene, MED24 gene, MSL1 gene, CASC3 gene, WIPF2 gene, THSD4 gene, MAPT gene, LONRF2 gene, TCEAL3 gene, DBNDD2 gene, FGD3 gene, GFRA1 gene, PARD6B gene, STC2 gene, SLC39A6 gene, ENPP5 gene, ZNF703 gene, EVL gene, TBC1D9 gene, CHAD gene, GREB1 gene, HPN gene, IL6ST gene, GASK1B gene, CA12 gene, KCNE4 gene, NAT1 gene, CYP2B6 (CYP2B7P) gene, ARMT1 gene, MAGED2 gene, CELSR1 gene, INPP5J gene, PADI2 gene, PPP1R1B gene, ESR1 gene, MLPH gene, FOXA1 gene, XBP1 gene, GATA3 gene, ZG16B gene, KIAA0040 gene, TMC4 gene, AGR2 gene, TFF3 gene, SCGB2A2 gene, MUCL1 gene, DDX11 gene, ATAD2 gene, GGH gene, CDCA3 gene, CCNA2 gene, CCNB2 gene, ANLN gene, UBE2C gene, CKS2 gene, MKI67 gene, FOXM1 gene, UBE2T gene, MCM4 gene, CKAP2 gene, JPT1 gene, KPNA2 gene, H2AFX gene, H2AFZ gene, CDK1 gene, PTTG1 gene, CDC20 gene, MYBL2 gene, and RRM2 gene.

In this specification, the genes included in the "differentiating gene group" include genes composed of nucleotide sequences including degenerate codons encoding the same amino acid sequence, mutant genes such as various mutants (variants) of individual genes and point-mutant genes, and ortholog genes of organisms of other species such as chimpanzee. Such genes include genes that are composed of a nucleotide sequence of a gene specified by the GenBank accession number shown in the table below and a nucleotide sequence having a base identity of 70% or more (preferably 75% or more, 80% or more, or 85% or more, and more preferably 90% or more, 95% or more, 96% or more, 97% or more, 98% or more, or 99% or more) and retain the function of the target gene.

For example, in one embodiment, the ABCF3 genes used in the present invention can be specified as genes including the nucleotide sequence indicated by sequence number 1 and, at this time, includes genes that are composed of the nucleotide sequence indicated by sequence number 1 and a nucleotide sequence having a base identity of 70% or more (preferably 75% or more, 80% or more, or 85% or more, and more preferably 90% or more, 95% or more, 96% or more, 97% or more, 98% or more, or 99% or more) and retain the function of the ABCF3 gene. It should be noted that, in this specification, the term "base identity" refers to a percentage (%) of the number of identical bases in nucleotide sequences of nucleotides to be compared with respect to the total number of bases of genes when two nucleotide sequences are aligned, if necessary with a gap such that the degree of matching between both nucleotide sequences is maximized.

The "differentiating gene group" can be divided into the following groups a to o as gene groups characteristic of each subtype of breast cancer (each group of the groups a to o does not necessarily have a one-to-one correspondence with each subtype). In this specification, the gene group showing an expression pattern characteristic of squamous cell carcinoma is classified as "group a," the gene group showing an expression pattern characteristic of phyllodes tumor is classified as "group b," the gene group showing an expression pattern characteristic of cancer is classified as "group c," the gene group showing an expression pattern characteristic of normal tissue is classified as "group d," the gene group showing an expression pattern characteristic of normal-like is classified as "group e," the gene (hereinafter referred to as "TNBC1") group showing an expression pattern characteristic of the triple negative group and showing an expression pattern characteristic of normal tissue or normal-like is classified as "group f," the gene (hereinafter referred to as "TNBC2") group showing an expression pattern characteristic of the triple negative is classified as "group g," the gene (hereinafter referred to as "TNBC3") group showing an expression pattern characteristic of the triple negative and similar to the expression pattern of genes defined as undeterminable is classified as "group h," the gene group showing an expression pattern characteristic of HER2+-like is classified as "group i," the gene (hereinafter referred to as "HER2 amplification-1") group related to HER2 amplification and positioned close to the HER2 gene on the chromosome is classified as "group j," the gene (hereinafter referred to as "HER2 amplification-2") group related to HER2 amplification other than group j is classified as "group k," the hormone sensitivity-related gene group is classified as "group 1," ESR1 genes are classified as "group m," differentiation-related genes are classified as "group n," and the cell cycle-related gene group is classified as "group o." The "differentiating gene groups" classified into groups a to o are shown in Tables 3A to 3G below. Tables 3A to 3G also show the control genes having little variation in gene expression for each subtype. It should be noted that, for one gene (MBOAT1) belonging to group i and two genes (PADI2 and PPP1R1B) belonging to group 1, the appearance of the characteristic thereof increases as the expression ratio decreases. When the MBOAT1 gene is used for differentiation or classification, it is preferable to perform operations as appropriate, such as using an inverted value as the score value.

| Classification | Symbol | Name | ID | Sequence number |
|---|---|---|---|---|
| control | FBXW5 | F-box and WD-40 domain protein 5 (FBXW5), transcript variant 2, mRNA. | NM_018998 | Sequence number 1 |
| control | PITPNM1 | phosphatidylinositol transfer protein, membrane-associated 1 (PITPNM1), mRNA. | NM_004910 | Sequence number 2 |
| control | MLLT1 | myeloid/lymphoid or mixed-lineage leukemia (trithorax homolog, Drosophila); translocated to, 1 (MLLT1), mRNA. | NM_005934 | Sequence number 3 |
| control | WDR1 | WD repeat domain 1 (WDR1), transcript variant 1, mRNA. | NM_017491 | Sequence number 4 |
| control | ABCF3 | ATP-binding cassette, sub-family F (GCN20), member 3 (ABCF3), mRNA. | NM_018358 | Sequence number 5 |
| control | NDUFS7 | NADH dehydrogenase (ubiquinone) Fe-S protein 7, 20kDa (NADH-coenzyme Q reductase) (NDUFS7), mRNA. | NM_024407 | Sequence number 6 |
| control | FAM234A | hypothetical protein DKFZp761D0211 (DKFZP761D0211), mRNA. | NM_032039 | Sequence number 7 |
| control | AP2A1 | adaptor-related protein complex 2, alpha 1 subunit (AP2A1), transcript variant 2, mRNA. | NM_130787 | Sequence number 8 |
| Squamous cell carcinoma | KRTDAP | keratinocyte differentiation-associated protein (KRTDAP), mRNA. | NM_207392 | Sequence number 9 |
| Squamous cell carcinoma | LGALS7 | lectin, galactoside-binding, soluble, 7 (galectin 7) (LGALS7), mRNA. | NM_002307 | Sequence number 10 |
| Squamous cell carcinoma | PI3 | protease inhibitor 3, skin-derived (SKALP) (PI3), mRNA. | NM_002638 | Sequence number 11 |
| Squamous cell carcinoma | SPRR1B | small proline-rich protein 1B (cornifin) (SPRR1B), mRNA. | NM_003125 | Sequence number 12 |
| Squamous cell carcinoma | SPRR2A | small proline-rich protein 2A (SPRR2A), mRNA. | NM_005988 | Sequence number 13 |
| Squamous cell carcinoma | KRT1 | keratin 1 (epidermolytic hyperkeratosis) (KRT1), mRNA. | NM_006121 | Sequence number 14 |
| Squamous cell carcinoma | SERPINB3 | serine (or cysteine) proteinase inhibitor, clade B (ovalbumin), member 3 (SERPINB3), mRNA. | NM_006919 | Sequence number 15 |
| Squamous cell carcinoma | KLK13 | kallikrein 13 (KLK13), mRNA. | NM_015596 | Sequence number 16 |
| Phyllodes tumor | SH3PXD2B | similar to KIAA1295 protein (LOC220776), mRNA. | NM_001017995 | Sequence number 17 |
| Phyllodes tumor | PTH1R | parathyroid hormone receptor 1 (PTHR1), mRNA. | NM_000316 | Sequence number 18 |
| Phyllodes tumor | SERPINH1 | serine (or cysteine) proteinase inhibitor, clade H (heat shock protein 47), member 1, (collagen binding protein 1) (SERPINH1), mRNA. | NM_001235 | Sequence number 19 |
| Phyllodes tumor | SNAI2 | snail homolog 2 (Drosophila) (SNAI2), mR | NM_003068 | Sequence number 20 |
| Phyllodes tumor | HAS2 | hyaluronan synthase 2 (HAS2), mRNA. | NM_005328 | Sequence number 21 |
| Phyllodes tumor | ITLN1 | intelectin 1 (galactofuranose binding) (ITLN1), mRNA. | NM_017625 | Sequence number 22 |
| Phyllodes tumor | GPR173 | super conserved receptor expressed in br | NM_018969 | Sequence number 23 |
| Phyllodes tumor | PAGE5 | PAGE-5 protein (PAGE-5), mRNA. | NM_130467 | Sequence number 24 |
| Cancer | FN1 | cellular fibronectin mRNA | NM_002026 | Sequence number 25 |
| Cancer | TAP1 | transporter 1, ATP-binding cassette, sub-family B (MDR/TAP) (TAP1), mRNA. | NM_000593 | Sequence number 26 |
| Cancer | MMP9 | matrix metalloproteinase 9 (gelatinase B, 92kDa gelatinase, 92kDa type IV collagenase) (MMP9), mRNA. | NM_004994 | Sequence number 27 |
| Cancer | CTHRC1 | collagen triple helix repeat containing | NM_138455 | Sequence number 28 |

Group a — Squamous cell carcinoma rows

Group b — Phyllodes tumor rows

Group c — Cancer rows

| Classification | | Symbol | Name | ID | Sequence number |
|---|---|---|---|---|---|
| Group d | Normal | CD36 | CD36 antigen (collagen type I receptor, | NM_000072 | Sequence number 29 |
| | Normal | LEP | leptin (obesity homolog, mouse) (LEP), mRNA | NM_000230 | Sequence number 30 |
| | Normal | LIFR | leukemia inhibitory factor receptor (LIFR), mRNA | NM_002310 | Sequence number 31 |
| | Normal | PLIN1 | perilipin (PLIN), mRNA | NM_002666 | Sequence number 32 |
| | Normal | TGFBR3 | transforming growth factor, beta receptor III (betaglycan, 300kDa) (TGFBR3), mRNA | NM_003243 | Sequence number 33 |
| | Normal | CAVIN2 | serum deprivation response (phosphatidylserine binding protein) (SDPR), mRNA | NM_004657 | Sequence number 34 |
| | Normal | ADIPOQ | adipocyte, C1Q and collagen domain containing (ACDC), mRNA | NM_004797 | Sequence number 35 |
| | Normal | GPD1 | glycerol-3-phosphate dehydrogenase 1 (soluble) (GPD1), mRNA | NM_005276 | Sequence number 36 |
| | Normal | LIPE | lipase, hormone-sensitive (LIPE), mRNA | NM_005357 | Sequence number 37 |
| | Normal | G0S2 | putative lymphocyte G0/G1 switch gene (G0S2), mRNA | NM_015714 | Sequence number 38 |
| Group e | Normal-like | KIT | v-kit Hardy-Zuckerman 4 feline sarcoma viral oncogene homolog (KIT), mRNA | NM_000222 | Sequence number 39 |
| | Normal-like | KRT5 | keratin 5 (epidermolysis bullosa simplex, Dowling-Meara/Kobner/Weber-Cockayne types) (KRT5), mRNA | NM_000424 | Sequence number 40 |
| | Normal-like | KRT14 | keratin 14 (epidermolysis bullosa simplex, Dowling-Meara, Koebner) (KRT14), mRNA | NM_000526 | Sequence number 41 |
| | Normal-like | DST | bullous pemphigoid antigen 1, 230/240kDa (BPAG1), transcript variant 1e, mRNA | NM_001723 | Sequence number 42 |
| | Normal-like | KRT15 | keratin 15 (KRT15), mRNA | NM_002275 | Sequence number 43 |
| | Normal-like | PIGR | polymeric immunoglobulin receptor (PIGR), mRNA | NM_002644 | Sequence number 44 |
| | Normal-like | WIF1 | WNT inhibitory factor 1 (WIF1), mRNA | NM_007191 | Sequence number 45 |
| | Normal-like | CAPN6 | calpain 6 (CAPN6), mRNA | NM_014289 | Sequence number 46 |
| | Normal-like | SYNM | desmuslin (DMN), transcript variant A, mRNA | NM_145728 | Sequence number 47 |
| Group f | TNBC1 | GABRP | gamma-aminobutyric acid (GABA) A receptor, pi (GABRP), mRNA | NM_014211 | Sequence number 48 |
| | TNBC1 | ELF5 | E74-like factor 5 (ets domain transcription factor) (ELF5), transcript variant 2, mRNA | NM_001422 | Sequence number 49 |
| | TNBC1 | MMP7 | matrix metalloproteinase 7 (matrilysin, uterine) (MMP7), mRNA | NM_002423 | Sequence number 50 |
| | TNBC1 | SFRP1 | secreted frizzled-related protein 1 (SFRP1), mRNA | NM_003012 | Sequence number 51 |
| | TNBC1 | MIA | melanoma inhibitory activity (MIA), mRNA | NM_006533 | Sequence number 52 |
| | TNBC1 | FDCSP | chromosome 4 open reading frame 7 (C4orf7), mRNA | NM_152997 | Sequence number 53 |

| Classification | Symbol | Name | ID | Sequence number |
|---|---|---|---|---|
| TNBC2 | WIPF3 | cDNA FLJ36931 fis, clone BRACE2005290. | NM_001080529 | Sequence number 54 |
| TNBC2 | PKP1 | plakophilin 1 (ectodermal dysplasia/skin fragility syndrome) (PKP1), mRNA. | NM_000299 | Sequence number 55 |
| TNBC2 | ART3 | ADP-ribosyltransferase 3 (ART3), mRNA. | NM_001179 | Sequence number 56 |
| TNBC2 | EN1 | engrailed homolog 1 (EN1), mRNA. | NM_001426 | Sequence number 57 |
| TNBC2 | FABP5 | fatty acid binding protein 5 (psoriasis-associated) (FABP5), mRNA. | NM_001444 | Sequence number 58 |
| TNBC2 | FOXC1 | forkhead box C1 (FOXC1), mRNA. | NM_001453 | Sequence number 59 |
| TNBC2 | DSG3 | desmoglein 3 (pemphigus vulgaris antigen) (DSG3), mRNA. | NM_001944 | Sequence number 60 |
| TNBC2 | ETV6 | ets variant gene 6 (TEL oncogene) (ETV6), mRNA. | NM_001987 | Sequence number 61 |
| TNBC2 | ITGB8 | integrin, beta 8 (ITGB8), mRNA. | NM_002214 | Sequence number 62 |
| TNBC2 | CRABP1 | cellular retinoic acid binding protein 1 (CRABP1), mRNA. | NM_004378 | Sequence number 63 |
| TNBC2 | DSC2 | desmocollin 2 (DSC2), transcript variant Dsc2b, mRNA. | NM_004949 | Sequence number 64 |
| TNBC2 | KLK7 | kallikrein 7 (chymotryptic, stratum corneum) (KLK7), transcript variant 1, mRNA. | NM_005046 | Sequence number 65 |
| TNBC2 | MFGE8 | milk fat globule-EGF factor 8 protein (MFGE8), mRNA. | NM_005928 | Sequence number 66 |
| TNBC2 | MELTF | antigen p97 (melanoma associated) identified by monoclonal antibodies 133.2 and 96.5 (MFI2), transcript variant 1, mRNA. | NM_005929 | Sequence number 67 |
| TNBC2 | PROM1 | prominin 1 (PROM1), mRNA. | NM_006017 | Sequence number 68 |
| TNBC2 | S100A1 | S100 calcium binding protein A1 (S100A1), mRNA. | NM_006271 | Sequence number 69 |
| TNBC2 | S100B | S100 calcium binding protein, beta (neural) (S100B), mRNA. | NM_006272 | Sequence number 70 |
| TNBC2 | MARCO | macrophage receptor with collagenous structure (MARCO), mRNA. | NM_006770 | Sequence number 71 |
| TNBC2 | SCRG1 | scrapie responsive protein 1 (SCRG1), mRNA. | NM_007281 | Sequence number 72 |
| TNBC2 | KLK5 | kallikrein 5 (KLK5), mRNA. | NM_012427 | Sequence number 73 |
| TNBC2 | TM4SF1 | transmembrane 4 superfamily member 1 (TM4SF1), mRNA. | NM_014220 | Sequence number 74 |
| TNBC2 | RASD2 | RASD family, member 2 (RASD2), mRNA. | NM_014310 | Sequence number 75 |
| TNBC2 | TFCP2L1 | transcription factor CP2-like 1 (TFCP2L1), mRNA. | NM_014553 | Sequence number 76 |
| TNBC2 | KRT23 | keratin 23 (histone deacetylase inducible) (KRT23), transcript variant 1, mRNA. | NM_015515 | Sequence number 77 |
| TNBC2 | VGLL1 | vestigial like 1 (Drosophila) (VGLL1), mRNA. | NM_016267 | Sequence number 78 |
| TNBC2 | TTYH1 | tweety homolog 1 (Drosophila) (TTYH1), mRNA. | NM_020659 | Sequence number 79 |
| TNBC2 | SPHK1 | sphingosine kinase 1 (SPHK1), mRNA. | NM_021972 | Sequence number 80 |
| TNBC2 | OGFRL1 | opioid growth factor receptor-like 1 (OGFRL1), mRNA. | NM_024576 | Sequence number 81 |
| TNBC2 | HORMAD1 | hypothetical protein DKFZp434A1315 (DKFZP434A1315), mRNA. | NM_032132 | Sequence number 82 |
| TNBC2 | COL27A1 | collagen, type XXVII, alpha 1 (COL27A1), | NM_032888 | Sequence number 83 |
| TNBC2 | TRIM47 | tripartite motif-containing 47 (TRIM47), mRNA. | NM_033452 | Sequence number 84 |
| TNBC2 | TSPYL5 | TSPY-like 5 (TSPYL5), mRNA. | NM_033512 | Sequence number 85 |
| TNBC2 | CYYR1 | cysteine and tyrosine-rich 1 (CYYR1), mRNA. | NM_052954 | Sequence number 86 |
| TNBC2 | A2ML1 | hypothetical protein FLJ25179 (FLJ25179), mRNA. | NM_144670 | Sequence number 87 |
| TNBC2 | RFLNA | hypothetical protein LOC144347 (LOC144347), mRNA. | NM_181709 | Sequence number 88 |
| TNBC3 | RNF150 | cDNA FLJ10151 fis, clone HEMBA1003402. | XM_005263150 | Sequence number 89 |
| TNBC3 | MZB1 | cDNA FLJ32987 fis, clone THYMU1000032. | NM_016459 | Sequence number 90 |
| TNBC3 | LYZ | lysozyme (renal amyloidosis) (LYZ), mRNA. | NM_000239 | Sequence number 91 |
| TNBC3 | SPIB | Spi-B transcription factor (Spi-1/PU.1 related) (SPIB), mRNA. | NM_003121 | Sequence number 92 |
| TNBC3 | KCNK5 | potassium channel, subfamily K, member 5 (KCNK5), mRNA. | NM_003740 | Sequence number 93 |
| TNBC3 | BCL2A1 | BCL2-related protein A1 (BCL2A1), mRNA. | NM_004049 | Sequence number 94 |
| TNBC3 | LMO4 | LIM domain only 4 (LMO4), mRNA. | NM_006769 | Sequence number 95 |

Group 3

Group 4

| Classification | Symbol | Name | ID | Sequence number |
|---|---|---|---|---|
| HER2+-like | GLYATL2 | BXMAS2-10 (BXMAS2-10), mRNA | NM_145016 | Sequence number 96 |
| HER2+-like | GGT1 | gamma-glutamyltransferase 1 (GGT1), transcript variant 1, mRNA | NM_013421 | Sequence number 97 |
| HER2+-like | NXPH4 | cDNA FLJ36912 fis, clone BRACE2003847, highly similar to Rattus norvegicus neurexophilin 4 (Nph4) mRNA | NM_007224 | Sequence number 98 |
| HER2+-like | ATP13A5 | cDNA FLJ16025 fis, clone CTONG2004062, highly similar to ATPase subunit 6. | NM_198505 | Sequence number 99 |
| HER2+-like | PLA2G2A | phospholipase A2, group IIA (platelets, synovial fluid) (PLA2G2A), mRNA | NM_000300 | Sequence number 100 |
| HER2+-like | HPGD | hydroxyprostaglandin dehydrogenase 15-(NAD) (HPGD), mRNA | NM_000860 | Sequence number 101 |
| HER2+-like | FABP7 | fatty acid binding protein 7, brain (FABP7), mRNA | NM_001446 | Sequence number 102 |
| HER2+-like | MPP3 | membrane protein, palmitoylated 3 (MAGUK p55 subfamily member 3) (MPP3), mRNA | NM_001932 | Sequence number 103 |
| HER2+-like | HSD17B2 | hydroxysteroid (17-beta) dehydrogenase 2 (HSD17B2), mRNA | NM_002153 | Sequence number 104 |
| HER2+-like | S100A9 | S100 calcium binding protein A9 (calgranulin B) (S100A9), mRNA | NM_002965 | Sequence number 105 |
| HER2+-like | SLPI | secretory leukocyte protease inhibitor (antileukoproteinase) (SLPI), mRNA | NM_003064 | Sequence number 106 |
| HER2+-like | TFAP2B | transcription factor AP-2 beta (activating enhancer binding protein 2 beta) (TFAP2B), mRNA | NM_003221 | Sequence number 107 |
| HER2+-like | THRSP | thyroid hormone responsive (SPOT14 homolog, rat) (THRSP), mRNA | NM_003251 | Sequence number 108 |
| HER2+-like | WNT5A | wingless-type MMTV integration site family, member 5A (WNT5A), mRNA | NM_003392 | Sequence number 109 |
| HER2+-like | KMO | kynurenine 3-monooxygenase (kynurenine 3-hydroxylase) (KMO), mRNA | NM_003679 | Sequence number 110 |
| HER2+-like | KYNU | kynureninase (L-kynurenine hydrolase) (KYNU), mRNA | NM_003937 | Sequence number 111 |
| HER2+-like | FASN | fatty acid synthase (FASN), mRNA | NM_004104 | Sequence number 112 |
| HER2+-like | LBP | lipopolysaccharide binding protein (LBP), mRNA | NM_004139 | Sequence number 113 |
| HER2+-like | PAPSS2 | 3'-phosphoadenosine 5'-phosphosulfate synthase 2 (PAPSS2), mRNA | NM_004670 | Sequence number 114 |
| HER2+-like | ENPP3 | ectonucleotide pyrophosphatase/phosphodiesterase 3 (ENPP3), mRNA | NM_005021 | Sequence number 115 |
| HER2+-like | MPHOSPH6 | M-phase phosphoprotein 6 (MPHOSPH6), mRNA | NM_005792 | Sequence number 116 |
| HER2+-like | CLCA2 | chloride channel, calcium activated, family member 2 (CLCA2), mRNA | NM_006536 | Sequence number 117 |
| HER2+-like | PXMP4 | peroxisomal membrane protein 4, 24kDa (PXMP4), transcript variant 1, mRNA | NM_007238 | Sequence number 118 |
| HER2+-like | SRPK3 | serine/threonine kinase 23 (STK23), mRNA | NM_014370 | Sequence number 119 |
| HER2+-like | SERHL2 | kraken-like (dJ222E13.1), mRNA | NM_014509 | Sequence number 120 |
| HER2+-like | VPS13D | vacuolar protein sorting 13D (yeast) (VP | NM_015378 | Sequence number 121 |
| HER2+-like | ABCA12 | ATP-binding cassette, sub-family A (ABC1), member 12 (ABCA12), transcript variant 2, mRNA | NM_015657 | Sequence number 122 |
| HER2+-like | TRPV6 | transient receptor potential cation chan | NM_018646 | Sequence number 123 |
| HER2+-like | SRD5A3 | hypothetical protein FLJ13352 (FLJ13352), mRNA | NM_024592 | Sequence number 124 |
| HER2+-like | MAB21L4 | hypothetical protein FLJ22671 (FLJ22671), mRNA | NM_024861 | Sequence number 125 |
| HER2+-like | C21orf58 | chromosome 21 open reading frame 58 (C21orf58), transcript variant 1, mRNA | NM_058180 | Sequence number 126 |
| HER2+-like | TMEM45B | hypothetical protein BC016153 (LOC120224), mRNA | NM_138788 | Sequence number 127 |
| HER2+-like | NUDT8 | nudix (nucleoside diphosphate linked moiety X)-type motif 8 (NUDT8), mRNA | NM_181843 | Sequence number 128 |
| HER2+-like | CLDN8 | claudin 8 (CLDN8), mRNA | NM_199328 | Sequence number 129 |
| HER2+-like | KRT7 | keratin 7 (KRT7), mRNA | NM_005556 | Sequence number 130 |
| HER2+-like | TMEM86A | hypothetical protein FLJ90119 (FLJ90119), mRNA | NM_153347 | Sequence number 131 |
| HER2+-like | MBOAT1 | cDNA FLJ16207 fis, clone CTONG2019822 | NM_001080480 | Sequence number 132 |

Group i

| Classification | Symbol | Name | ID | Sequence number |
|---|---|---|---|---|
| HER2 amplification-1 | PGAP3 | per1-like domain containing 1 (PERLD1), mRNA. | NM_033419 | Sequence number 133 |
| HER2 amplification-1 | STARD3 | START domain containing 3 (STARD3), mRNA. | NM_006804 | Sequence number 134 |
| HER2 amplification-1 | ERBB2 | v-erb-b2 erythroblastic leukemia viral oncogene homolog 2, neuro/glioblastoma derived oncogene homolog (avian) (ERBB2), mRNA. | NM_004448 | Sequence number 135 |
| HER2 amplification-1 | MIEN1 | chromosome 17 open reading frame 37 (C17orf37), mRNA. | NM_032339 | Sequence number 136 |
| HER2 amplification-1 | GRB7 | growth factor receptor-bound protein 7 (GRB7), mRNA. | NM_005310 | Sequence number 137 |
| HER2 amplification-2 | GSDMB | gasdermin-like (GSDML), mRNA. | NM_018530 | Sequence number 138 |
| HER2 amplification-2 | ORMDL3 | ORM1-like 3 (S. cerevisiae) (ORMDL3), mRNA. | NM_139280 | Sequence number 139 |
| HER2 amplification-2 | MED24 | thyroid hormone receptor associated protein 4 (THRAP4), mRNA. | NM_014815 | Sequence number 140 |
| HER2 amplification-2 | MSL1 | cDNA FLJ30816 fis, clone FEBRA2001571. | NM_001012241 | Sequence number 141 |
| HER2 amplification-2 | CASC3 | cancer susceptibility candidate 3 (CASC3), mRNA. | NM_007359 | Sequence number 142 |
| HER2 amplification-2 | WIPF2 | WIRE protein (WIRE), mRNA. | NM_133264 | Sequence number 143 |

Group j

Group k

18

| Classification | Symbol | Name | ID | Sequence number |
|---|---|---|---|---|
| Hormone sensitivity | GFRA1 | GDNF family receptor alpha 1 (GFRA1), transcript variant 1, mRNA. | NM_005264 | Sequence number 144 |
| Hormone sensitivity | MAPT | microtubule-associated protein tau (MAPT), transcript variant 1, mRNA. | NM_016835 | Sequence number 145 |
| Hormone sensitivity | EVL | Enah/Vasp-like (EVL), mRNA. | NM_016337 | Sequence number 146 |
| Hormone sensitivity | CA12 | carbonic anhydrase XII (CA12), transcrip | NM_206925 | Sequence number 147 |
| Hormone sensitivity | LONRF2 | cDNA FLJ31811 fis, clone NT2RI2009402. | NM_198461 | Sequence number 148 |
| Hormone sensitivity | CYP2B6 | cytochrome P450-IIB (hIIB3) mRNA, complete cds. | NM_000767 | Sequence number 149 |
| Hormone sensitivity | PARD6B | par-6 partitioning defective 6 homolog b | NM_032521 | Sequence number 150 |
| Hormone sensitivity | TBC1D9 | KIAA0882 protein (KIAA0882), mRNA. | NM_015130 | Sequence number 151 |
| Hormone sensitivity | ESR1 | estrogen receptor 1 (ESR1), mRNA. | NM_000125 | Sequence number 152 |
| Hormone sensitivity | NAT1 | N-acetyltransferase 1 (arylamine N-acetyltransferase) (NAT1), mRNA. | NM_000662 | Sequence number 153 |
| Hormone sensitivity | CHAD | chondroadherin (CHAD), mRNA. | NM_001267 | Sequence number 154 |
| Hormone sensitivity | HPN | hepsin (transmembrane protease, serine 1) (HPN), transcript variant 2, mRNA. | NM_002151 | Sequence number 155 |
| Hormone sensitivity | IL6ST | interleukin 6 signal transducer (gp130, oncostatin M receptor) (IL6ST), transcript variant 1, mRNA. | NM_002184 | Sequence number 156 |
| Hormone sensitivity | STC2 | stanniocalcin 2 (STC2), mRNA. | NM_003714 | Sequence number 157 |
| Hormone sensitivity | SLC39A6 | solute carrier family 39 (zinc transporter), member 6 (SLC39A6), mRNA. | NM_012319 | Sequence number 158 |
| Hormone sensitivity | GREB1 | GREB1 protein (GREB1), transcript variant a, mRNA. | NM_014668 | Sequence number 159 |
| Hormone sensitivity | GASK1B | hypothetical protein DKFZp434L142 (DKFZp434L142), mRNA. | NM_016613 | Sequence number 160 |
| Hormone sensitivity | DBNDD2 | chromosome 20 open reading frame 35 (C20orf35), mRNA. | NM_018478 | Sequence number 161 |
| Hormone sensitivity | ENPP5 | ectonucleotide pyrophosphatase/phosphodiesterase 5 (putative function) (ENPP5), mRNA. | NM_021572 | Sequence number 162 |
| Hormone sensitivity | THSD4 | hypothetical protein FLJ13710 (FLJ13710), mRNA. | NM_024817 | Sequence number 163 |
| Hormone sensitivity | ZNF703 | hypothetical protein FLJ14299 (FLJ14299), mRNA. | NM_025069 | Sequence number 164 |
| Hormone sensitivity | TCEAL3 | hypothetical protein MGC15737 (MGC15737), mRNA. | NM_032926 | Sequence number 165 |
| Hormone sensitivity | FGD3 | FGD1 family, member 3 (FGD3), mRNA. | NM_033086 | Sequence number 166 |
| Hormone sensitivity | KCNE4 | potassium voltage-gated channel, Isk-related family, member 4 (KCNE4), mRNA. | NM_080671 | Sequence number 167 |
| Hormone sensitivity | ARMT1 | chromosome 6 open reading frame 211 (C6orf211), mRNA. | NM_024573 | Sequence number 168 |
| Hormone sensitivity | MAGED2 | melanoma antigen, family D, 2 (MAGED2), transcript variant 2, mRNA. | NM_177433 | Sequence number 169 |
| Hormone sensitivity | CELSR1 | cadherin, EGF LAG seven-pass G-type receptor 1 (flamingo homolog, Drosophila) (CELSR1), mRNA. | NM_014246 | Sequence number 170 |
| Hormone sensitivity | INPP5J | phosphatidylinositol (4,5) bisphosphate 5-phosphatase, A (PIB5PA), mRNA. | NM_001002837 | Sequence number 171 |
| Hormone sensitivity | PADI2 | peptidyl arginine deiminase, type II (PADI2), mRNA. | NM_007365 | Sequence number 172 |
| Hormone sensitivity | PPP1R1B | protein phosphatase 1, regulatory (inhibitor) subunit 1B (dopamine and cAMP regulated phosphoprotein, DARPP-32) (PPP1R1B), mRNA. | NM_032192 | Sequence number 173 |

Group 1

[Table 3G]

| Classification | Symbol | Name | ID | Sequence number |
|---|---|---|---|---|
| Differentiated | GATA3 | GATA binding protein 3 (GATA3), mRNA. | NM_002051 | Sequence number 174 |
| Differentiated | SCGB2A2 | secretoglobin, family 2A, member 2 (SCGB2A2), mRNA. | NM_002411 | Sequence number 175 |
| Differentiated | TFF3 | trefoil factor 3 (intestinal) (TFF3), mRNA. | NM_003226 | Sequence number 176 |
| Differentiated | FOXA1 | forkhead box A1 (FOXA1), mRNA. | NM_004496 | Sequence number 177 |
| Differentiated | XBP1 | X-box binding protein 1 (XBP1), mRNA. | NM_005080 | Sequence number 178 |
| Differentiated | AGR2 | anterior gradient 2 homolog (Xenopus laevis) (AGR2), mRNA. | NM_006408 | Sequence number 179 |
| Differentiated | KIAA0040 | KIAA0040 gene product (KIAA0040), mRNA. | NM_014656 | Sequence number 180 |
| Differentiated | MLPH | melanophilin (MLPH), mRNA. | NM_024101 | Sequence number 181 |
| Differentiated | MUCL1 | small breast epithelial mucin (LOC118430), mRNA. | NM_058173 | Sequence number 182 |
| Differentiated | TMC4 | transmembrane channel-like 4 (TMC4), mRNA. | NM_144686 | Sequence number 183 |
| Differentiated | ZG16B | similar to common salivary protein 1 (LOC124220), mRNA. | NM_145252 | Sequence number 184 |
| Cell cycle | RRM2 | ribonucleotide reductase M2 polypeptide (RRM2), mRNA. | NM_001034 | Sequence number 185 |
| Cell cycle | CCNA2 | cyclin A2 (CCNA2), mRNA. | NM_001237 | Sequence number 186 |
| Cell cycle | CDC20 | CDC20 cell division cycle 20 homolog (S. cerevisiae) (CDC20), mRNA. | NM_001255 | Sequence number 187 |
| Cell cycle | CDK1 | cell division cycle 2, G1 to S and G2 to M (CDC2), transcript variant 1, mRNA. | NM_001786 | Sequence number 188 |
| Cell cycle | CKS2 | CDC28 protein kinase regulatory subunit 2 (CKS2), mRNA. | NM_001827 | Sequence number 189 |
| Cell cycle | H2AFX | H2A histone family, member X (H2AFX), mRNA. | NM_002105 | Sequence number 190 |
| Cell cycle | H2AFZ | H2A histone family, member Z (H2AFZ), mRNA. | NM_002106 | Sequence number 191 |
| Cell cycle | KPNA2 | karyopherin alpha 2 (RAG cohort 1, importin alpha 1) (KPNA2), mRNA. | NM_002266 | Sequence number 192 |
| Cell cycle | MKI67 | antigen identified by monoclonal antibody Ki-67 (MKI67), mRNA. | NM_002417 | Sequence number 193 |
| Cell cycle | MYBL2 | v-myb myeloblastosis viral oncogene homolog (avian)-like 2 (MYBL2), mRNA. | NM_002466 | Sequence number 194 |
| Cell cycle | GGH | gamma-glutamyl hydrolase (conjugase, folylpolygammaglutamyl hydrolase) (GGH), mRNA. | NM_003878 | Sequence number 195 |
| Cell cycle | PTTG1 | pituitary tumor-transforming 1 (PTTG1), mRNA. | NM_004219 | Sequence number 196 |
| Cell cycle | DDX11 | DEAD/H (Asp-Glu-Ala-Asp/His) box polypeptide 11 (CHL1-like helicase homolog, S. cerevisiae) (DDX11), transcript variant 2, mRNA. | NM_004399 | Sequence number 197 |
| Cell cycle | CCNB2 | cyclin B2 (CCNB2), mRNA. | NM_004701 | Sequence number 198 |
| Cell cycle | UBE2C | ubiquitin-conjugating enzyme E2C (UBE2C), transcript variant 1, mRNA. | NM_007019 | Sequence number 199 |
| Cell cycle | ATAD2 | ATPase family, AAA domain containing 2 (ATAD2), mRNA. | NM_014109 | Sequence number 200 |
| Cell cycle | UBE2T | HSPC150 protein similar to ubiquitin-conjugating enzyme (HSPC150), mRNA. | NM_014176 | Sequence number 201 |
| Cell cycle | JPT1 | hematological and neurological expressed 1 (HN1), mRNA. | NM_016185 | Sequence number 202 |
| Cell cycle | CKAP2 | cytoskeleton associated protein 2 (CKAP2), mRNA. | NM_018204 | Sequence number 203 |
| Cell cycle | ANLN | anillin, actin binding protein (scraps homolog, Drosophila) (ANLN), mRNA. | NM_018685 | Sequence number 204 |
| Cell cycle | FOXM1 | forkhead box M1 (FOXM1), transcript variant 2, mRNA. | NM_021953 | Sequence number 205 |
| Cell cycle | CDCA3 | cell division cycle associated 3 (CDCA3), mRNA. | NM_031299 | Sequence number 206 |
| Cell cycle | MCM4 | MCM4 minichromosome maintenance deficient 4 (S. cerevisiae) (MCM4), transcript variant 2, mRNA. | NM_182746 | Sequence number 207 |

Group n (Differentiated); Group o (Cell cycle)

The "subtype differentiation score" is a score capable of differentiating or classifying a subtype of breast cancer. One or a plurality of "subtype differentiation scores" can be used when differentiating or classifying a subtype of breast cancer. Examples of "subtype differentiation scores" include a cancer score, a cell cycle score, a squamous cell score, a phyllodes tumor score, a normal-like score, a triple negative score, a HER2-like score, a HER2 amplification score, and a hormone sensitivity score.

Each "subtype differentiation score" can be determined by measuring the expression levels of genes included in the groups a to o. Here, the gene groups required to determine each "subtype differentiation score" are shown in Table 4 below.

[Table 4]

| Subtype differentiation score | Gene groups required for score calculation |
|---|---|
| Cancer score | Group c and group d |
| Cell cycle score | Group o |
| Squamous cell score | Group a |
| Phyllodes tumor score | Group b |
| Normal-like score | Group e |
| Triple negative score | Group f, group g, group h, and group n |
| HER2-like score | Group i |
| HER2 amplification score | Group j and group k |
| Hormone sensitivity score | Group l and group m |

The gene for which the expression level is measured to calculate the "subtype differentiation score" need only be a gene belonging to the gene group corresponding to each subtype differentiation score shown in the above-described table. The gene for which the expression level is measured need only be at least one gene included in each gene group, and is preferably two or more, three or more, four or more, five or more, six or more, seven or more, eight or more, nine or more, ten or more, 15 or more, or 20 or more. Even if the number of genes for which the expression levels are measured is one gene included in each gene group, the subtype differentiation score can be calculated and ultimately used to differentiate or classify the subtype of the test sample. In a case in which the number of genes for which the expression level is measured is one gene as described above, the task is simplified and the cost can be suppressed, which is preferable. On the other hand, as the number of genes for which the expression levels are measured increases, the accuracy of subtype differentiation or classification improves, which is preferable. In one preferred embodiment of the present invention, the genes for which expression levels are measured are all genes included in each gene group.

It should be noted that, in some cases, the differentiation marker gene set of the present invention does not include one or a plurality of specific genes in the above-described "differentiating gene group." In one embodiment, the differentiation marker gene set of the present invention does not include a specific gene used for differentiating or classifying a specific subtype (specific gene included in the gene group composed of the groups a to o). Here, "a specific gene used for differentiating or classifying a specific subtype" not being included means that the differentiation marker gene set does not include the specific gene in an aspect used for differentiating or classifying the specific subtype, but does not exclude that the specific gene is included in an aspect used for differentiating or classifying other subtypes. Further, in another embodiment, the differentiation marker gene set of the present invention does not include a specific gene (specific gene included in the gene group composed of the groups a to o).

The "subtype differentiation score" required for differentiating or classifying a breast cancer subtype differs depending on the desired subtype to be differentiated or classified, and one or a combination of a plurality of the subtype differentiation scores listed above may be used. Specifically, by using one or a combination of a plurality of subtype differentiation scores in Table 5 below for each breast cancer subtype, it is possible to differentiate or classify whether the test sample is the desired subtype.

[Table 5]

| Breast cancer subtype | Subtype differentiation scores required for differentiation of subtype |
|---|---|
| Luminal A | Hormone sensitivity score, cell cycle score, and HER2 amplification score |
| Luminal B (HER2 positive) | HER2 amplification score and hormone sensitivity score |
| Luminal B (HER2 negative) | Hormone sensitivity score, cell cycle score, and HER2 amplification score |
| HER2 positive | Hormone sensitivity score, HER2 amplification score, and HER2-like score |
| HER2 positive-like | HER2-like score and HER2 amplification score |
| Triple negative | Triple negative score |
| Phyllodes tumor | Phyllodes tumor score |
| Squamous cell carcinoma | Squamous cell score |

(continued)

| Breast cancer subtype | Subtype differentiation scores required for differentiation of subtype |
|---|---|
| Undeterminable | Cancer score and all other scores |
| Normal-like | Normal-like score and cancer score |
| Normal | Cancer score |

The differentiation marker gene set for differentiating or classifying a subtype of breast cancer, which is the first aspect of the present invention, includes a combination of genes obtained by selecting at least one gene from each gene group of at least one gene group selected from gene groups composed of the groups a to o. Here, at least one gene group selected from the gene group composed of the groups a to o is selected as appropriate in accordance with the desired subtype to be differentiated or classified.

As the "desired subtype to be differentiated or classified," one of the subtypes of breast cancer may be selected, or a plurality of the subtypes may be selected. When a plurality of the subtypes are selected as the "desired subtype to be differentiated or classified," there is no limit to the combination of subtypes, and all combinations of subtypes selected from the group composed of luminal A, luminal B (HER2 positive), luminal B (HER2 negative), HER2 positive, HER2 positive-like, triple negative, phyllodes tumor, squamous cell carcinoma, normal-like, normal, and undeterminable are included. Although not limited to the following examples, in one embodiment, for example, the combination of subtypes is the four subtypes of the luminal A and B group, the HER2+-like group, the HER2+ group, and the triple negative group and, at this time, the differentiation marker gene set is a combination of genes obtained by selecting at least one gene from each gene group of the group f, the group g, the group i, the group j, the group k, the group 1, the group m, the group n, and the group o. In such an embodiment, the sample to be differentiated or classified is a subtype of any of the luminal A and B group, the HER2+-like group, the HER2+ group, and the triple negative group, or the sample can be differentiated or classified as another subtype. Further, in yet another embodiment, all subtypes (11 subtypes) can be targeted as the "desired subtype to be differentiated or classified." At this time, the differentiation marker gene set is a combination of genes obtained by selecting at least one gene from each gene group of the groups a to o.

With selection of the "desired subtype to be differentiated or classified," the subtype differentiation score required to differentiate the subtype is determined with reference to the above-described Table 5, and the gene groups required to calculate the subtype differentiation score are selected from the groups a to o with reference to the above-described Table 4. The differentiation marker gene includes at least one gene belonging to each of the gene groups thus selected.

For example, in a case in which the "desired subtype to be differentiated or classified" is luminal A, the subtype differentiation scores are the hormone sensitivity score, the cell cycle score, and the HER2 amplification score. Accordingly, the gene groups required to calculate the subtype differentiation scores are the group 1 and the group m required to calculate the hormone sensitivity score, the group o required to calculate the cell cycle score, and the group j and the group k required to calculate the HER2 amplification score. As a result, at least one gene can be selected from each group of the group j, the group k, the group 1, the group m, and the group o and used as a differentiation marker gene.

The differentiation marker gene set according to the present invention, in one embodiment, includes a combination of genes obtained by selecting at least one gene from each gene group of 15 gene groups composed of the groups a to o. According to this embodiment, by measuring the expressions of the genes included in the differentiation marker gene set, it is possible to differentiate or classify a plurality of subtypes at one time.

In addition, the differentiation marker gene set according to the present invention, in one embodiment, can further include at least one gene selected from a control group composed of ABCF3, FBXW5, MLLT1, FAM234A, PITPNM1, WDR1, NDUFS7, and AP2A1. The genes included in this control group are genes newly discovered as genes for which significant variation in expression does not occur in any of the breast cancer subtypes, and can be suitably used as controls.

2. Method of Differentiating Subtype of Breast Cancer

2-1. Overview

Another aspect of the present invention is a method of differentiating or classifying a subtype of breast cancer in a test sample. It should be noted that the method of differentiating or classifying according to the present invention can be used in combination with a histopathological examination or the like and therefore, in one embodiment, can be called a method of assisting differentiation or classification.

The method of differentiating or classifying according to the present invention is a method of measuring the expression level of a differentiation marker included in a sample collected from a subject, and differentiating or classifying the subtype of breast cancer in the subject on the basis of an expression profile of the differentiation marker. The "expression profile of the differentiation marker" means information related to the expression level of each gene constituting the differentiation marker. In this specification, this expression profile particularly includes information related to the expression levels of a plurality of differentiating genes. In general, the larger the expression profile of the genes to be acquired, the more accurate the differentiation can become.

2-2. Measurement Method

The method of differentiating or classifying according to the present invention includes, as an essential step, a step of measuring the expression level of at least one gene included in the differentiating gene group. More specifically, the method of differentiating or classifying according to the present invention includes (a) a step of measuring, in a test sample, expression levels of genes included in the gene set for differentiating or classifying a subtype of breast cancer. Hereinafter, the measurement method will be specifically described.

The "measurement step" is a step of measuring the expression level of the differentiation marker in the test sample and obtaining the measured value. For the measurement of the expression of the differentiation marker, measuring the expression level per unit amount of each differentiation marker is preferred.

In this specification, the "test sample" is a sample collected from a subject and includes breast cancer tissue or tissue suspected to be breast cancer tissue or a portion thereof. Further, in this specification, the term "subject" refers to a human individual who provides a sample and is subjected to an examination. The subject may be either an individual having a history of breast cancer or an individual suspected of having breast cancer. The "individual having a history of breast cancer" referred to herein includes a patient currently suffering from breast cancer and a person having a history of breast cancer who has previously suffered from breast cancer. The subject in the method according to the present invention is preferably a subject having a history of breast cancer of a subtype that is difficult to differentiate or classify by a conventional histopathological examination.

In this specification, the "breast cancer patient having a desired subtype to be differentiated or classified" means a patient suffering from breast cancer belonging to a specific subtype to be differentiated or classified (for example, a patient suffering from luminal A-type breast cancer, a patient suffering from luminal B- (HER2 positive)-type breast cancer, or the like). In particular, for comparison with the test sample, the term may refer to the source that provides the sample, and samples derived from breast cancer belonging to specific subtypes can be obtained from patient suffering from breast cancer of these specific subtypes. The specific subtype to be differentiated or classified is not limited to one subtype, but may be a combination of two or more subtypes.

The "subjects" and "patients suffering from breast cancer of each subtype" used in this aspect are not particularly limited in terms of physical conditions such as gender, age, height, and weight, and the number of individuals is not particularly limited, but the "patients suffering from breast cancer of each subtype" to be compared preferably have the same physical conditions as or similar physical conditions to the subjects, such as age, height, and weight. It should be noted that, in this specification, a group composed of a plurality of "patients suffering from breast cancer of a specific subtype" is referred to as "a group of patients suffering from breast cancer of a specific subtype" (for example, a group composed of a plurality of patients suffering from luminal A-type breast cancer is referred to as "a group of patients suffering from luminal A-type breast cancer").

In this specification, the "sample" is a sample collected from the subject and used in the differentiation method of this aspect, and corresponds to, for example, a tissue, a cell, a body fluid, or a peritoneal washing. The "tissue" and "cell" referred to herein may be derived from any area of the subject, but are preferably specimens, more specifically, breast tissue or breast cells, collected by biopsy or excised by surgery. Breast cancer cells collected by biopsy, or breast cancer tissue or breast cancer cells suspected of having breast cancer, are particularly preferred. It should be noted that these tissues or cells may be formalin-fixed paraffin embedded (FFPE). Further, the term "body fluid" referred to herein means a liquid biological sample collected from a subject. Examples thereof include blood (including serum, plasma, and interstitial fluid), spinal fluid (cerebrospinal fluid), urine, lymph, digestive fluid, ascites, pleural fluid, perineural fluid, and extracts of tissues or cells. The preferred body fluid is blood.

Sample collection, for tissues or cells, may be carried out by biopsy or surgical removal. Further, for a body fluid, collection may be carried out on the basis of a method known in the field. For example, for blood or lymph, a known blood collection method need only be followed. The amount of the sample required for the differentiation or classification method of this aspect is not particularly limited. For tissues or cells, at least 10 μg, preferably at least 0.1 mg, is desirable. Further, the sample may be biopsy material. For body fluid such as blood or lymph, a volume of at least 0.1 mL, preferably at least 1 mL, and more preferably at least 10 mL, is sufficient. The sample can be prepared and treated as necessary so that the differentiation marker can be measured. If the sample is tissues or cells, examples include homogenizing treatment, cytolysis treatment, impurity removal by centrifugation or filtration, and addition of a protease inhibitor. Details of these treatments are described in Green & Sambrook, Molecular Cloning, 2012, Fourth Ed., Cold Spring Harbor Laboratory Press, which can be used as reference.

In this specification, the term "unit amount" refers to an arbitrarily determined amount of the sample. For example, volume (represented by μL or mL) and weight (represented by μg, mg, or g) are applicable. Although the unit amount is not particularly specified, it is preferable that the unit amount to be measured by the differentiation method in a series is constant. In this step, differentiation with higher accuracy is possible by keeping the unit amount of the sample derived from the subject to be used for measurement and the sample derived from the breast cancer patient of each subtype to be compared constant. In particular, when the expression level of the differentiation marker is to be measured as an absolute value, it is necessary to keep the unit amount constant.

Hereinafter, the measurement method of a transcription product of a gene will be specifically described. It should be noted that measurement methods of a transcription product of a gene are known. Hereinafter, description related to the measurement method of a transcription product or a translation product of a gene set forth in Japanese Laid-Open Patent Application No. 2016-13081 will be referenced or cited. It should be noted that, in the following, typical measurement methods of a transcription product or a translation product of a gene will be described, but the present invention is not limited to these methods, and a known measurement method can be used.

The measurement of a transcription product of a differentiating gene may be measurement of mRNA amounts or measurement of cDNA amounts obtained by reverse transcription of mRNA. In general, for measurement of a transcription product of a gene, a method of measuring the expression level of the gene as an absolute value or a relative value by using a nucleotide including all or a portion of the nucleotide sequence of the above-described gene as a primer or a probe is adopted.

The primer or the probe of this aspect is usually constituted by natural nucleic acids such as DNA and RNA. DNA is particularly preferable because of high stability and the ease of synthesis at an inexpensive cost. Further, natural nucleic acids can be combined with chemically-modified nucleic acids or pseudo-nucleic acids as necessary. Examples of chemically-modified nucleic acids and pseudo-nucleic acids include peptide nucleic acid (PNA), locked nucleic acid (LNA; registered trademark), methyl phosphonate DNA, phosphorothioate DNA, and 2'-O-methyl RNA. Further, the primers and the probes may be labeled or modified with fluorescent substances and/or quencher substances, or labeling substances such as radioactive isotopes (for example, 32P, 33P, or 35S), or a modifying substance such as biotin or (strept) avidin, or magnetic beads. The labeling substance is not limited, and commercially available products can be used. For example, as a fluorescent substance, FITC, Texas, Cy3, Cy5, Cy7, Cyanine3, Cyanine5, Cyanine7, FAM, HEX, VIC, fluorescamine and derivatives thereof, rhodamine and derivatives thereof, and the like can be used. As a quencher substance, AMRA, DABCYL, BHQ-1, BHQ-2, BHQ-3, and the like can be used. A position for labeling a primer or a probe with a labeling substance can be determined, as appropriate, depending on the properties or intended use of the modifying substance. In general, the 5' or 3' end is often modified. Further, a single primer or probe molecule may be labeled with one or more labeling substances. A nucleotide can be labeled with these substances by a known method.

A nucleotide used as a primer or probe may be any nucleotide composed of a sense strand or an antisense strand of each gene constituting the above-described differentiation marker.

A base length of a primer or a probe is not particularly limited. In the case of a probe, if used in a hybridization method described later, the base length thereof is from at least a 10-base length to the full-length of the gene, preferably from a 15-base length to the full-length of the gene, more preferably from a 30-base length to the full-length of the gene, and even more preferably from a 50-base length to the full-length of the gene. In the case of microarray use, the base length thereof is a 10- to 200-base length, preferably a 20- to 150-base length, and more preferably a 30- to 100-base length. In general, a longer probe results in higher hybridization efficiency and higher sensitivity. On the other hand, a shorter probe results in lower sensitivity, but conversely also results in higher specificity. On the other hand, in the case of a primer, each of a forward primer and a reverse primer may have a length of 10 to 50 bp, preferably 15 to 30 bp.

Preparation of the primer or the probe described above is known to those skilled in the art and can be performed, for example, according to the method described in Green & Sambrook, Molecular Cloning (2012) mentioned above. Further, it is also possible to provide a contracted manufacturer for nucleic acid synthesis with sequence information and entrust the manufacturer with manufacturing.

Measurement of the transcription product of the differentiating gene may be performed by a known nucleic acid detection and quantification method, and the method is not particularly limited. Examples include a hybridization method, a nucleic acid amplification method, or an RNA sequencing (RNA-Seq) analysis method.

The "hybridization method" is a method of detecting and quantifying a target nucleic acid or a fragment thereof by using, as a probe, a nucleic acid fragment having a nucleotide sequence complementary to all or a portion of the nucleotide sequence of a target nucleic acid to be detected, and by utilizing base pairing between the nucleic acid and the probe. In this aspect, the target nucleic acids correspond to mRNAs or cDNAs of each gene constituting the differentiation markers or a fragment thereof. In general, the hybridization method is preferably performed under stringent conditions to eliminate non-target nucleic acids nonspecifically hybridized. The highly stringent conditions mentioned above at a low salt concentration and a high temperature are more preferable. As the hybridization method, several methods involving different detection means are known and, for example, a Northern blot method (Northern hybridization method), a microarray method, a surface plasmon resonance method, or a quartz crystal microbalance method is preferable.

The "Northern blot method" is one method of analyzing gene expression, and is a method in which total RNA or mRNA prepared from a sample is separated by electrophoresis through agarose gel, polyacrylamide gel, or the like under denatured conditions and transferred (blotted) on a filter, and then a target nucleic acid is detected by using a probe having a nucleotide sequence specific to a target RNA. It is also possible to quantify a target nucleic acid by labeling the probe with a suitable marker such as a fluorescent dye or a radioactive isotope, and by using, for example, a measurement device such as a chemiluminescence imaging analyzer (for example, Light Capture; ATTO Corporation), a scintillation counter, or an imaging analyzer (for example, Fujifilm Corporation: BAS series). The Northern blot method is a well-known, prominent technique in the field and, for example, reference need only be made to Green, M.R. and Sambrook, J. (2012) mentioned above.

The "microarray method" is a method of detecting a nucleic acid hybridized to a substrate spot by fluorescence or the like by allowing a sample including a target nucleic acid to react on a microarray or microchip in which a nucleic acid fragment complementary to all or a portion of the nucleotide sequence of the target nucleic acid as a probe is disposed as a small spot at a high density on a substrate and solid-phased. The target nucleic acid may be RNA, such as mRNA, or DNA, such as cDNA. Detection and quantification can be achieved by detecting and measuring fluorescence or the like based on the hybridization of the target nucleic acid or the like with a microplate reader or a scanner. The measured fluorescence intensity can be used to determine the mRNA amount or cDNA amount or an abundance ratio thereof with respect to reference mRNA. The microarray method is also a well-known technique in the field. For example, reference need only be made to the DNA microarray method (*DNA Maikuroarei to Saishin PCR Hou* (DNA Microarray and the Latest PCR Methods) (2000), by Masaaki Muramatsu and Hiroyuki Nawa, Shujunsha Co., Ltd.) and the like.

The "surface plasmon resonance (SPR) method" is a method of detecting and quantifying with extreme high sensitivity a substance adsorbed on the surface of a thin metal film by utilization of the so-called surface plasmon resonance phenomenon in which as the thin metal film is irradiated with laser beam at varying angles of incidence, reflected light intensity remarkably attenuates at a particular angle of incidence (resonance angle). In the present invention, for example, a probe having a sequence complementary to the nucleotide sequence of the target nucleic acid is immobilized on a thin film metal surface, another thin metal film surface portion is blocked. Subsequently, a sample collected from a subject or a healthy body or a healthy body group is distributed on the thin metal film surface, thereby forming a base pairing between the target nucleic acid and the probe. The target nucleic acid can then be detected and quantified from the difference in the measured values before and after sample distribution. The detection and quantification by the surface plasmon resonance method can be performed by using an SPR sensor commercially available from Biacore, for example. This technique is well-known in the field. Reference can be made to, for example, Kazuhiro Nagata and Hiroshi Handa, Real-Time Analysis of Biomolecular Interactions, Springer Fairlark Tokyo, Tokyo, 2000.

The "quartz crystal microbalance (QCM) method" is a mass measurement method of quantitatively identifying an exceedingly small amount of an absorbed substance on the basis of the amount of change in resonance frequency by utilization of the phenomenon in which the resonance frequency of a quartz crystal decreases in accordance with the mass of the substance adsorbed onto the surface of electrodes attached to a quartz crystal resonator. Similar to the SPR method, detection and quantification by this method can also be performed by utilizing a commercially available QCM sensor and, for example, the target nucleic acid can be detected and quantified by base pairing a probe having a sequence complementary to the nucleotide sequence of the target nucleic acid and immobilized on the electrode surface and a target nucleic acid in a sample collected from a subject or a healthy body or a healthy body group. This technique is well-known in the field, and reference can be made to, for example, J. Christopher Love, et al., 2005, Self-Assembled Monolayers of a Form of Nanotechnology, Chemical Review, 105: 1103-1169, and Toyosaka Moriizumi and Takamichi Nakamoto, (1997), Sensa Kougaku (Sensor Engineering), Shokodo Co., Ltd.

The term "nucleic acid amplification method" refers to a method of amplifying a specific region of a target nucleic acid by nucleic acid polymerases by using forward/reverse primers. Examples include a PCR method (including a RT-PCR method), an NASBA method, an ICAN method, and a LAMP (registered trademark) method (including an RT-LAMP method). Preferably, the method is the PCR method. As a method of measuring a transcription product of a gene using the nucleic acid amplification method, a quantitative nucleic acid amplification method such as a real-time RT-PCR method is used. Further, as the real-time RT-PCR method, an intercalator method using SYBR (registered trademark) Green or the like, a TaqMan (registered trademark) probe method, a digital PCR method, and a cycling probe method are known, and any of these methods can be used. Any of these is a known method and described in appropriate protocol in the art, and thus reference can be made thereto.

The term "RNA sequencing (RNA-Seq) analysis method" refers to a method of measuring the expression level of a gene by converting RNA into cDNA by a reverse transcription reaction, and using next-generation sequencers (for example, HiSeq series (Illumina) and an Ion Proton system (Thermo Fisher), but not limited thereto) to count the number of reads. Any of these is a known method and described in appropriate protocol in the art, and thus reference can be made thereto.

A method of quantifying the transcription product of a gene by the real-time RT-PCR method will be briefly described below with an example. The real-time RT-PCR method is a method of quantifying a nucleic acid by PCR using a temperature cycler system provided with a function for detecting fluorescence intensity derived from an amplification product in a reaction system in which a PCR amplification product is specifically fluorescence-labeled using, as a template, cDNA prepared from mRNA in a sample by a reverse transcription reaction. The amount of the amplification product of the target nucleic acid in the reaction is monitored in real-time, and regression analysis of the results is performed by a computer. Methods of labeling the amplification product include a method using a fluorescence-labeled probe (for example, the TaqMan (registered trademark) PCR method) and an intercalator method using a reagent that specifically binds to double-stranded DNA. The TaqMan (registered trademark) PCR method is a method using a probe modified with a quencher substance at the 5' end and a fluorescent dye at the 3' end. Normally, the quencher substance at the 5' end suppresses the fluorescent dye at the 3' end. However, as a result of PCR, the probe is degraded due to 5'->3' exonuclease activity of the Taq polymerase, which releases the suppression by the quencher substance, resulting in the emission of fluorescence. The fluorescence amount reflects the amount of the amplification product. The number of cycles (CT) when the amplification product reaches the detection limit and the initial template amount are inversely correlated, and thus the initial template amount is quantified by measuring CT in the real-time measurement method. An absolute value of the initial template amount of an unknown sample can be calculated with a calibration curve created by measuring CT using a template of known amounts of several stages. As a reverse transcriptase used in RT-PCR, for example, M-MLV RTase, ExScript RTase (TaKaRa), and Super Script II RT (Thermo Fisher Scientific) can be used.

The reaction conditions of real-time PCR generally vary depending on the base length of the nucleic acid fragment to be amplified, the amount of a nucleic acid for a template, the base lengths and Tm values of the primers to be used, the optimum reaction temperature and optimum pH of the nucleic acid polymerase to be used, and the like, and therefore need only be determined as appropriate based on the known PCR method in accordance with these conditions. As an example, normally an elongation reaction can be carried out by repeating about 15 to 40 cycles including, as one cycle, a denaturation reaction at 94° C to 95° C for five seconds to five minutes, an annealing reaction at 50° C to 70° C for ten seconds to one minute, and an elongation reaction at 68° C to 72° C for 30 seconds to three minutes. In a case in which a kit commercially available from a manufacturer is used, in principle, the protocol provided with the kit need only be followed.

The nucleic acid polymerase used in real-time PCR is a DNA polymerase, particularly a heat-resistant DNA polymerase. Such a nucleic acid polymerase is commercially available in various kinds, and these commercially available products can be used. Examples include Taq DNA polymerase provided with the Applied Biosystems TaqMan MicroRNA Assays Kit (Thermo Fisher Scientific). In particular, such a commercially available kit is useful because a buffer optimized for the activity of the provided DNA polymerase or the like is provided therewith.

2-3. Method of Differentiating or Classifying

The differentiation method of the present invention is a method of differentiating or classifying the subtype of breast cancer to which the test sample belongs on the basis of the expression level of the differentiation marker measured as described above. That is, the method of differentiating or classifying according to the present invention includes (b) a step of differentiating or classifying whether the test sample is a desired subtype to be differentiated or classified from the expression levels of the genes included in the marker gene set measured.

Differentiation markers are genes characteristic of each subtype of breast cancer, and make it possible to differentiate or classify the subtype of breast cancer to which the test sample belongs on the basis of the expression profile of the gene set obtained by combining these genes.

Here, one embodiment of the method of differentiating or classifying according to the present invention includes, in the step (b), differentiation or classification of the subtype of the test sample by acquiring an expression profile of the gene set from the expression levels of the genes measured, and comparing the expression profile thus acquired and an expression profile of a corresponding gene set in a sample derived from a breast cancer patient having the desired subtype to be differentiated or classified.

As the expression profile of each gene included in the gene set in the sample derived from a breast cancer patient having a desired subtype to be differentiated or classified, which is to be compared with the test sample, a pre-measured profile may be used, or a profile acquired by measuring the expression level of each gene included in a gene set of a sample derived from a breast cancer patient having the desired subtype to be newly differentiated or classified may be used.

Accordingly, the method of differentiating or classifying according to the present invention, in one embodiment, further includes a step of measuring expression levels of each gene included in a gene set for differentiating or classifying a subtype of breast cancer in the sample derived from a breast cancer patient having the desired subtype to be differentiated or classified. The expression profile acquired by this step can be compared with the expression profile of the test sample. The sample derived from a breast cancer patient having the desired subtype to be differentiated or classified may be a sample derived from one individual or may include samples derived from two or more individuals. As the number of individuals from whom samples are derived increases, the individual differences of the samples can be further averaged, increasing the accuracy of differentiation, which is thus preferred.

In another embodiment of the method of differentiating or classifying according to the present invention, in the step (b), the expression profile thus acquired and the expression profile of the corresponding gene set in the sample derived from a breast cancer patient having the desired subtype to be differentiated or classified are compared, and the test sample can be evaluated as being breast cancer of the subtype thus compared when having an expression profile equivalent to the expression profile of the sample thus compared, or can be evaluated as not being breast cancer of the subtype thus compared when having an expression profile of genes different from the expression profile of the sample thus compared.

For example, when the expression profiles of the test sample and the sample derived from a luminal A-type breast cancer patient are compared and it is determined that the samples have equivalent gene expression profiles, the test sample can be differentiated or classified as breast cancer belonging to the luminal A type. On the other hand, when the expression profiles of the test sample and the sample derived from a luminal A-type breast cancer patient are compared and it is determined that the samples have different gene expression profiles, the test sample can be differentiated or classified as breast cancer not belonging to the luminal A type.

Here, "have equivalent gene expression profiles" means that the expression profiles of each gene included in the gene set for differentiating or classifying the subtype of breast cancer are similar. Further, "have different gene expression profiles" means that the expression profiles of each gene included in the gene set for differentiating or classifying the subtype of breast cancer are not similar.

As a specific technique of determining whether the expression profiles of each gene in the gene set are equivalent or different, a known method can be adopted. Although not limited to the following, examples include (i) a method of classifying a test sample into breast cancer of a desired subtype to be differentiated or classified on the basis of hierarchical cluster analysis, (ii) a method of evaluation by comparison of expression levels of genes, and (iii) a method of differentiating whether the test sample belongs to a desired subtype to be differentiated or classified by setting a threshold value.

(i) Hierarchical cluster analysis

The method of differentiating or classifying according to the present invention is, in one embodiment, a method of differentiating or classifying a subtype of a test sample by cluster analysis of the expression profile of each gene included in the differentiation marker gene set of the test sample.

More specifically, hierarchical cluster analysis can be performed by comparing the expression profile of the gene set measured in the test sample with the expression profile of a corresponding gene set in a sample derived from a patient with breast cancer belonging to the subtype to be differentiated or classified. When a subtype of a test sample is differentiated or classified by hierarchical cluster analysis, in addition to the expression profile of the gene set in the test sample and the expression profile of the gene set in the sample derived from the patient with breast cancer belonging to the subtype to be differentiated or classified, expression profiles of gene sets in samples derived from patients with breast cancer belonging to subtypes other than the subtype to be differentiated or classified is required so that hierarchical clusters can be created. The samples derived from patients with breast cancer belonging to subtypes other than the subtypes to be differentiated or classified are subtypes other than the subtype to be differentiated or classified, and are samples derived from patients with breast cancer belonging to subtypes selected from a group composed of luminal A, luminal B (HER2 positive), luminal B (HER2 negative), HER2 positive, HER2 positive-like, triple negative, phyllodes tumor, squamous cell carcinoma, normal-like, normal, and undeterminable. The samples derived from patients with breast cancer belonging to subtypes other than the subtype to be differentiated or classified may be samples derived from two or more (three, four, five, six, seven, eight, nine, ten, or 11) patients with breast cancer belonging to different subtypes. In a case in which two or more samples derived from patients with breast cancer belonging to subtypes other than the subtype to be differentiated or classified are used, preferably the samples are an embodiment in which samples derived from patients with breast cancer belonging to all subtypes.

As the technique of hierarchical cluster analysis, a known technique can be adopted. A particularly preferred embodiment of the present invention is cluster analysis by a group-average method based on the Euclidean distance. For cluster analysis, known software can be used and, for example, while not limited to the following, Expression View Pro software (MicroDiagnostic, Tokyo, Japan) can be used as commercially available software.

By performing hierarchical cluster analysis, it is possible to draw a hierarchical structure (tree diagram) composed of test samples and samples derived from patients with breast cancer belonging to the subtype to be differentiated or classified, and divide the samples into clusters for each subtype. As a result, it is possible to confirm the cluster of the subtype into which the test sample is classified, and differentiate or classify the subtype to which there is a high possibility of the sample belonging.

(ii) Method of evaluation by comparison of expression levels of genes

Further, in one embodiment, the histological type of the test sample can be evaluated by comparing the total value of the expression levels of the genes in the combination of each gene included in the gene set in the test sample and the total value of the expression levels of the genes in the sample derived from the patient with breast cancer belonging to the subtype to be differentiated or classified.

Although not limited to the following, the present invention will be described with reference to one embodiment. The total values of the expression levels of the genes included in the gene set of a group of patients suffering from breast cancer belonging to the subtype to be differentiated or classified and a group of patients suffering from breast cancer belonging to subtypes other than the subtype to be differentiated or classified subtype are respectively plotted as a group scatter diagram, and the position where the total value of the expression levels of the genes of the gene set in the test sample is plotted is checked. The plotted positions can be used to assess the subtype of breast cancer to which there is a high possibility of the test sample belonging.

It should be noted that the gene set and the genes included therein are selected in accordance with the subtype as genes characteristic of the desired subtype to be differentiated or classified, and therefore the group of patients suffering from breast cancer belonging to subtypes other than the subtype to be differentiated or classified, which is the comparison control, may be a group of patients suffering from breast cancer belonging to any subtype as long as a subtype other than the subtype to be differentiated or classified. Preferably, the group is a group of patients suffering from breast cancer belonging to the normal subtype. Further, in one embodiment, the samples derived from patients with breast cancer belonging to subtypes other than the subtype to be differentiated or classified, which is used as the comparison control, are two or more (three, four, five, six, seven, eight, nine, ten, or 11) samples derived from patients with breast cancer belonging to different subtypes and, in a more preferred embodiment, are samples derived from patients with breast cancer belonging to all subtypes.

It should be noted that, when the total value of the expression levels of the genes is used for differentiation, the values of the obtained expression levels of the MBOAT1 gene, PADI2 gene, and PPP1R1B gene are used after being inverted. For example, when the total value of the expression level of each gene obtained by a method such as microarray is utilized, the expression levels of the MBOAT1 gene, PADI2 gene, and PPP1R1B gene (for example, Log2 ratio with respect to a common reference) are multiplied by -1 to find the inverted values, and the total value of the inverted values and the expression levels of the other genes is calculated.

(iii) Method of differentiating or classifying by setting threshold value

Further, in one embodiment, the histological type can be differentiated by comparing the expression profile of the gene set of a test sample with a predetermined threshold value.

Here, the term "predetermined threshold value" refers to a predetermined cutoff value based on the expression profile of the differentiation marker in the sample derived from the breast cancer patient group belonging to the desired subtype to be differentiated or classified. The cutoff value can be set as follows, for example, but is not limited thereto. That is, the expression levels of genes included in the gene sets in samples derived from a breast cancer patient group (discriminant patient group) belonging to a desired subtype to be differentiated or classified and a breast cancer patient group (control patient group) belonging to a subtype other than the subtype to be differentiated or classified are measured, and the expression levels of the genes are calculated for each sample. Next, a predetermined cutoff value can be derived by creating a receiver operating characteristic curve (ROC) curve from the values of the expression levels of the genes thus obtained. By setting a cutoff value, it is possible to differentiate the histological type to which there is a high possibility of the ovarian cancer belonging by whether or not the cutoff value is exceeded.

In one embodiment, a group of patients suffering from breast cancer (control patient group) belonging to a subtype other than the subtype to be differentiated or classified may be a group of patients suffering from breast cancer belonging to any subtype as long as a subtype other than the subtype to be differentiated or classified. Further, in another embodiment, as the samples derived from patients with breast cancer belonging to subtypes other than the subtype to be differentiated or classified, two or more (three, four, five, six, seven, eight, nine, ten, or 11), preferably all, samples derived from patients with breast cancer belonging to different subtypes are used as comparison controls.

As a more specific embodiment, when cutoff values for genes belonging to the groups a to o are set, it is possible to create an ROC curve upon comparison of the gene expression levels between the following two groups (discriminant patient group and control patient group), and set the cutoff value. Nevertheless, the setting of the cutoff value is not limited to the following embodiment.

Group a: A group with a high expression of genes included in the gene group (group a) showing an expression pattern characteristic of squamous cell carcinoma can be set as the discriminant patient group for "squamous cell carcinoma," and a group with a low expression of the genes can be set as the control patient group for "non-squamous cell carcinoma."

Group b: A group with a high expression of genes included in the gene group (group b) showing an expression pattern characteristic of phyllodes tumor can be set as the discriminant patient group for "phyllodes tumor," and a group with a low expression of the genes can be set as the control patient group for "non-phyllodes tumor."

Group c: A group having normal tissue with a low expression of genes included in the gene group (group c) showing an expression pattern characteristic of cancer can be set as the control patient group for "non-cancer," and a group having breast cancer tissue belonging to any subtype other than that of normal tissue with a high expression of the genes can be set as the discriminant patient group for "cancer."

Group d: A group having normal tissue with a high expression of genes included in the gene group (group d) showing an expression pattern characteristic of normal tissue can be set as the discriminant patient group for "normal," and a group having tissue other than normal tissue with a low expression of the genes can be set as the control patient group for "non-normal." (It should be noted that the normal-like group that resembles normal and the group lacking characteristics are not included in either "normal" or "non-normal.")

Group e: A group with a high expression of genes included in the gene group (group e) showing an expression pattern characteristic of normal-like can be set as the discriminant patient group for "normal-like," and a group of luminal A, luminal B, HER2 amplification+, HER2-like, or triple negative with a low expression of the genes can be set as the control patient group for "non-normal-like."

Groups f, g, h: A triple negative group with a high expression of genes included in the gene group (group f) showing an expression pattern characteristic of triple negative and showing an expression pattern characteristic of normal tissue or normal-like, the gene group (group g) showing an expression pattern characteristic of triple negative, and the gene group (group h) showing an expression pattern characteristic of triple negative and similar to the expression pattern of genes defined as undeterminable can be set as the discriminant patient group for "TNBC," and a group of luminal A, luminal B, HER2 amplification+, and HER2-like with a low expression of the genes can be set as the control patient group for "non-TNBC."

Group i: A group of HER2-like and HER2 amplification+ with a high expression of genes included in the gene group (group i) showing an expression pattern characteristic of HER2+-like can be set as the discriminant patient group for "HER2-like," and a group of luminal A, luminal B, and triple negative with a low expression of the genes can be set as the control patient group for "non-HER2-like."

Groups j, k: A group of HER2 amplification+ and luminal B with a high expression of genes included in the gene group (group j) related to HER2 amplification and positioned close to the HER2 gene on the chromosome, or in a gene group (group k) related to HER2 amplification and other than the group j can be set as the discriminant patient group for "amplification," and a luminal A group, a HER2-like group, and a triple negative group with a low expression of the genes can be set as the control patient group for "no amplification." At this time, the group "amplification" may have variations in the expression of the genes included in the group j and the group k, and preferably a group with a high expression of at least five genes included in the group j and the group k is adopted.

Groups 1, m: A group of luminal A or luminal B with a high expression of genes included in a hormone sensitivity-related gene group (group 1) or ESR1 genes (group m) can be set as the discriminant patient group for "hormone sensitivity," and a HER2 amplification+ group, a HER2-like group, and a triple negative group with a low expression of the genes can be set as the control patient group for "no hormone sensitivity."

Group n: A group of luminal A, luminal B, HER2 amplification+, and HER2-like with a high expression of genes included in the differentiation-related gene group (group n) can be set as the discriminant patient group for "differentiated," and a group of triple negative with a low expression of the genes can be set as the control patient group for "undifferentiated."

Group o: A "fast-growth group" with a high expression of genes included in the cell cycle-related gene group (group o) can be set as the discriminant patient group, and a "slow-growth group" with a low expression of the genes can be set as the control patient group.

It should be noted that, for the discriminant patient groups and the control patient groups, the results of respective classification by cluster analysis may be used. For example, in the group e, the patient group classified as "normal-like" by cluster analysis can be adopted in the discriminant patient group of "normal-like", and the patient group classified as luminal A, luminal B, HER2 amplification+, HER2-like, and triple negative by cluster analysis can be adopted in the control patient group of "non-normal-like".

A "receiver operating characteristic (ROC) curve" is created by plotting with a vertical axis representing the true position fraction (TPF), that is, sensitivity, and a horizontal axis representing the false position fraction (FPF), that is, (1-specificity), while changing the cutoff point as a parameter, which represents the threshold value for determining the result of the test as positive. Specificity means a rate at which a negative subject is accurately determined to be negative.

The method of setting the cutoff value from the created ROC curve can basically be set to increase both sensitivity and specificity (to approach 1). For that purpose, the cutoff value need only be set to a value giving a point closest to the point (0, 1) on the ROC curve. In the most preferred embodiment, a cutoff value is set to a value that can clearly differentiate a sample derived from a group of patients suffering from breast cancer belonging to a subtype to be differentiated or classified and a sample derived from all breast cancer patient groups belonging to subtypes other than the subtype to be differentiated or classified.

When a predetermined threshold value is set as described above, the comparison between the threshold value and the expression profile of the gene set in the sample derived from the subject need only be a comparison between the threshold value and the total value of the expression levels of the genes in the gene set for differentiating or classifying the predetermined subtype in the test sample.

(iv) Method of differentiation or classification on the basis of subtype differentiation score

Further, in one embodiment, the subtype of breast cancer can be differentiated or classified on the basis of a subtype differentiation score.

As mentioned above, by using one subtype differentiation score or a combination of a plurality of subtype differentiation scores for each breast cancer subtype, it is possible to differentiate or classify whether the test sample is the desired subtype (above-described Table 5).

Here, the subtype differentiation score can be determined by measuring the expression levels of genes included in the appropriate groups from the differentiating gene groups of the groups a to o, in accordance with the above-described Table 4. More specifically, each subtype differentiation score can be calculated by the following equations (I) to (IX):

$$
\begin{aligned}
\text{Cancer score} = (&\text{c x "Regression coefficient calculated by multiple logistic regression} \\
&\text{analysis in group c" - d x "Regression coefficient calculated by multiple logistic regression} \\
&\text{analysis in group d")} \div (\text{"Regression coefficient calculated by multiple logistic regression} \\
&\text{analysis in group c" + "Regression coefficient calculated by multiple logistic regression} \\
&\text{analysis in group d")} \qquad \text{(I)}
\end{aligned}
$$

$$
\text{Cell cycle score} = \text{o} \qquad \text{(II)}
$$

$$
\text{Squamous cell score} = \text{a} \qquad \text{(III)}
$$

$$
\text{Phyllodes tumor score} = \text{b} \qquad \text{(IV)}
$$

$$
\text{Normal-like score} = \text{e} \qquad \text{(V)}
$$

$$
\begin{aligned}
\text{Triple negative score} = (&\text{f x "Regression coefficient calculated by multiple logistic} \\
&\text{regression analysis in group f" + g x "Regression coefficient calculated by multiple logistic} \\
&\text{regression analysis in group g" + h x "Regression coefficient calculated by multiple logistic} \\
&\text{regression analysis in group h" - n x "Regression coefficient calculated by multiple logistic} \\
&\text{regression analysis in group n")} \div (\text{"Regression coefficient calculated by multiple logistic} \\
&\text{regression analysis in group f" + "Regression coefficient calculated by multiple logistic} \\
&\text{regression analysis in group g" + "Regression coefficient calculated by multiple logistic} \\
&\text{regression analysis in group h" + "Regression coefficient calculated by multiple logistic} \\
&\text{regression analysis in group n")} \qquad \text{(VI)}
\end{aligned}
$$

$$\text{HER2-like score} = I \qquad \qquad \text{(VII)}$$

$$\text{HER2 amplification score} = (j \times \text{"Regression coefficient calculated by multiple logistic regression analysis in group } j\text{"} + k \times \text{"Regression coefficient calculated by multiple logistic regression analysis in group } k\text{"}) \div (\text{"Regression coefficient calculated by multiple logistic regression analysis in group } j\text{"} + \text{"Regression coefficient calculated by multiple logistic regression analysis in group } k\text{"}) \qquad \text{(VIII)}$$

$$\text{Hormone sensitivity score} = (l \times \text{"Regression coefficient calculated by multiple logistic regression analysis in group } l\text{"} + m \times \text{"Regression coefficient calculated by multiple logistic regression analysis in group } m\text{"}) \div (\text{"Regression coefficient calculated by multiple logistic regression analysis in group } l\text{"} + \text{"Regression coefficient calculated by multiple logistic regression analysis in group } m\text{"}) \qquad \text{(IX)}$$

(In the general equations (I) to (IX), a to o respectively mean "gene expression scores of the groups a to o.")

Here, the "gene expression score of the groups a to o," in one embodiment, can be calculated by comparison with the cutoff value. For example, the score can be set to "1" in a case in which the gene expression is higher than the cutoff value, and to "-1" in a case in which the gene expression is lower than the cutoff value. In a case in which a plurality of genes are selected from each group of the groups a to o, the average value of the scored values (maximum value is 1, and minimum value is -1) can be calculated and used for each group.

As described above, in one embodiment, the subtype differentiation score can be calculated using the "gene expression scores of the groups a to o." At this time, the values can be scored using 1 as the maximum value and -1 as the minimum value, and a higher score indicates a higher possibility of the corresponding subtype.

When a plurality of differentiating gene groups are used to calculate the subtype differentiation score (for example, calculation of the triple negative score), for example, the regression coefficients calculated by multiple logistic regression analysis can be multiplied by the average value of the gene expression scores of each differentiating gene group and then that value can be divided by the sum of the regression coefficients so that the maximum value is 1 and the minimum value is -1.

After the subtype differentiation score is determined, the histological type of breast cancer can be differentiated as follows:

(1) The highest score of the scores of "triple negative," "HER2 amplification," "hormone sensitivity," "HER2+-like," "phyllodes tumor," and "normal-like" is found.

(2) If "triple negative" has the highest score of the six scores and the score is high (for example, a score higher than 0.2), the type is determined to be "TNBC."

(3) If "HER2 amplification" has the highest score of the six scores and the score is high (for example, a score higher than 0.2), and the score of "hormone sensitivity" is high (for example, a score higher than 0), the type is determined to be "luminal B-HER2+." If the score of "HER2 amplification" is the highest and a high score (for example, a score higher than 0.2), and the score of "hormone sensitivity" is low (for example, a score of 0 or lower), the type is determined to be "HER2+."

(4) If "hormone sensitivity" has the highest score of the six scores and the score is high (for example, a score higher than 0), and the score of "HER2 amplification" is high (for example, a score higher than 0.2), the type is determined to be "luminal B-HER2+." If the score of "hormone sensitivity" is the highest and a high score (for example, a score higher than 0), and the score of "HER2 amplification" is low (for example, a score of 0.2 or lower), the type is determined to be "luminal A (provisional)." Among "luminal A (provisional)," if the score of "cell cycle" is high (for example, a score higher than 0), the type is determined to be "luminal B-HER2-," and if the score of "cell cycle" is low (for example, a score of 0 or lower), the type is determined to be "luminal A."

(5) If "HER2+-like" has the highest score of the six scores and the score is high (for example, a score higher than 0.2), and the score of "HER2 amplification" is high (for example, a score higher than 0.2), the type is determined to be "HER2+." If the score of "HER2+-like" is the highest and a high score (for example, a score of 0.2 or higher), and the score of "HER2 amplification" is low (for example, a score of 0.2 or lower), the type is determined to be "HER2+-like."

(6) If "phyllodes tumor" has the highest score of the six scores and the score is high (for example, a score higher than 0.1), the type is determined to be "phyllodes tumor."

(7) If "normal-like" has the highest score of the six scores and the score is high (for example, a score higher than 0.1), the type is determined to be "normal-like."

(8) Among the cases that do not belong to any of the above types based on each determination up to (7), for a case in which both the "cancer" and "cell cycle" scores are low (for example, 0 or lower), the type is determined to be "normal-like."

(9) For a case that does not belong to any of the above types based on each determination up to (8), the type is determined to be "undeterminable."

(10) For a case in which the score of "squamous cell carcinoma" is high (for example, 0.2 or higher), "squamous cell carcinoma" is also added to the subtype determination described above.

Here, "a subtype differentiation score is high" basically means that the score exceeds 0 when scored with the maximum value being 1 and the minimum value being -1. "A subtype differentiation score is low" basically means that the score is less than 0 when scored with the maximum value being 1 and the minimum value being -1. At this time, the closer each subtype differentiation score is to 1, the higher the possibility of that subtype, and the closer to -1, the higher the possibility of not being that subtype. As illustrated in the example described above, the criteria for "high score" or "low score" can be set as appropriate. By setting the criteria closer to the maximum value 1 (upper limit) side or the minimum value -1 (lower limit) side from 0, it is possible to lower the pseudo-positive rate at the time of differentiation or classification of the breast cancer subtype.

Further, the method of differentiating the histological type of breast cancer using the subtype differentiation score is not limited to the method described above, and may be set as appropriate according to the types and number of subtype differentiation scores to be used.

2-3. Advantageous Effects

According to the differentiation or classification method of this aspect, by examining a specimen removed by biopsy or surgery, it is possible to differentiate or classify the subtype of breast cancer to which the specimen belongs. With the differentiation method of this aspect having a high accuracy, it is possible to diagnose the subtype to which the breast cancer belongs, resulting in the advantage that action can be taken in consideration of recurrence risk and treatment method determination.

3. Kit for Differentiating or Classifying Subtype of Breast Cancer to Which Test Sample Derived from Breast Cancer Patient Belongs

3-1. Overview

Another aspect of the present invention is a reagent (differentiating reagent) for differentiating or classifying the subtype to which the breast cancer belongs. By applying the differentiating reagent of this aspect to, for example, a sample derived from a subject suffering from breast cancer, it is possible to differentiate the subtype to which the breast cancer of the subject belongs.

3-1-1. Configuration

The differentiating reagent of this aspect includes a set of probes or primers for detecting transcription products, that is, mRNAs or cDNAs, of a differentiating gene group constituting differentiation markers. A specific configuration thereof is described in the section on the measurement step. For example, in a case in which the transcription products of a differentiating gene group of four kinds of genes constituting differentiation markers are to be detected, the differentiating kit may include a group of four kinds of probes capable of detecting the transcription products of the corresponding genes.

In a case in which the differentiating reagent of this aspect are probes such as described above, the differentiating reagent can also be provided in a state of a DNA microarray or a DNA microchip in which each probe is immobilized on a substrate. Although material of the substrate for immobilizing each probe is not limited, a glass plate, a quartz plate, a silicon wafer, or the like is usually used. Examples of the size of the substrate include 3.5 mm $\times$ 5.5 mm, 18 mm $\times$ 18 mm, and 22 mm $\times$ 75 mm, which can be set variously depending on the number of spots and spot sizes for each probe.For a probe, 0.1 $\mu$g to 0.5 $\mu$g of nucleotides are usually used per spot. Examples of a method of immobilizing nucleotides include a method in which nucleotides are electrostatically bound to a solid-phase carrier surface-treated with a polycation such as polylysine, poly-L-lysine, polyethyleneimine, or polyalkylamine with the use of charges of nucleotides, and a method in which nucleotides, into which a functional group such as an amino group, an aldehyde group, an SH group, or biotin has been introduced, are covalently bound to the surface of a solid phase, onto which a functional group such as an amino group, an aldehyde group, or an epoxy group has been introduced.

3-2. Advantageous Effects

By using the differentiating kit of this aspect and applying the kit to a subject having a history of breast cancer, it is possible to objectively and accurately differentiate the subtype to which the breast cancer belongs.

The detection kit of the present invention may include other reagents necessary for the detection of a differentiation marker, such as, for example, a buffer and a secondary antibody, and instructions for detection and differentiation of results.

4. Treatment Method Based on Results of Differentiation or Classification

As another aspect, the present invention provides a treatment method in which an anticancer drug is administered to a subject for whom breast cancer has been differentiated or classified as belonging to a subtype by the differentiation method described above, based on the result of differentiation or classification.

That is, an effective amount of an anticancer drug effective for breast cancer belonging to a specific subtype is administered to a subject for whom breast cancer has been differentiated or classified as belonging to the specific subtype by the differentiation method of the present invention.

Anticancer drugs (for example, paclitaxel, cisplatin, carboplatin, or docetaxel) and combinations thereof, administration methods, dosages, and the like effective for breast cancer belonging to each subtype are known, and those skilled in the art can implement chemotherapy in accordance with the histological type, as appropriate.

Hereinafter, the present invention will be described in more detail with reference to examples, but is not limited to the following embodiments.

Examples

(Example 1. Preparation of RNA)

For breast cancer tissue collected surgically, total RNA was extracted using ISOGEN (Nippon Gene Co., Ltd., Tokyo, Japan). Further, normal mammary gland tissue and a portion of breast cancer tissue were purchased from overseas dealers, and total RNA was extracted in the same manner. Regarding the samples for which 125 $\mu$g or more of total RNA was successfully acquired, poly(A)+RNA was subsequently purified therefrom using a MicroPoly(A) purist Kit (Ambion, Austin, TX, USA).

As the human common reference RNA, Human Universal Reference RNA Type I (MicroDiagnostic) or Human Universal Reference RNA Type II (MicroDiagnostic) was used.

(Example 2. Comprehensive gene expression analysis)

A DNA microarray used for gene expression profile acquisition based on poly(A)+RNA (named "System 1") was prepared by forming an array, using a custom arrayer, of 31,797 kinds of synthetic DNA (80 mers) (MicroDiagnostic) corresponding to human-derived transcription products on a slide glass. On the other hand, a DNA microarray for gene expression profile acquisition based on total RNA (named "System 2") was prepared by forming an array, using a custom arrayer, of 14,400 kinds of synthetic DNA (80 mers)

(MicroDiagnostic) corresponding to human-derived transcription products on a slide glass.

A specimen-derived RNA was prepared by synthesizing labeled cDNA from 2 $\mu$g of poly(A)+RNA for System 1 and from 5 $\mu$g of total RNA for System 2 using SuperScript II (Invitrogen Life Technologies, Carlsbad, CA, USA) and Cyanine 5-dUTP (Perkin-Elmer Inc.). Similarly, human common reference RNA was prepared by synthesizing labeled cDNA from 2 $\mu$g of poly(A)+RNA or 5 $\mu$g of total RNA using SupreScript II and Cyanine 3-dUTP (Perkin-Elmer Inc.).

Hybridization with a DNA microarray was performed using a Labeling and Hybridization kit (MicroDiagonostic).

The fluorescence intensity after hybridization with the DNA microarray was measured using a GenePix 4000B Scanner (Axon Instruments, Inc., Union City, CA, USA). Further, the expression ratio (fluorescence intensity of Cyanine-5-labeled cDNA derived from the specimen / fluorescence intensity of Cyanine-3-labeled cDNA derived from the human common reference RNA) was calculated by dividing the fluorescence intensity of the Cyanine-5-labeled cDNA derived from the specimen by the fluorescence intensity of the Cyanine-3-labeled cDNA derived from the human common reference RNA. Furthermore, using GenePix Pro 3.0 software (Axon Instruments, Inc.), the calculated expression ratio was multiplied by a normalization factor for normalization. Next, the expression ratio was converted to Log2, and the converted value was named the Log2 ratio. It should be noted that the expression ratio was converted using Excel software (Microsoft, Bellevue, WA, USA) and a MDI gene expression analysis software package (MicroDiagnostic).

(Example 3. Effective markers for subtype differentiation of breast cancer)

The present invention provides a set of 199 gene markers (207 when including the eight control genes used as controls) having expression patterns correlated with subtypes of breast cancer by cluster analysis.

The gene expression profiles of 14,400 genes were acquired from each specimen of 470 cases including breast cancer tissue (453 cases) and normal mammary gland tissue (17 cases). Eight genes were selected as controls from among genes for which a signal could be detected in three or less specimens, the absolute value of the expression ratio was less than 0.45, the standard deviation was less than 0.35, the maximum - minimum value was less than 2.2, and the average value of "sum of medians" exceeded 400.

Next, the 453 cases of breast cancer tissue excluding normal tissue were classified into a group having ESR1 and ERBB2 expression level ratios of 2.0 or greater, a group having an ESR1 expression level ratio of 2.0 or greater and an ERBB2 expression level ratio less than 2.0, a group having an ERBB2 expression level ratio of 2.0 or greater and an ESR1 expression level ratio less than 2.0, and a group having ESR1 and ERBB2 expression level ratios less than 2.0. A four-group comparison was conducted on the basis of these four groups, and 374 kinds of genes having a p-value less than 0.01 and an absolute value of the difference in the averages of the expression ratios of 1.0 or more were extracted.

Furthermore, genes characteristic of squamous cell carcinoma, phyllodes tumor, and normal tissue were selected by a two-group comparison by a t-test. These genes were classified into a gene group showing the expression pattern characteristic of squamous cell carcinoma (hereinafter referred to as "group a"), a gene group showing an expression pattern characteristic of a phyllodes tumor (hereinafter referred to as group "b"), a gene group showing an expression pattern characteristic of cancer (hereinafter referred to as "group c"), a gene group showing an expression pattern characteristic of normal tissue (hereinafter referred to as "group d"), a gene group showing an expression pattern characteristic of normal-like (hereinafter referred to as "group e"), a gene (hereinafter referred to as "TNBC1") group showing an expression pattern characteristic of the triple negative group and showing an expression pattern characteristic of normal tissue or normal-like (hereinafter referred to as "group f"), a gene (hereinafter referred to as "TNBC2") group showing an expression pattern characteristic of the triple negative (hereinafter referred to as "group g"), a gene (hereinafter referred to as "TNBC3") group showing an expression pattern characteristic of the triple negative and similar to the expression pattern of genes defined as undeterminable (hereinafter referred to as "group h"), a gene group showing an expression pattern characteristic of HER2+-like (hereinafter referred to as "group i"), a gene (hereinafter referred to as "HER2 amplification-1") group related to HER2 amplification and positioned close to the HER2 gene on the chromosome (hereinafter referred to as "group j"), a gene (hereinafter referred to as "HER2 amplification-2") group related to HER2 amplification other than group j (hereinafter referred to as "group k"), a hormone sensitivity-related gene group (hereinafter referred to as "group 1"), ESR1 (hereinafter referred to as "group m"), differentiation-related gene group (hereinafter referred to as "group n"), and a cell cycle-related gene group (hereinafter referred to as "group o"). Considering the overall balance, each group was adjusted to be between one and 37 genes. For groups with an insufficient number of genes, genes showing behavior similar to the genes in the group were added using a correlation coefficient. From among the genes extracted in this way, 199 genes that could clearly classify clusters (eight genes in group a, eight genes in group b, four genes in group c, ten genes in group d, nine genes in group e, six genes in group f, 35 genes in group g, seven genes in group h, 37 genes in group i, five genes in group j, six genes in group k, 29 genes in group 1, one gene in group m, 11 genes in group n, and 23 genes in group o) were selected. The above-described eight genes selected as controls were combined with these to form a marker gene group of 207 genes for breast cancer subtype differentiation. It should be noted that the selected 199 genes described above are the gene group shown in Tables 2A and 2B, and the 199 genes are classified into the groups a to o as shown in Tables 2A and 2B. Further, the sequence information of the probes for the 207 genes used in this example is shown in the tables below.

# EP 4 001 431 A1

[Table 6A]

| Symbol | Sequence ID | Sequence of probes | Sequence number |
|---|---|---|---|
| ABCF3 | NM_018358 | TGACTATGCCCTGCCCCAACTTCTACATTCTGGATGAACCCACAAACCACCTGGACATGGAGACCATTGAGGCTCTGGGC | Sequence number 208 |
| FBXW5 | NM_018998 | ACCACTGGCTGCCTCACCTACTCCCCACACCAGATCGGCATCAAGCAGATCCTGCCACACCAGATGACCACGGCAGGGCC | Sequence number 209 |
| MLLT1 | NM_005934 | ATCTGATCGAGGGAGACTGGCCACTTCAATGTCACCAACACCACCTTCGACTTCGACCTCTTCTCCCTGGACGAGACCACC | Sequence number 210 |
| FAM234A | NM_032089 | TGGCACCGACAGACAGATCCTGTTTCTGGACCTTGGCACTGGAGCCGTCCTGTGTAGCCTAGCCCTCCCGAGCCTCCCTG | Sequence number 211 |
| PITPNM1 | NM_004910 | CACTCCAGCCTCTTTCTGGAGGAGCTGGAGATGCTGGTGCCCTCAACACCCACCTCTACTAGCGGTGCCTTCTGGAAGGG | Sequence number 212 |
| WDR1 | NM_017491 | AGCCTGGCCTGGCTGGACGGAGCACACGCTGGTCACGACCTCCCATGATGCCTCTGTCAAGGAGTGGACAATCACCTACTG | Sequence number 213 |
| NDUFS7 | NM_024407 | ACTATTCCTACTCGGTGGTGAGGGGCTGCGACCGCATCGTGCCCGTGGACATCTACATCCCAGGCTGCCCACCTACGGCC | Sequence number 214 |
| AP2A1 | NM_130787 | AGCATTCCAACGCCAAGAACGCCATCCTCTTCGAGACCATCAGCCTCATCATCCACTATGACAGTGAGCCCAACCTCCTG | Sequence number 215 |
| KRTDAP | NM_207392 | CTTTGAGTCTATCAAAAGGAAACTTCCTTTCCTCAACTGGGATGCCTTTCCTAAGCTGAAAGGACTGAGGGAGCGCAACTC | Sequence number 216 |
| SERPINB3 | NM_006919 | TGGAAGAGAGCTATGACCTCAAGGACACGTTGAGAACCATGGGAATGGTGGATATCTTCAATGGGGATGCAGACCTCTCA | Sequence number 217 |
| SPRR2A | NM_005988 | CCTCAGCAGTGCCAGCAGAAATATCCTCCTGTGCACCCTTCCCCACCCTGCCAGTCAAAGTATCCACCCAAGAGCAAGTA | Sequence number 218 |
| SPRR1B | NM_003125 | GTTTTCAGCTGCTCAGAATTCATCTGAAGAGAGACTTAAGATGAAAGCAAATGATTCAGCTCCCTTATACCCCCATTAAA | Sequence number 219 |
| KLK13 | NM_015596 | CCGTGTCTCAAGATACGTCCTGTGGATCCGTGAAACAATCCGAAAATATGAAAACCCAGCAGCAAAAATGGTTGAAGGGCC | Sequence number 220 |
| KRT1 | NM_006121 | TCCGAAGAAGAGTGGACCAACTGAAGAGTGATCAATCTCGGTTGGATTCGGAACTGAAGAACATGCAGGACATGGTGGAG | Sequence number 221 |
| LGALS7 | NM_002307 | GCAGCCCTTCGAGGTGCTCATCATCGCGTCAGACGACGGCTTCAAGGCCGTGGTTGGGGACGCCCAGTACCACCACTTCC | Sequence number 222 |
| PI3 | NM_002638 | GTTCCTGTTAAAGGTCAAGACACTGTCAAAGGCCGTGTTCCATTCAATGGACAAGATCCCGTTAAAGGACAAGTTTCAGT | Sequence number 223 |
| SERPINH1 | NM_001235 | CCCAAGCTGTTCTACGCCGACCACCCCTTCATCTTCCTAGTGCGGGACACCCAAAGCGGCTCCCTGCTATTCATTGGGCG | Sequence number 224 |
| SNAI2 | NM_003068 | GCTCCTTCCTGGTCAAGAAGCATTTCAACGCCTCCAAAAAGCCAAACTACAGCGAACTGGACACACATACAGTGATTATT | Sequence number 225 |
| GPR173 | NM_018969 | CACGGCTCTTCATGGACCTTCAGTGCACTCAGCTGCAAGATTGTGGCCTTTATGGCCGTGCTCTTTTGCTTCCATGCGGC | Sequence number 226 |
| HAS2 | NM_005328 | CAGACAGTTCTAATTGTTGGAACGTTGCTCTATGCATGCTATTGGGTCATGCTTTTGACGCTGTATGTAGTTCTCATCAA | Sequence number 227 |
| PTH1R | NM_000316 | TTTTGTCGCAATCATATACTGTTTCTGCAATGGCGAGGTACAAGCTGAGATCAAGAAATCTTGGAGCCGCTGGACACTGG | Sequence number 228 |
| PAGE5 | NM_130467 | TGATGTGGAAGCTTTTCAACAGGAACTGGCTCTGCTTAAGATAGAGGATGCACCTGGAGATGGTCCTGATGTCAGGGAGG | Sequence number 229 |
| ITLN1 | NM_017625 | TGCATTTGATGGCCTGTATTTTCTCCCGCACTGAGAATGGTGTTATCTACCAGACCTTCTGTGACATGACCTCTGGGGGTG | Sequence number 230 |
| SH3PXD2B | NM_001017995 | AGATGCCACTCCCCAGAATCCCTTCTTGAAGTCCAGACCTCAGGTTAGGCCAAAACCAGCTCCTTCCCCCAAAACGGAGC | Sequence number 231 |
| TAP1 | NM_000593 | ACCCAGTGGTCTGTTGACTCCCTTACACTTGGAGGGCCTTGTCCAGTTCCAAGATGTCTCCTTTGCCTACCCAAACCGCC | Sequence number 232 |
| FN1 | NM_002026 | AAGACATACCCACGTAGGAGAACAGTGGCAGAAGGAATATCTCGGTGCCATTTGCTCCTGCACATGCTTTGGAGGCCAGCG | Sequence number 233 |
| CTHRC1 | NM_138455 | GAAAGCTTTGAGGGAGTCCTGGACACCCAACTACAAGCAGTGTTCATGGGAGTTCATTGAATTATGGCATAGATCTTGGGAA | Sequence number 234 |
| MMP9 | NM_004994 | GTGAGTTCCCGGAGTGAGTTGAACCAGGTGGACCAAGTGGGCTACGTGACCTATGACATCCTGCAGTGCCCTGAGGACTA | Sequence number 235 |
| ADIPOQ | NM_004797 | CTATATGAAGGATGTGAAGGTCAGCCTCTTCAAGAAGGACAAGGCTATGCTCTTCACCTATGATCAGTACCAGGGAAAATA | Sequence number 236 |
| CD36 | NM_000072 | GCTGTCATTGGTGCTGTCCTGGCTGTGTGTTTGGAGGTATTCTAATGCCAGTTGGAGACCTGCTTATCCAGAAGACAATTAA | Sequence number 237 |
| G0S2 | NM_015714 | GGAAACGGTCCAGGAGCTGATCCCCCTGGCCAAGGAGATGATGGCCCAGAAGCGCAAGGGGAAGATGGTGAAGCTGTACG | Sequence number 238 |
| GPD1 | NM_005276 | GAAGACATTGGAGGCAAAAAGCTGACTGAGATCATCAACACGCAGCATGAGAATGTCAAATACCTGCCAGGGCACAAGTT | Sequence number 239 |
| LEP | NM_000230 | ACACTGGCAGTCTACCAACAGATCCTCACCAGTATGCCTTCCAGAAACGTGATCCAAATATCCAACGACCTGGAGAACCT | Sequence number 240 |
| LIPE | NM_005357 | CCACTGGGCACGGATTCCCTCAAGAACCTGACCCTGAGGGGACTTGAGCCTGAGGGGAAACTCCGAGACGTCGTCGGACAC | Sequence number 241 |
| PLIN1 | NM_002666 | CTGGGTGGTGTGGCACATACCCTGCAGAAGCACCTCCAGACCACCATCTCGGCTGTGACATGGGCACCTGCAGCTGTGCT | Sequence number 242 |
| CAVIN2 | NM_004657 | CCTGCCTAGCAGTGAGCAGATGCCAAATGACCAGGAAGAGGGAGTCCTTTGCAGAGGGTCATTCCGAAGCGTCCCTCGCCA | Sequence number 243 |
| LIFR | NM_002310 | ATTTAGTGTCTCCAGACTCTCCTAGATCCATAGACAGCAACAGTGAGATTGTCTCATTTGGAAGTCCATGCTCCATTAAT | Sequence number 244 |
| TGFBR3 | NM_003243 | GGATAAGAAGCCGATTCAGCTTTGTCTTCAAGCCTGTCTTCAACACCTCACTGCTTTTCTACAGTGTAGCTGAGCTGACGCTGT | Sequence number 245 |
| CAPN6 | NM_014289 | AAGGGTGGTCCAACTGCCAAAGTCAAGCAAGGCCACATCAGCTTCAAGGTTATTTCCAGCGATGATCTCACTGAGCTCTA | Sequence number 246 |
| PIGR | NM_002644 | CAAGAAGGCAAAAAGGTCATCCAAGGAGGAAGCCGAGATGGCCTACAAAGACTTCCTGCTCCAGTCCAGCACCGTGGCCG | Sequence number 247 |
| KRT15 | NM_002275 | GGTAGCAGCAGCAATTTCCACATCAATGTAGAAGAGTCAGTGGATGGACAGGTGGTTTCTTCCCACAAGAGAGAAATCTA | Sequence number 248 |
| KRT5 | NM_000424 | TGCAGACTCAGTGGAGAAGGAGTTGGACCAGTCAACATCTCTGTTGTCACAAGCAGTGTTTCCTCTGGATATGGCAGTGG | Sequence number 249 |
| KRT14 | NM_000526 | GAGATCAAAGACTACAGCTCCTACATCAAGACCATTGAGGACCTGAGGAACAAGATTCTCACAGCCACAGTGGACAATGC | Sequence number 250 |
| DST | NM_001723 | CAAAGCCTTGGTCAAAAAGTATCAGGAAGGCCTCATCACACTTACAGAACTTGCTGATTCGTTTGCTGAGCCGGTTAGTCC | Sequence number 251 |
| WIF1 | NM_007191 | CCCACCTGGATTCTATGGAGTGAACTGTGACAAAGCAAACTGCTCAACCACCTGCTTTAATGGAGGGACCTGTTTCTACC | Sequence number 252 |
| SYNM | NM_145728 | AAGGAGCAGGCCATGTTTGATAAGAAGGTGCAGCTCCAGAGAATGGTAGACCAAAGGTCGGTGATTTCAGATGAAAAGAA | Sequence number 253 |
| KIT | NM_000222 | TAGATTCTAGTGCATTCAAGCACAATGGCACGGTTGAATGCAAAGCTTACAACGATGTGGGCAAGACTTCTGCCTATTTT | Sequence number 254 |
| GABRP | NM_014211 | CTACAGTGACTTGACAATGAAAACCAGCGACAAGTTCAAGTTTGTCTTCCCAGAAAAGATGGGCAGGATTGTTGATTATT | Sequence number 255 |
| SFRP1 | NM_003012 | ATGGCGACAAGAAGATTGTCCCCAAGAAGAAGAAGCCCCTGAAGTTGGGGCCCATCAAGAAGAAGGACCTGAAGAAGCTT | Sequence number 256 |
| ELF5 | NM_001422 | GAATTTGTACGAGACCTGCTTCTATCTCCTGAAGAAAACTGTGGCATTCTGGAATGGGAAGATAGGGAACAAGGAATTTT | Sequence number 257 |
| MIA | NM_006533 | AGCATTGTCCGAGAGGACCAGACCCTGAAACCTGGCAAAGTCGATGTGAAGACAGACAAATGGGATTTCTACTGCCAGTG | Sequence number 258 |
| MMP7 | NM_002423 | CCATTCTTTGGGTATGGGACATTCCTCTGATCCTAATGCAGTGATGTATCCAACCTATGGAAATGGAGATCCCCAAAATT | Sequence number 259 |
| FDCSP | NM_152997 | TTTGTGTTCCCTTACCCATATCCATTTCGCCCACTTCCACCAATTCCATTTCCAAGATTTCCATGGTTTAGACGTAATTT | Sequence number 260 |

[Table 6B]

| Symbol | Sequence ID | Sequence of probes | Sequence number |
|---|---|---|---|
| CRABP1 | NM_004578 | GAGGAGGAGACCGTGGACGGACGCAAGTGCAGGAGTTTAGCCACTTGGGAGAATGAGAACAAGATCCACTGCACCCAAAC | Sequence number 261 |
| PROM1 | NM_006017 | CGCACAGGGAATGGGATTGTTGGAGAGAGTAACTAGGGATTCTTAGCTTCTCTGGATTTTGCTCAGAACTTCATCACAAACAA | Sequence number 262 |
| KRT25 | NM_015515 | AAGATCAAGGCCATAACCCAGGAGACCATCAACGCGGAAGATTAGTTCTTTGTCAAGTGAATGAAATCCAAAAGCACGCATG | Sequence number 263 |
| S100A1 | NM_006271 | ATGCCCAGAAGGATGTGGATGCTGTGGACAAGGTGATGAAGGAGCTAGACGAGAATGGAGACGGGGAGGTGGACTTCCAG | Sequence number 264 |
| WIPF3 | NM_001080529 | TGCGAAATGGAAGCCTGCACATCATTGATGACTTCGAGTCTAAATTCACGTTCCATTCTGTGGAAGACTTTCCCCCTCCG | Sequence number 265 |
| CYYR1 | NM_052954 | CTGCTCTTTGTCTACGCAGATGATTGCCTTGCTCAGTGTGGCAAAGATTGCAAATCTTACTGCTGTGATGGAACCACGCC | Sequence number 266 |
| TFCP2L1 | NM_014553 | TGTACCACGCCATCTTCCTGGAAGAGCTGACCACCTTGGAGCTGATTGAGAAGATTGCCAACCTGTACAGCATCTCCCCC | Sequence number 267 |
| DSC2 | NM_004949 | GTTGGGGCATAGCATTGCTCTTTTGCATCCTGTTTACGCTGGTCTGTGGGGCTTCTGGGACGTCTAAACAACCAAAAGTAA | Sequence number 268 |
| MFGE8 | NM_005928 | TGGCAGCAGTAAGATCTTCCCTGGCAACTGGGACAACCACTCCCACAAGAAGAACTTGTTTGAGACGCCCATCCTGGCTC | Sequence number 269 |
| KLK7 | NM_005046 | CAACCCAATGACCCAGGAGTCTACACTCAAGTGTGCAAGTTCACCAAGTGGATAAATGACACCATGAAAAAGCATCGCTA | Sequence number 270 |
| KLK5 | NM_012427 | CTTCTGGGGGTCACAGAGCATGTTCTCGCCAACAATGATGTTTCCTGTGACCACCCCTCTAACACCGTGCCCTCTGGGAG | Sequence number 271 |
| DSG3 | NM_001944 | CCATCCCATAGAAGTCCAGCAGACAGGATTTGTTAAGTGCCAGACTTTGTCAGGAAGTCAAGGAGCTTCTGCTTTGTCCG | Sequence number 272 |
| TTYH1 | NM_020659 | GACTACGATGACACAGACGATGACGACCCTTTCAACCCTCAGGAATCCAAGCGCTTTGTGCAGTGGCAGTCGTCTATCTG | Sequence number 273 |
| SCRG1 | NM_007281 | TTCAGCGAATTGCTCTGCTGCCCAAAAGACGTTTTCTTTGGACCAAAGATCTCTTTCGTGATTCCTTGCAACAATCAATG | Sequence number 274 |
| S100B | NM_006272 | GGGGAGACAAGCACAAGCTGAAGAAATCCGAACTCAAGGAGCTCATCAACAATGAGCTTTCCCATTTCTTAGAGGAAATCA | Sequence number 275 |
| ETV6 | NM_001987 | CTCATTCAGGTGATGTGCTCTATGAACTCCTTCAGCATATTCTGAAGCAGAGGAAACCTCGGATTCTTTTTTTCACCATTC | Sequence number 276 |
| OGFRL1 | NM_024576 | AAGAAATACAGAGAAGGACAGTAATGCTGAGAACATGAATTCTCAACCTGAGAAAACAGTTACTACTCCCACAGAAAAAA | Sequence number 277 |
| MELTF | NM_005929 | ACAATAAGAACGGGTTCAAAATGTTCGACTCCTCCAACTATCATGGCCAAGACCTGCTTTTCAAGGATGCCACCGTCCGG | Sequence number 278 |
| HORMAD1 | NM_032132 | AGTCCTCCATCACTTTGATTCTTCTAGTCAAGAGTCAGTGCCAAAAAGGAGAAAGTTTAGTGAACCAAAGGAACATATAT | Sequence number 279 |
| PKP1 | NM_000299 | ATGTGGTCCAGCAAGGAACTGCAGGGGTGTCCTCAGACAGCAAGGTTTCGATAGGAACATGCTGGGAACCTTAGCTGGGGC | Sequence number 280 |
| FOXC1 | NM_001453 | GAACAACTCTCCAGTGAACGGGAATAGTAGCTGTCAAATGGCCTTCCCTTCCAGCCAGTCTCTGTACCGCACGTCCGGAG | Sequence number 281 |
| ITGB8 | NM_002214 | TCATGTGCTCTCATGGAACAACAGCATTATGTCGACCAAACTTCAGAATGTTTCTCCAGCCCAAGCTACTTGAGAATATT | Sequence number 282 |
| VGLL1 | NM_016267 | AGTACCAGCCTTCCAAATGAAACTCTTTCAGAGTTAGAGACACCTGGGAAATACTCACTTACACCACCAAACCACTGGGG | Sequence number 283 |
| ART3 | NM_001179 | GCTTGAAGACCATGGTGAGAAAAACCAGAAGCTTGAAGACCATGGTGTGAAAATCCTTGAACCCACCCAAATACCTGCTC | Sequence number 284 |
| EN1 | NM_001426 | TCTCTATTCCCAGTATAAGGGACGAAACTGCGAACTCCTTAAAGCTCTATCTAGCCAAACCGCTTACGACCTTGTATATA | Sequence number 285 |
| SPHK1 | NM_021972 | TTCCGCTTGGAGACCCAAGGATGGGAAAGGTGTGTTTGCAGTGGATGGGGAATTGATGGTTAGCGAGGCCGTGCAGGGCCA | Sequence number 286 |
| TRIM47 | NM_033452 | GCAGACAAGTTCCTGCAGCTGTTTGGAACCAAAGGTGTCAAGAGGGTGCTGTGTCCTATCAACTACCCCTTGTCGCCCAC | Sequence number 287 |
| COL27A1 | NM_032888 | CTTGGCTGCTCCTCTGACACCATCGAGGTCTCCTGCAACTTCACTCATGGTGGACAGACTGTGTCTCAAGCCCATCACGGC | Sequence number 288 |
| RFLNA | NM_181709 | CCCACGCATGAGATCCGCTGCAACTCTGAGGTCAAGTACGCCTCGGAGAAGCATTTCCAGGACAAGGTCTTCTATGCGCC | Sequence number 289 |
| RASD2 | NM_014310 | GTCCTTCGATGAGGTCAAGCGCCTTCAGAAGCAGATCCTGGAGGTCAAGTCCTGCCTGAAGAACAAGACCAAGGAGGCGG | Sequence number 290 |
| A2ML1 | NM_144670 | AAACCAGCAACCATCAAGGTCTATGACTACTACCTACCAGATGAACAGGCAACAATTCAGTATTCTGATCCCTGTGAATG | Sequence number 291 |
| MARCO | NM_006770 | GGACAATTTGCGATGACGAGTGGCAAAATTCTGATGCCATTGTCTTCTGCCGCATGCTGGGTTACTCCAAAGGAAGGGCC | Sequence number 292 |
| TSPYL5 | NM_033512 | TCAACGAAGAATTGTGGCCCAATCCCTTGCAGTTCTACCTTTTGAGTGAAGGGGCTCGTGTAGAGAAAGGAAAGGAAAAA | Sequence number 293 |
| TM4SF1 | NM_014220 | GATGCTTTCTTCTGTATTGGCTGCTCTCATTGGAATTGCAGGATCTGGCTACTGTGTCATTGTGGCAGCCCTTGGCTTAG | Sequence number 294 |
| FABP5 | NM_001444 | ACAATAACAAGAAAAATTGAAAGATGGGAAATTAGTGGTGGAGTGTGTCATGAACAATGTCACCTGTACTCGGATCTATGA | Sequence number 295 |
| SPIB | NM_003121 | CTTCAGCTGTCTGTACCCAGATGGCGTCTTCTATGACCTGGACAGCTGCAAGCATTCCAGCTACCCTGATTCAGAGGGGG | Sequence number 296 |
| BCL2A1 | NM_004049 | TGCCAGAACACTATTCAACCAAGTGATGGAAAAGGAGTTTGAAGACGGCATCATTAACTGGGGAAGAATTGTAACCATAT | Sequence number 297 |
| MZB1 | NM_016459 | CTCCCGGAACTGGCAGGACTACGGAGTTCGAGAAGTGGACCAAGTGAAACGTCTCACAGGCCCAGGACTTAGCGAGGGGC | Sequence number 298 |
| KCNK5 | NM_003740 | AAGGACGTCAACATCTTCAGCTTTCTTTCCAAGAAGGGAAGAGACCTACAACGACCTCATCAAGCAGATCGGGAAGAAGGC | Sequence number 299 |
| LMO4 | NM_006769 | ACATGATAGACCTACAGCTCTCATCAATGGCCATTTGAATTCACTTCAGAGCAATCCACTACTGCCAGACCAGAAGGTCT | Sequence number 300 |
| RNF150 | XM_005263150 | TCTAAGTTTCTTTTCCTTTTCTGTCTGTATCTGTTTTTCTCTGACTGCCTATATCTTACTTTGTATACCCATACATAAAT | Sequence number 301 |
| LYZ | NM_000239 | GTCATTTATCCTGCAGTGCTTTGCTGCAAGATAACATCGCTGATGCTGTAGCCTTGTGCAAAGAGGGTTGTCCGTGATCCA | Sequence number 302 |

## [Table 6C]

| Symbol | Sequence ID | Sequence of probes | Sequence number |
|---|---|---|---|
| C21orf58 | NM_058180 | CATGGTGGAGCTGCTGCTGCTGCAGAACGCACAGGTGCACCAGTTGGTCCTGCAGAACTGGATGCTCAAGGCCCTGCCCC | Sequence number 303 |
| ATP13A5 | NM_198505 | CTTCTCTATGTGAAGCAGCAGCCTTGGTATTGTGAGGTCTACCAATACAGTGAGTGTTTTCTGGCCAACCAAAGCCCATA | Sequence number 304 |
| NUDT8 | NM_181843 | ACCGTGGTGCCAGTGCTTGCTGGTGTAGGCCCACTGGATCCCCAGAGCCTCAGGCCCAACTCGGAGGAGGTGAGCTGGGG | Sequence number 305 |
| HSD17B2 | NM_002153 | GACTGACTACAAACAATGCATGGCCGTGAACTTCTTTGGAACTGTGGAGGTCACAAAGACGTTTTTGCCTCTTCTTAGAA | Sequence number 306 |
| ABCA12 | NM_015657 | AAGTCCTATGAAACTGCTGATACCAGCAGCCAAGGTTCCACTATAAGTGTTGACTCACAAGATGACCAGATGGAGTCTTA | Sequence number 307 |
| ENPP3 | NM_005021 | AACACTGATGTTCCCATCCCAACACACTACTTTGTGGTGCTGACCAGTTGTAAAAACAAGAGCCACACACCGGAAAACTG | Sequence number 308 |
| WNT5A | NM_003392 | GCCACTGCAAGTTCCACTGGTGCTGCTACGTCAAGTGCAAGAAGTGCACGGAGATCGTGGACCAGTTTGTGTGCAAGTAG | Sequence number 309 |
| MPP3 | NM_001932 | GGTGCCTACAGCCAGCTCAAAGTGGTCTTAGAGAAGCTGAGCAAGGACACTCACTGGGTACCTGTTAGTTGGGTCAGGTA | Sequence number 310 |
| VPS13D | NM_015378 | AATCGTGGATGGCAGATTACTGTAAAGATGACAAGGACATAGAGTCAGCTAAATCAGAAGACTGGATGGGCTCTTCGGTG | Sequence number 311 |
| PXMP4 | NM_007238 | ACCTACCTCTATGAGGACAGCAATGTATGGCACGACATCTCAGACTTCCTCGTCTATAACAAGAGCCGTCCCTCCAATTA | Sequence number 312 |
| GGT1 | NM_013421 | CCCGGTCAGCGGGATCCTGTTCAATAATGAAATGGACGACTTCAGCTCTCCCAGCATCACCAACGAGTTTGGGGTACCCC | Sequence number 313 |
| TRPV6 | NM_018646 | AGCATGAACGCCAAGGAATGTACGTTGAGAATCACTGCTCCAGGCCTGCATTACTCCTTCAGCTCTGGGGCAGAGGAAGC | Sequence number 314 |
| MAB21L4 | NM_024861 | AACATCCAGGATAAGGACCGGATCTCTGCCATGCAGAGCATCTTCCAGAAGACCAGGACTCTGGGAGGCGAGGAGAGCTG | Sequence number 315 |
| CLDN8 | NM_199328 | CCTTCCCATCGCACAACCCAAAAAAGTTATCACACACGGAAAGAAGTCACCGAGCGTCTACTCCAGAAGTCAGTATGTGTA | Sequence number 316 |
| LBP | NM_004139 | AACTATTACATCCTTAACACCCTCTACCCCAAGTTCAATGATAAGTTGGCCGAAGGCTTCCCCCCTTCCTCTGCTGAAGCG | Sequence number 317 |
| SRD5A3 | NM_024592 | GGAGACTGGTTTGAATATGTTTCTTCCCCTAACTACTTAGCAGAGCTGATGATCTACGTTTCCATGGCCGTCACCCTTTGG | Sequence number 318 |
| PAPSS2 | NM_004670 | ATTCCGAGTGGCTGCCTACAACAAAGCCAAAAAAGCCATGGACTTCTATGATCCAGCAAGGCACAATGAGTTTGACTTCA | Sequence number 319 |
| TMEM45B | NM_138788 | ACTATTCTCTTGTTTACTGCCTTTTGACTCGGATGAAGAGACACGGAAGGGGAGAAATCATTGGAATTCAGAAGCTGAAT | Sequence number 320 |
| CLCA2 | NM_006536 | AAAGCTTATGGCTCTGTGATGATATTAGTGACCAGCGGAGATGATAAGCTTCTTGGCAATTGCTTACCCACTGTGCTCAG | Sequence number 321 |
| FASN | NM_004104 | TGGTCTTGAGAGATGGCTTGCTGGAGAACCAGACCCCAGAGTTCTTCCAGGACGTCTGCAAGCCCAAGTACAGCGGCACC | Sequence number 322 |
| MPHOSPH6 | NM_005792 | GTTGAAGATGAAACAGTAGAGCTTGATGTGTCAGATGAAGAGATGGCTAGAAGATATGAGACCTTGGTGGGGACAATTGG | Sequence number 323 |
| NXPH4 | NM_007224 | CTGTGCCAAGCCCTTCAAAGTCATCTGTATCTTCGTCTCTTTCCTCAGCTTTGACTACAAACTGGTGCAGAAGGTGTGCC | Sequence number 324 |
| HPGD | NM_000860 | CTAATCTTATGAACAGTGGTGTGAGACTGAATGCCATTTGTCCAGGCTTTGTTAACACAGCCATCCTTGAATCAATTGAA | Sequence number 325 |
| KYNU | NM_003937 | TTGGATTACAGGAGATGAGAGTATTGTAGGCCTTATGAAGGACATTGTAGGAGCCAATGAGAAAGAAATAGCCCTAATGA | Sequence number 326 |
| GLYATL2 | NM_145016 | CAGGCACTGAACAATTTGGGGGTTTAAGATTTGTCCCTGTGGCTGGCATCAGTGGAAATGCACCCCCAAGAAATGTTGTTG | Sequence number 327 |
| KMO | NM_003679 | CATGTCACCACGATCGTTTCCTCTGCTTGAGAAGACCATGGAACTGGATAGCTCACTTCCGGAATACAACATGTTTCCCCG | Sequence number 328 |
| SRPK3 | NM_014370 | TGTTCGAGCCGCATTCTGGAGAAGAGACTACAGTCGTGATGAGGACCACACATCGCTCACATAGTGGAGCTTCTGGGGGACATC | Sequence number 329 |
| THRSP | NM_003251 | CATCACATCCTCATGCACCTCACCGAGAAAGCCCAGGAGGTGACAAGGAAATACCAGGAAATGACGGGACAAGTTTGGTA | Sequence number 330 |
| PLA2G2A | NM_000300 | TCGCTGCTGTGTCACTCATGACTGTTGCTACAAACGTCTGGAGAAACGTGGATGTGGCACCAAATTTCTGAGCTACAAGT | Sequence number 331 |
| TFAP2B | NM_003221 | CCTGCACTCCCGAAAGAATATGCTGTTGGCCACCAAGCAACTTTGTAAAGAATTTACGGATCTACTGGCGCAGGACCGGA | Sequence number 332 |
| FABP7 | NM_001446 | GCACATTCAAGAACACGGAGATTAGTTTCCAGCTGGGAGAAGAGTTTGATGAAACCACTGCAGATGATAGAAACTGTAAG | Sequence number 333 |
| SLPI | NM_003064 | GTCCTTCAAAGCTGGAGTCTGTCCTCCTAAGAAATCTGCCCAGTGCCTTAGATACAAGAAACCTGAGTGCCAGAGTGACT | Sequence number 334 |
| SERHL2 | NM_014509 | ATGAAATGGAGAACTTGCTGACCTACAAGCGGGAGAGCCATAGAGCACGTGCTGCAGGTAGAGGCCTCCCAGGAGCCCTCG | Sequence number 335 |
| S100A9 | NM_002965 | ATGGAGGACCTGGACACAAATGCAGACAAGCAGCTGAGCTTCGAGGAGTTCATCATGCTGATGGCGAGGCTAACCTGGGC | Sequence number 336 |
| KRT7 | NM_005556 | AGCTTCTCCAGCAGTGCGGGTCCTGGGCTCCTGAAGGCTTATTCCATCCGGACCGCATCCGCCAGTCGCAGGAGTGCCCG | Sequence number 337 |
| TMEM86A | NM_153347 | AGTGGTGCACTCTTCTTTATCATCTCAGACCTGACCATCGCCCTCAACAAATTCTGTTTTCCTGTGCCCTACTCTCGGGC | Sequence number 338 |
| MBOAT1 | NM_001080480 | CTGTCTCTTACACGGTAGCACCGTTTGTGATGTTGGCAGTTGAACCGACCATCAGCTTATACAAGTCCATGTACTTTTAT | Sequence number 339 |
| PGAP3 | NM_033419 | TCCTGTGCTGCTGTCTGGTTGAGAGCCTGCCACCGTGTGTCGGGAGTGTGGGCCAGGCTGAGTGCATAGGTGACAGGGCC | Sequence number 340 |
| STARD3 | NM_006804 | CCTGCTCTGGATCATCGAACTGAATACCAACACAGGCATCCGTAAGAACTTGGAGCAGGAGATCATCCAGTACAACTTTA | Sequence number 341 |
| ERBB2 | NM_004448 | TGATGGGGAGAATGTGAAAATTCCAGTGGCCATCAAAGTGTTGAGGGAAAACACATCCCCCAAAGCCAACAAAGAAATCT | Sequence number 342 |
| MIEN1 | NM_032339 | ATTGAGGCCATCCGAAGAGCCAGTAATGGAGAAACCCTAGAAAAGATCACCAACAGCCGTCCTCCCTGCGTCATCCTGTG | Sequence number 343 |
| GRB7 | NM_005310 | CTCGATGCACACACTGGTATATCCCATGAAGACCTCATCCAGAACTTCCTGAATGCTGGCAGCTTTCCTGAGATCCAGGG | Sequence number 344 |
| GSDMB | NM_018530 | CCTGACATGGACTATGACCCTGAGGCACGAATTCTCTGTGCGCTGTATGTTGTTGTCTCTATCCTGCTGGAGCTGGCTGA | Sequence number 345 |
| ORMDL3 | NM_139280 | TCGTGCGTGTACTTCCTCACCAGCTTCTACAACTAAGTACGACCAGATCCATTTTGTGCTCAACACCGTGTCCCTGATGAGC | Sequence number 346 |
| MED24 | NM_014815 | ACGATGTGCAGCCTTCGAAGTTGATGCGACTGCTGAGCGTCTAATGAGGACGGATGCCAACATCCTTTCGAGCCCCACAGAC | Sequence number 347 |
| MSL1 | NM_001012241 | TGTATCACATCCACTTCTCAAGTATTCCTTCATTGGGGCTTCATCCTTTTAGCAGAACTCTTGGTGGTGGGGATAGAGACTTA | Sequence number 348 |
| CASC3 | NM_007359 | ATGAAGATCGGAAGAATCCAGCCATACACATACCTCGGAAAGGGGCTCTTCTTTGAGCATGATCTTCGAGGGCAAACTCAGGAG | Sequence number 349 |
| WIPF2 | NM_133264 | TGTCCGGTCTTTCTTGGATGATTTTGAGTCAAAGTATTCCTTCCATCCAGTAGAAGACTTTCCTGCTCCAGAAGAATATA | Sequence number 350 |

[Table 6D]

| Symbol | Sequence ID | Sequence of probes | Sequence number |
|---|---|---|---|
| THSD4 | NM_024817 | ACATGACTCTAAGTAACCTCTGTGACCCTCAGTTGAAACCAGAAGAGAGAGAATCTTGTAACCCTCAGGACTGTGTCCCT | Sequence number 351 |
| MAPT | NM_016835 | ACCTTCCTCTCCTAAACACGTCTCTTCTGTCACTTCCCGAACTGGCAGTTCTGGAGCAAAGGAGATGAAACTCAAGGGGG | Sequence number 352 |
| LONRF2 | NM_198461 | TGCTGGCTCGGTTATCAGACTCTCATCCAACACAAGCTCACAGGGAAAGCCGTTCCTTGCTCCTTGTGGAGGGAGCTACC | Sequence number 353 |
| TCEAL5 | NM_052926 | GCATCTGAGCAGTGAGGAGATGATGAGAGAAATGTGGAGATGTGTCAAGGGCTCAAGAGGAGCTAAGGAAAAACAGAAAA | Sequence number 354 |
| DRNDE2 | NM_015473 | AATTCTTCGAGGACAATTTACAGCCAGAGACAGAGTTTGTCTTTCCTCTCGTCCCATCTGCATCTCGAGTCGCAGAGACCC | Sequence number 355 |
| PGD5 | NM_055086 | CCAGGTGCAGGATATCGTCAAGCCAAACACAGCACATACATTCATCATAACAGGAAGAAAAAGGTCCCTCGAGCTGCAGA | Sequence number 356 |
| GFRA1 | NM_005264 | GTCTCGGTGCTTCCAGCCACGATAACCACAAAATCAATGGCTGCTCCTCCAAGCTGTGGTCTGAGCCCACTGCTGGTCCTG | Sequence number 357 |
| PARD6B | NM_032521 | CTCCGGCACTATCGAGGTGAAGAGCAAGTTTGGAGCTGAATTTCGTCCGTTTTCGCTGGAAAGATCAAAACCTGGAAAATT | Sequence number 358 |
| STC2 | NM_003714 | GTGCAGGTTCAGTGTGAGCAGAACTGGGCGAAGCCTGTGCTCCATCTTGAGCTTCTGCACCTCGGCCATCCAGAAGCCTCC | Sequence number 359 |
| SLC39A6 | NM_012319 | CTCGTTCCTCGTCTTCGTCATGAGTTGCCTCATGAATTAGGTGACTTTGCTGTTCTACTAAAGGCTGGCATGACCGTTAAG | Sequence number 360 |
| RNF5 | NM_021572 | CAATATCACCGCCATGCCACACAATGGATCATTCTGGAATGTCCAGGATCTGCTCAATTCAGCAATGCCAAGGGTGGTCC | Sequence number 361 |
| ZNF703 | NM_025069 | TCGCTCGTCCTTGCGGAATCCACACACAGTTTGGGCCTAAGCCGGTACCACCCCTATGGCAAGAGCCCACTTATCCACAGCGGG | Sequence number 362 |
| RVL | NM_016337 | GCAAGTAAAGAAGAGGCAACCACGTTCTCCAATGCAATGCTGTTTGCCCTGAACATCATGAATTCCCAAGAAGGGAGGCCC | Sequence number 363 |
| TBC1D9 | NM_015130 | GGTGCCAGATGATGGCTTTGTTACGGTGAGCCTAAAGCCAGACAAAGGGAAGAGAGCAAATTCCCAAGAAAATCGTAATTA | Sequence number 364 |
| CHAD | NM_001267 | CGCTGAAACACGTCCATTTGGAGAACAACCGCTTGAACCAGCTACCCTCGAACTTCCCCTTCGAGAGCCTCGAGACCCTC | Sequence number 365 |
| GREB1 | NM_014668 | TGGCCTTTCATTGTGATCTCTGATGACTCCTGCGTGATGTCGAACGTGGTGGATGTCAACTCTGCTGGGGAGAGAAGCAG | Sequence number 366 |
| HPN | NM_002151 | ATAATCAGCAATGATGTCTGCAATGGCGCTGACTTCTATGGAAACCAGATCAAGCCCAAGATGTTCTGTGCTGGCTACCC | Sequence number 367 |
| ILST | NM_002184 | CGGTGATGGTATTTTGCCCAGGCAACAGTACTTCAAACAGAACTGCAGTCAGCATGAATCCAGTCCAGATATTTCAACATT | Sequence number 368 |
| GASK1B | NM_016615 | CCAGCTTCTGCAGTTTCTGTTTTGAAGAGCCAGCACTTACGGCAGAAGCACTTCTTCAGTCTCTGTTTCTTGATAAAGTGTA | Sequence number 369 |
| CA12 | NM_206925 | CACAGGATTCCTGTGGGTCCAGCTTTGGAACTGGGCAAAACCTTTCTTCGGATCCGCACTCATTCCACTGATGCCAGCTGCC | Sequence number 370 |
| KCNK4 | NM_080671 | TCTACATTCTGGTTGTCATGTCCTTCTACGGCATTTCTTGATCGGAATCGATGCTGGGCTACATGAAATCCAAGAGGCGG | Sequence number 371 |
| ESR1 | NM_000125 | TGGTGGAGATCTTCGACATGCTGCTGGCTACATCATCTCGGTTCCGCATGATGAATCTGCAGGGAGAGGAGTTTGTGTGC | Sequence number 372 |
| NAT1 | NM_000662 | ATGAAGAATTCTTCATTCTGATCTCCTAGAAGACAGCAAATACCGAAAAATCTACTCCTTTACTCTTAAGCCTGAAAGTGTA | Sequence number 373 |
| CYP2B6(CYP2B7P) | NM_000767 | CTGAGCACTGCTCTCCGTGACCCACACTACTTTGAAAAACCAGACGCCTTCAATCCTGACCACTTTCTGGATGCCAATGG | Sequence number 374 |
| ARMT1 | NM_024575 | AAAATGGGAGTTTTCTGTTCCATTTCATCAGGCTCTGAATGGCTTCCATCGTGCACCACTCTGTACCATAAGAACAATTAA | Sequence number 375 |
| MAGED2 | NM_177433 | TTTGGGCTCTTCGCTGGCCTTCGGTGGAGCTGGTGCCAGCACCAGTGGCAGCTCTGGTGCCTGTGGTTCTCCTACAAGTG | Sequence number 376 |
| CFLAR1 | NM_014345 | ACCAGACGGAAAACCCGTTTCTGTGCACAGTGGTTGCCATCCTCCTCCACATCACATGAGCACCTTTGCCCTGGACC | Sequence number 377 |
| INPP5J | NM_001002837 | TGGAGACTTCATCCTGGGCTACTATAGTCACAACCACAGCATCCTCATCGGCATCACTGAACCCTTCCAGAATCTCGCTGC | Sequence number 378 |
| PADI2 | NM_007365 | TGAGCAGCCAAGCGAATCACCATCAACAAGATTCTGTCCAACGACAGAGCCTTGTGCAGGAGAACCTGTACTTCCAGCGCTGC | Sequence number 379 |
| PPP1R1B | NM_032192 | CCCTCTGGATGAGTCCGGAGAGAGATGGAGGCTCTGAGGACCGAAGTGGAAGAACCCAGCCACTAAGTGAGCCTGGGGAGGAAC | Sequence number 380 |
| MLPH | NM_024101 | AAGGCAATGGCGTGCCCCTATCTTCTGAGAAGAAAGTTCAGTAATTCCCTGAAAAGTCAAGGTAAAGATGAATGATTCTTT | Sequence number 381 |
| FOXA1 | NM_004496 | CTCCGGAGCCAGCACATAACTGGACTTCAAGGCGATACGAACAGGCACTGCAATACTCGCCCTTACGGCTCTACGTTGCCCG | Sequence number 382 |
| XBP1 | NM_005080 | CAGATTCAGAGTCTGATATCCTGTTGGGCATTCTGGACAACTTGGACCCAGTCATGTTCTTCAAATGCCCTTCCCCAGAG | Sequence number 383 |
| GATA3 | NM_002051 | AATGTCTAGCAAATCCAAAAGTGCAAAAAGTGCATGACTGCAGGGACTTCCCCAAGAACAGCTCGGTTTAACCCGG | Sequence number 384 |
| ZG16B | NM_145252 | GGGCATCTATCGCCAGTATCAACTCCTTGGCATCAAGAGCATTGGCTTTGAATGGAATTATCCACTAGAGGAGCCGACCA | Sequence number 385 |
| KIAA0040 | NM_014656 | TGGGCAAGTATATTCTGCTCATCAAATGGTCATTGGCTATGTACTTTGTGCAGGGAAGTACATTATCTACAGTCAGAAAA | Sequence number 386 |
| TMC4 | NM_144668 | CCTTTACTGCCCGTGCTTAACACGGTCAAGTTCCTGCTGCTTTTCTACCTGAAGAAAGCTTACCCTCTTCTCCAACCTGCTC | Sequence number 387 |
| AGR2 | NM_006408 | TATGCTTACGAACCTGCAGATACAGTGGATGACATGAGGAAAATGATGGAGTCTGTGGCTCGTTGCTTGGAAGACTGAATTGTA | Sequence number 388 |
| TFF3 | NM_003226 | CCAGGATCCCTGGAGTGCCTTGGTGTTTCAAGCCCCTGACTAGGAAGACAGAATGCACCTTCTGAGGCACCTCCAGCTGC | Sequence number 389 |
| SCGB2A2 | NM_002411 | TGCCATAGATGAATTGAAGGAATGTTTTCTTAACCAAACGGATGAAACTCTGAGCAATGTTGAGGTGTTTATGCAATTAA | Sequence number 390 |
| MUCL1 | NM_058173 | GCTTCTACCACTGCTCGTAAAGACATTCCAGTTTTACCCAAATGGGTTGGGGATCTCCCGAATGGTAGAGTGTGTCCCTG | Sequence number 391 |
| DDX11 | NM_004399 | ACCTGTTCAAGGTGCAAGTACTGTGAGAAGAGCATGATCAGCAGAAAAGCTCTTTGGATTCCACGGTGACGGCACGGACGA | Sequence number 392 |
| ATAD2 | NM_014109 | CAGCTCATCACTGTTGAAAAGGCTTTGGCAATTCTTTCTCAGCCTACACCCTCACTTGTTGTCGATCATGAGCGATTAAA | Sequence number 393 |
| GGH | NM_003878 | CATCCAGAGAAAGCCACCTTATGAGTGGAAGAATTTCGGATGCGCATTTCCCATGCACCTAATGCTGTGAAAAACCCGCATTTA | Sequence number 394 |
| CDCA5 | NM_051299 | ACCCTGAGACTCCCAGATCTTCAGGTTCTATGCGCAATAGATGGAAACCAAACAGCAGCAAGGTACTAGGGGAGATCCCCC | Sequence number 395 |
| CCNA2 | NM_001237 | AGCACTCTACACAGTCACGGGACAAAAGCTGGCCCTGAATCATTAATACGAAAAGACTGGATATACCCTGGAAAGTCTTAAGC | Sequence number 396 |
| CCNB2 | NM_004701 | GAATAAGTATGCAAGCAGCAAACTCCTGAAAGATCAGCCATGAACCCTCAGCTGAACTCAAAAGCCGTCAAAGACCTTGCCT | Sequence number 397 |
| ANLN | NM_018685 | CTCTCGTGTTACCCAAGAACTGGCTGTCTGCCAGATACTAAAGAAGAGCGGGATCTCTGGATGCAAAAACTCAATCAAGTTCT | Sequence number 398 |
| UBE2C | NM_007019 | GCTGCCGGAGCTCTGGAAAAACCCCACACGTTTTAAGGAACTAGCTGCAAGAAACCTACTCAAAGCAGGTCACCAGCCAGGA | Sequence number 399 |
| CKS2 | NM_001827 | TGGGTTCATTACATGATTCATGAGCCAGAACCACATATTCTTCTCTTTAGACGACCTCTTCCAAAAGATCAACAAAAATG | Sequence number 400 |
| MKI67 | NM_002417 | AGCCTGCAGCAAGCACTTTGGAGAGCAAATCTGTGCAGGAGATGTAACGCGGAGTGTCAAGAGGTGTGCAGAAAATCCAAAG | Sequence number 401 |
| FOXM1 | NM_021953 | GAAGGCCTGGTCCTGGACACAATGAATGACAGCCTCAGCAAGATCCTGCTGGACATCAGCTTTCCTGGCCTGGACGAGGA | Sequence number 402 |
| UBE2T | NM_014176 | GACAGAGAAGCGTGCCAAGACAGAACAAAAGGCTGATGAGGAAGAGACTCCTTGATAATCTACCAGAGGCTGGTGACTCCGA | Sequence number 403 |
| MCM4 | NM_182746 | GAATAGGTTCTGCATACCCTGACAGCGTAGAGTCATTAATCCGCTTAGCCAGAAGCCCATGCTAAAGGTAAGATTGTCTAAC | Sequence number 404 |
| CKAP2 | NM_018204 | TAAGAGATCATTATCCTTGTGTGTCTTCATTGGAACAGCTAACCGAGCTGGGAAGAGAAACTGATGCTTTTGTATGCCGC | Sequence number 405 |
| HN1 | NM_016185 | GGACTGCAGAGAAGGAACTCCTCTGAAGCAAGCTCCGGAGACTTCTTAGATCTGAAGGGGAGAAGGTGATATTCATGAAAA | Sequence number 406 |
| KPNA2 | NM_002266 | CTGTAGAGGAAGAGGAAGATCAAAACGTTGTACCAGAAACTACCTCTGAAAGGGTACACTTTGCAAGTTCAGGATGGGGCT | Sequence number 407 |
| H2AFX | NM_002105 | GGCTTCTGAACTGGAATTCTGCAGCTAACCCTTCCACGACTAGAAACCTTAGGCATTGGGGAGTTTTAGATGGACTAATTT | Sequence number 408 |
| H2AFZ | NM_002106 | ATCTCAGGACTCTAAATACTCTAACACTGGTCCAGTGGTTGGTGATTCCAGTGGACTGTATCTCTGTGAAAAACACAATTT | Sequence number 409 |
| CDK1 | NM_001786 | CATTTGGAGTATAGGCACCATATTTGCTGAACTAGCAACTAAGAAACCACTTTTCCATGGGGATTCAGAAATTGATCAAC | Sequence number 410 |
| PTTG1 | NM_004219 | TCCAAATCTGTTCCAGTCTCCTTCAAGACTCTGTGAACCGTGATGTTGAATTGCCACCTGTTTGCTGCTAAGGTCTGTGAACATACAGTAT | Sequence number 411 |
| CDC20 | NM_001255 | CGCCCACCAAGAACGAACATCAGAAAGCCTGGGCTTTGAACCTGAACGGTTTTGATGTGAGGAAGCCAAGATCCTTCGG | Sequence number 412 |
| MYBL2 | NM_002466 | GAGGAAGACCAAAAGTCATCGAGCTCGGTTAAGAAGTATGCGACAAAGCAGTGGACACTGATTGCCAAGCACCTGAAGGG | Sequence number 413 |
| RRM2 | NM_001034 | TTTGTGGCGACAGACTTATGCTGGAACTGGGTTTTAGCAAGGTTTTCAGAGTAGAGAACCCATTTGACTTTATGGAGAA | Sequence number 414 |

(Example 4. Cluster analysis using differentiation marker gene set for breast cancer - 1)

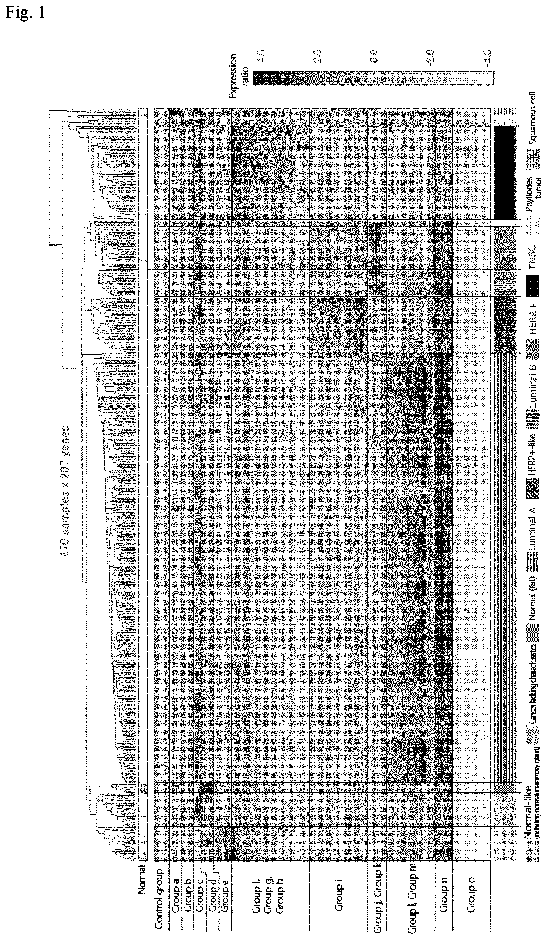

Using the set of the 207 kinds of genes selected in Example 3 as the differentiation marker gene set, the gene expression level of each gene was measured (data not shown), and cluster analysis was performed. Further, cluster analysis was performed by the group-average method based on Euclidean distance using Expression View Pro software (MicroDiagnostic). The results of cluster analysis are shown in Fig. 1.As shown in Fig. 1, when hierarchical cluster analysis was performed on the basis of the expression profiles of the extracted 207 genes, the genes could be classified into the clusters of a normal-like group, an undeterminable group, a normal group, a luminal A group, a HER2+-like group, a luminal B group, a HER2+ group, a triple negative group, and an other group.

(Example 5. Scoring by differentiation marker gene set)

ROC analysis of each gene was performed for the differentiation marker gene set including the 207 kinds of genes selected in Example 3 to determine cutoff values. It should be noted that the cutoff value was appropriately determined for each gene group as a value at which sensitivity = specificity. The details of the ROC analysis are as follows.

When a cluster analysis of 470 cases was performed using eight genes belonging to the group a, the cases were classified into two clusters. A cluster including five cases with a high marker expression, including cases clinically diagnosed as squamous cell carcinoma, was defined as "squamous cell carcinoma," and a cluster including 465 cases with a low expression was defined as "non-squamous cell carcinoma." ROC analysis was conducted for each of the eight genes in the 470 cases, with "squamous cell carcinoma" as the discriminant patient group and "non-squamous cell carcinoma" as the control patient group, and the cutoff value was calculated.

When a cluster analysis of 470 cases was performed using eight genes belonging to the group b, the cases were classified into two clusters. A cluster including three cases with a high marker expression, including cases clinically diagnosed as malignant phyllodes tumor, was defined as "phyllodes tumor," and a cluster including 467 cases with a low expression was defined as "non-phyllodes tumor." ROC analysis was conducted for each of the eight genes in the 470 cases, with "phyllodes tumor" as the discriminant patient group and "non-phyllodes tumor" as the control patient group, and the cutoff value was calculated.

Normal tissue was defined as "non-cancer," and other tissue was defined as "cancer." ROC analysis was conducted for each of four genes belonging to the group c in the 470 cases, with "cancer" as the discriminant patient group and "non-cancer" as the control patient group, and the cutoff value was calculated.

Normal tissue was defined as "normal," and other tissue was defined as "non-normal." However, the normal-like group that resembles normal and the group lacking characteristics were excluded from "normal" and "non-normal." ROC analysis was conducted for each of ten genes belonging to the group d in 435 cases, with "normal" as the discriminant patient group and "non-normal" as the control patient group, and the cutoff value was calculated.

In the cluster analysis of 207 genes x 470 cases, cases included in the cluster of normal-like (including normal tissue) were defined as "normal-like," and cases included in the cluster of luminal A, luminal B, HER2 amplification+, HER2-like, and triple negative were defined as "non-normal-like." ROC analysis was conducted for each of nine genes belonging to the group e in 428 cases, with "normal" as the discriminant patient group and "non-normal" as the control patient group, and the cutoff value was calculated.

In the cluster analysis of 207 genes x 470 cases, cases included in the cluster of the triple negative group were defined as "TNBC," and cases included in the cluster of luminal A, luminal B, HER2 amplification+, and HER2-like were defined as "non-TNBC." ROC analysis was conducted for each of 48 genes belonging to the group f, the group g, or the group h in the 407 cases, with "TNBC" as the discriminant patient group and "non-TNBC" as the control patient group, and the cutoff value was calculated.

In the cluster analysis of 207 genes x 470 cases, cases included in the cluster of HER2-like and HER2 amplification+ were defined as "HER2-like," and cases included in the cluster of luminal A, luminal B, and triple negative were defined as "non-HER2-like." ROC analysis was conducted for each of 37 genes belonging to the group i in 407 cases, with "HER2-like" as the discriminant patient group and "non-HER2-like" as the control patient group, and the cutoff value was calculated.

In the cluster analysis of 207 genes x 470 cases, 44 cases included in the cluster of HER2 amplification+ and luminal B were subjected to cluster analysis with 11 genes belonging to the group j or the group k. With each amplification region being different and the clusters being divided according to the range, the cases were divided into "amplification" and "no amplification" for each amplification region (29 cases of "amplification" in all 11 genes; three cases of "amplification" in eight genes; three cases of "amplification" in seven genes; and nine cases of "amplification" in five genes). Cases included in the cluster of the luminal A group, the HER2-like group, and the triple negative group were defined as "no amplification." ROC analysis was conducted for each of the 11 genes in 407 cases, with "amplification" as the discriminant patient group and "no amplification" as the control patient group, and the cutoff value was calculated.

In the cluster analysis of 207 genes x 470 cases, cases included in the cluster of the luminal A group and the luminal B group were defined as "hormone sensitivity," and cases included in the cluster of the HER2 amplification+ group, the HER2-like group, and the triple negative group were defined as "no hormone sensitivity." ROC analysis was conducted for each of 30 genes included in the group 1 or the group m in 407 cases, with "hormone sensitivity" as the discriminant patient group and "no hormone sensitivity" as the control patient group, and the cutoff value was calculated.

In the cluster analysis of 207 genes x 470 cases, cases included in the cluster of the luminal A group, the luminal B group, the HER2 amplification+ group, and the HER2-like group were defined as "differentiated," and cases included in the cluster of triple negative were defined as "undifferentiated." ROC analysis was conducted for each of 11 genes belonging to the group n in the 407 cases, with "differentiated" as the discriminant patient group and "undifferentiated" as the control patient group, and the cutoff value was calculated.

When a cluster analysis of 470 cases was performed using 23 genes belonging to the group o, the cases were classified into two clusters. A cluster with a high marker expression, including many cases clinically diagnosed as triple negative, was defined as "fast-growth group," and a cluster with a low expression was defined as "slow-growth group." ROC analysis was conducted for each of the 23 genes in 470 cases, with "fast-growth group" as the discriminant patient group and "slow-growth group" as the control patient group, and the cutoff value was calculated.

For each case, given 1 when the expression ratio was larger than the cutoff value, -1 when the expression ratio was smaller than the cutoff value, and 0 when the data was 0, the average value was calculated for each gene group of the groups a to o. However, for one gene (MBOAT1) belonging to the group i and two genes (PADI2 and PPP1R1B) belonging to the group 1, the appearance of the characteristics thereof increases as the expression ratio decreases, which is the reverse of the other genes, and therefore the average value was calculated given 1 when the expression ratio was smaller than the cutoff value, -1 when the expression ratio was larger than the cutoff value, and 0 when the data was 0.

The subtype differentiation scores (cancer score, cell cycle score, squamous cell score, phyllodes tumor score, normal-like score, triple negative score, HER2-like score, HER2 amplification score, and hormone sensitivity score) were calculated using the average value of each gene group obtained in each case. Specifically, each subtype differentiation score was calculated as follows. The maximum value of each subtype differentiation score was 1, the minimum value was -1, and a higher score indicates a higher likelihood.

$$\text{Cancer score} = (c \times 2.5 - d \times 5.5) \div 8$$

Cell cycle score = o
Squamous cell score = a
Phyllodes tumor score = b
Normal-like score = e

$$\text{Triple negative score} = (f \times 2.6 + g \times 11.4 + h \times 2.4 - n \times 6.8) \div 23.2$$

HER2-like score = i

$$\text{HER2 amplification score} = (j \times 3.8 + k \times 0.7) \div 4.5$$

$$\text{Hormone sensitivity score} = (l \times 5.3 + m \times 0.4) \div 5.7$$

It should be noted that, in this example, because a plurality of gene groups (groups a to o) are used for calculation, the regression coefficients calculated by multiple logistic regression analysis were respectively multiplied by the average value of each gene group, and then that value was divided by the sum of the regression coefficients so that the maximum value was 1 and the minimum value was -1. 470 cases were used for each, and the details are as follows: For "cancer," the objective variable was set to 0 for normal tissue and to 1 for non-normal tissue, and the scores of the "cancer" and "normal" gene groups were used as explanatory variables. For "triple negative," the objective variable was set to 1 for cases included in the cluster of triple negative by the cluster analysis of 207 genes x 470 cases and to 0 for all others, and the scores of "TNBC1," "TNBC2," and "TNBC3" were used as explanatory variables. For "HER2 amplification," the objective variable was set to 1 for cases included in the HER2+ amplification and luminal B by the cluster analysis of 207 genes x 470 cases and to 0 for all others, and the scores of "HER2 amplification 1" and "HER2 amplification 2" were used as explanatory variables. For "hormone sensitivity," the objective variable was set to 1 for cases included in the luminal A and luminal B by the cluster analysis of 207 genes x 470 cases and to 0 for all others, and the scores of "hormone sensitivity" and "ESR1" were used as explanatory variables.

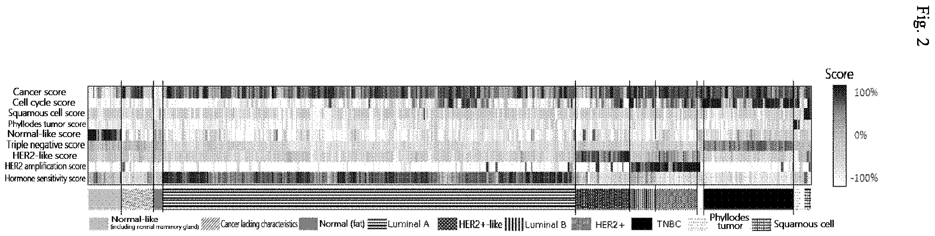

By the above-described equations, it was possible to score each calculated subtype differentiation score for the 470 cases. The scored results are shown in Fig. 2 (the vertical axis of the heat map in Fig. 2 indicates the subtype differentiation score, and the horizontal axis indicates the samples derived from 470 cases. Further, in Fig. 2, the arrangement of the samples derived from 470 cases on the horizontal axis shows the same arrangement as in the heat map in Fig. 1, and the subtype classification shown in the lower section of Fig. 2 shows the cluster analysis results in Fig. 1).

(Example 6. Histological type differentiation of breast cancer by subtype differentiation score - 1)

On the basis of the subtype differentiation score obtained in Example 5, the histological type of breast cancer was differentiated as follows.

(1) The highest score of the scores of "triple negative," "HER2 amplification," "hormone sensitivity," "HER2+-like," "phyllodes tumor," and "normal-like" was found.
(2) If "triple negative" had the highest score of the six scores and the score was higher than 0.2, the type was determined to be "TNBC."
(3) If "HER2 amplification" had the highest score of the six scores and the score was higher than 0.2, and the score of "hormone sensitivity" was higher than 0, the type was determined to be "luminal B-HER2+." If the score of "HER2 amplification" was the highest and higher than 0.2, and the score of "hormone sensitivity" was 0 or lower, the type was determined to be "HER2+."
(4) If "hormone sensitivity" had the highest score of the six scores and the score was higher than 0, and the score of "HER2 amplification" was higher than 0.2, the type was determined to be "luminal B-HER2+." If the score of "hormone sensitivity" was the highest and higher than 0, and the score of "HER2 amplification" was 0.2 or lower, the type was determined to be "luminal A (provisional)." Among "luminal A (provisional)," if the score of "cell cycle" was higher than 0, the type was determined to be "luminal B-HER2-," and if the score of "cell cycle" was 0 or lower, the type was determined to be "luminal A."
(5) If the score of "HER2+-like" had the highest score of the six scores and the score was higher than 0.2, and the score of "HER2 amplification" was higher than 0.2, the type was determined to be "HER2+." If the score of "HER2+-like" was the highest and higher than 0.2, and the score of "HER2 amplification" was 0.2 or lower, the type was determined to be "HER2+-like."
(6) If "phyllodes tumor" had the highest score of the six scores and the score was higher than 0.1, the type was determined to be "phyllodes tumor."
(7) If "normal-like" had the highest score of the six scores and the score was higher than 0.1, the type was determined to be "normal-like."
(8) Among the cases that did not belong to any of the above types based on each determination up to (7), for a case in which both the "cancer" and "cell cycle" scores were 0 or lower, the type was determined to be "normal-like."
(9) For a case that did not belong to any of the above types based on each determination up to (8), the type was determined to be "undeterminable."
(10) For a case in which the score of "squamous cell carcinoma" was 0.2 or higher, "squamous cell carcinoma" was also added to the subtype determination described above.

(Example 7. Cluster analysis using differentiation marker gene set for breast cancer - 2)

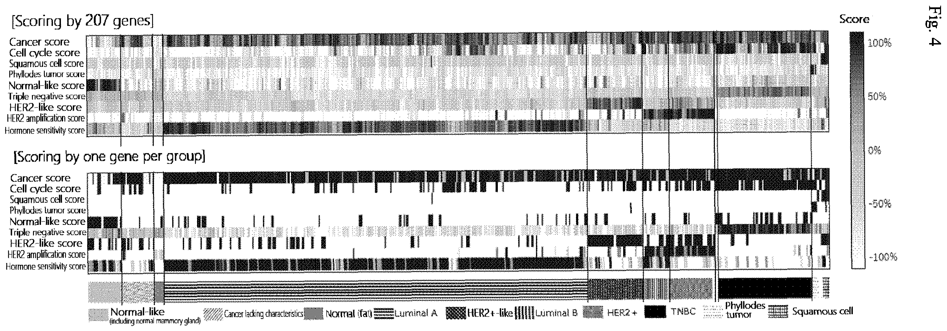

In this example, a gene group obtained by selecting one gene from each of the 15 differentiating gene groups of the groups a to o selected in Example 3 was used as the differentiation marker gene set and, for the 470 cases with clear breast cancer subtypes, the expression levels of the 15 genes were measured (data not shown) and cluster analysis was conducted. Specifically, the genes of SPRR2A from the group a, SERPINH1 from the group b, FN1 from the group c, CAVIN2 from the group d, KRT15 from the group e, GABRP from the group f, EN1 from the group g, LYZ from the group f, CLCA2 from the group i, GRB7 from the group j, ORMDL3 from the group k, CYP2B6 from the group 1, ESR1 from the group m, FOXA1 from the group n, and CDC20 from the group o were each selected, and the subtype differentiation scores were calculated. Further, the cluster analysis was performed using Expression View Pro software (MicroDiagnostic) by the group-average method based on Euclidean distance, similar to Example 4. The results of cluster analysis are shown in Fig. 4. As shown in Fig. 4, when hierarchical cluster analysis was performed on the basis of the expression profiles of the genes obtained by selecting one gene from each of the 15 differentiating gene groups of the groups a to o, the genes could be classified into the cluster of a normal-like group, an undeterminable group, a normal group, a luminal A group, a HER2+-like group, a luminal B group, a HER2+ group, a triple negative group, and an other group.

(Example 8. Histological type differentiation of breast cancer by subtype differentiation score - 2)

Fig. 4 shows the results of calculating each subtype differentiation score from the expression level of each gene in the 470 cases with clear breast cancer subtypes by the same method as in Example 5 by using the differentiation marker gene set used in Example 7. It should be noted that the calculation of the scores was performed as follows.
The subtype differentiation scores (cancer score, cell cycle score, squamous cell score, phyllodes tumor score, normal-like score, triple negative score, HER2-like score, HER2 amplification score, and hormone sensitivity score) were calculated using the average value of each gene group obtained in each case. Specifically, each subtype differentiation score was calculated as follows. The maximum value of each subtype differentiation score was 1, the minimum value was -1, and a higher score indicates a higher likelihood.

$$\text{Cancer score} = (c \times 1.3 - d \times 27.4) \div 28.7$$

Cell cycle score = o
Squamous cell score = a
Phyllodes tumor score = b
Normal-like score = e

$$\text{Triple negative score} = (f \times 3.0 + g \times 1.2 + h \times 0.7 - n \times 2.1) \div 7$$

HER2-like score = i

$$\text{HER2 amplification score} = (j \times 2.5 + k \times 1) \div 3.5$$

$$\text{Hormone sensitivity score} = (l \times 1.1 + m \times 1.9) \div 3$$

Fig. 4 shows the scoring heat map (Fig. 2) obtained in Example 5 for comparison and, as shown in Fig. 4, even in a case in which one gene was selected from each of the 15 differentiating gene groups of the groups a to o to form the differentiation marker gene set, the subtype of breast cancer could be differentiated or classified by calculating the subtype differentiation score.

(Example 9. Cluster analysis using differentiation marker gene set for breast cancer - 3)

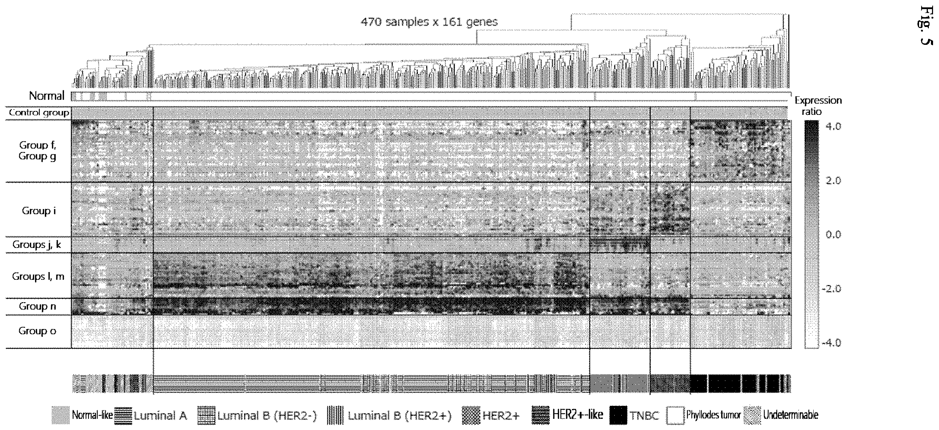

The gene expression level of each gene was measured using a set of 153 kinds of genes belonging to the group f, the group g, the group i, the group j, the group k, the group 1, the group m, the group n, and the group o as the differentiation marker gene set (data not shown), gene expression levels of each gene (eight kinds) in the control group were measured (data not shown), and cluster analysis was conducted upon combining these. Further, cluster analysis was performed by the group-average method based on Euclidean distance using Expression View Pro software (Micro-Diagnostic). The results of cluster analysis are shown in Fig. 5.As shown in Fig. 5, when hierarchical cluster analysis was performed on the basis of the expression profiles of the extracted 161 genes, the genes could be classified into the cluster of a luminal A and B group, a HER2+-like group, a HER2+ group, a triple negative group, and an other group.

(Example 10. Histological type differentiation of breast cancer by subtype differentiation score - 3)

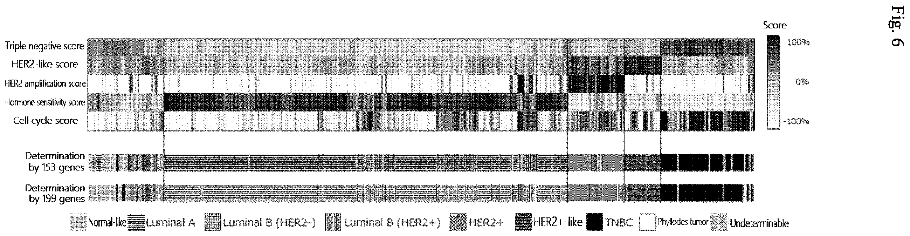

Fig. 6 shows the results of calculating each subtype differentiation score from the expression level of each gene in the 470 cases with clear breast cancer subtypes by the same method as in Example 5 by using the differentiation marker gene set used in Example 9. Each subtype differentiation score was calculated as follows. It should be noted that the calculation of the scores was performed as follows.
The subtype differentiation scores (cancer score, cell cycle score, squamous cell score, phyllodes tumor score, normal-like score, triple negative score, HER2-like score, HER2 amplification score, and hormone sensitivity score) were calculated using the average value of each gene group obtained in each case. Specifically, each subtype differentiation score was calculated as follows. The maximum value of each subtype differentiation score was 1, the minimum value was -1, and a higher score indicates a higher likelihood.
Cell cycle score = o

$$\text{Triple negative score} = (f \times 1.5 + g \times 7.1 - n \times 5.9) \div 14.5$$

HER2+-like score = i

$$\text{HER2 amplification score} = (j \times 6.5 + k \times 1.8) \div 8.3$$

$$\text{Hormone sensitivity score} = (l \times 7.20 + m \times 0.15) \div 7.35$$

On the basis of the obtained subtype differentiation scores, the histological type of breast cancer was differentiated as follows.

(1) The highest score of the scores of "triple negative score," "HER2+-like score," "HER2 amplification score," and "hormone sensitivity score" was found.

(2) Among cases having a "triple negative score" that was the highest score of the four scores and a score higher than 0.2, a case with a "cell cycle score" higher than -0.6 was determined to be "triple negative" and a case with a "cell cycle score" of -0.6 or lower was determined to be "normal."

(3) Among cases having an "HER2 amplification score" that was the highest score of the four scores and a score higher than 0.2, a case with a "hormone sensitivity score" higher than 0 was determined to be "luminal B-HER2+" and a case with a "hormone sensitivity score" of 0 or lower was determined to be "HER2+."

(4) Among cases having a "hormone sensitivity score" that was the highest score of the four scores and a score higher than 0, a case with a "HER2 amplification score" higher than 0.2 was determined to be "luminal B-HER2+." In addition, among cases having a "hormone sensitivity score" that was the highest score and a score higher than 0, and further having a "HER2 amplification score" of 0.2 or lower, a case with a "cell cycle score" higher than 0 was determined to be "luminal B-HER2-" and a case with a "cell cycle score" of 0 or lower was determined to be "luminal A."

(5) A case having an "HER2+-like score" that was the highest score of the four scores and a score higher than 0.2, and further having a "HER2 amplification score" higher than 0.2 was determined to be "HER2+."

(6) A case having a "HER2+-like score" that was the highest score of the four scores and a score of 0.2 or higher, and further having a "HER2 amplification score" of 0.2 or lower was determined to be "HER2+-like."

(7) A case that did not belong to any of the above types based on each determination up to (6) was determined to be "undeterminable group." As a result, breast cancer subtypes could be differentiated or classified into the luminal A and B group, the HER2+-like group, the HER2+ group, the triple negative group, and the undeterminable group. It should be noted that, when the results were compared with the results of each subtype differentiation score calculated in the same manner by using the differentiation marker gene set of 199 genes included in the groups a to o, the differentiation or classification of the subtypes of breast cancer into the luminal A and B group, the HER2+-like group, the HER2+ group, the triple negative group, or the undeterminable group was of similar accuracy.

Further, the histological type of breast cancer was differentiated using the subtype differentiation score, making it possible to further distinguish, among the luminal A and B group, the luminal A group, the luminal B group (HER2 positive), and the luminal B group (HER2 negative).

[Sequence table] MDCP1801 Seq listing_190304_ST25.txt

**Claims**

1.   A method of differentiating or classifying a subtype of breast cancer in a test sample, the method comprising:

(a) a step of measuring, in the test sample, expression levels of genes included in a differentiation marker gene set for differentiating or classifying a subtype of breast cancer; and
(b) a step of differentiating or classifying whether the test sample is a desired subtype to be differentiated or classified from the expression levels of the genes included in the differentiation marker gene set thus measured,

the differentiation marker gene set including a combination of genes obtained by selecting at least one gene from each gene group of at least one gene group selected from gene groups composed of groups a to o shown in the tables below, and
the at least one gene group being selected in accordance with the desired subtype to be differentiated or classified.

[Table 1A]

| Gene group | Gene symbol | Gene group | Gene symbol | Gene group | Gene symbol |
|---|---|---|---|---|---|
| Group a | KRTDAP | Group e | KRT14 | Group g | ART3 |
| | SERPINB3 | | DST | | EN1 |
| | SPRR2A | | WIF1 | | SPHK1 |
| | SPRR1B | | SYNM | | TRIM47 |
| | KLK13 | | KIT | | COL27A1 |
| | KRT1 | Group f | GABRP | | RFLNA |
| | LGALS7 | | SFRP1 | | RASD2 |
| | PI3 | | ELF5 | | A2ML1 |
| Group b | SERPINH1 | | MIA | | MARCO |
| | SNAI2 | | MMP7 | | TSPYL5 |
| | GPR173 | | FDCSP | | TM4SF1 |
| | HAS2 | Group g | CRABP1 | | FABP5 |
| | PTH1R | | PROM1 | Group h | SPIB |
| | PAGES | | KRT23 | | BCL2A1 |
| | ITLN1 | | S100A1 | | MZB1 |
| | SH3PXD2B | | WIPF3 | | KCNK5 |
| Group c | TAP1 | | CYYR1 | | LM04 |
| | FN1 | | TFCP2L1 | | RNF150 |
| | CTHRC1 | | DSC2 | | LYZ |
| | MMP9 | | MFGE8 | Group i | C21orf58 |
| Group d | ADIPOQ | | KLK7 | | ATP13A5 |
| | CD36 | | KLK5 | | NUDT8 |
| | GOS2 | | DSG3 | | HSD17B2 |
| | GPD1 | | TTYH1 | | ABCA12 |
| | LEP | | SCRG1 | | ENPP3 |
| | LIPE | | S100B | | WNT5A |
| | PLIN1 | | ETV6 | | MPP3 |
| | CAVIN2 | | OGFRL1 | | VPS13D |
| | LIFR | | MELTF | | PXMP4 |
| | TGFBR3 | | HORMAD1 | | GGT1 |
| e群 | CAPN6 | | PKP1 | | TRPV6 |
| | PIGR | | FOXC1 | | MAB21L4 |
| | KRT15 | | ITGB8 | | CLDN8 |
| | KRT5 | | VGLL1 | | LBP |

[Table 1B]

| Gene group | Gene symbol | Gene group | Gene symbol | Gene group | Gene symbol |
|---|---|---|---|---|---|
| Group i | SRD5A3 | Group l | THSD4 | Group n | GATA3 |
| | PAPSS2 | | MAPT | | ZG16B |
| | TMEM45B | | LONRF2 | | KIAA0040 |
| | CLCA2 | | TCEAL3 | | TMC4 |
| | FASN | | DBNDD2 | | AGR2 |
| | MPHOSPH6 | | FGD3 | | TFF3 |
| | NXPH4 | | GFRA1 | | SCGB2A2 |
| | HPGD | | PARD6B | | MUCL1 |
| | KYNU | | STC2 | Group o | DDX11 |
| | GLYATL2 | | SLC39A6 | | ATAD2 |
| | KMO | | ENPP5 | | GGH |
| | SRPK3 | | ZNF703 | | CDCA3 |
| | THRSP | | EVL | | CCNA2 |
| | PLA2G2A | | TBC1D9 | | CCNB2 |
| | TFAP2B | | CHAD | | ANLN |
| | FABP7 | | GREB1 | | UBE2C |
| | SLPI | | HPN | | CKS2 |
| | SERHL2 | | IL6ST | | MKI67 |
| | S100A9 | | GASK1B | | FOXM1 |
| | KRT7 | | CA12 | | UBE2T |
| | TMEM86A | | KCNE4 | | MCM4 |
| | MBOAT1 | | NAT1 | | CKAP2 |
| Group j | PGAP3 | | CYP2B6 (CYP2B7P) | | JPT1 |
| | STARD3 | | ARMT1 | | KPNA2 |
| | ERBB2 | | MAGED2 | | H2AFX |
| | MIEN1 | | CELSR1 | | H2AFZ |
| | GRB7 | | INPP5J | | CDK1 |
| Group k | GSDMB | | PADI2 | | PTTG1 |
| | ORMDL3 | | PPP1R1B | | CDC20 |
| | MED24 | Group m | ESR1 | | MYBL2 |
| | MSL1 | Group n | MLPH | | RRM2 |
| | CASC3 | | FOXA1 | | |
| | WIPF2 | | XBP1 | | |

2. The method of differentiating or classifying according to claim 1, wherein
the step (b) is a step of differentiating or classifying a subtype of the test sample by acquiring an expression profile of the differentiation marker gene set from the expression levels of the genes thus measured, and comparing the expression profile thus acquired and an expression profile of a corresponding differentiation marker gene set in a sample derived from a breast cancer patient having the desired subtype to be differentiated or classified.

3. The method of differentiating or classifying according to claim 2, wherein
in the step (b), the expression profile thus acquired and the expression profile of a corresponding differentiation marker gene set in the sample derived from a breast cancer patient having the desired subtype to be differentiated or classified are compared, and
the test sample is evaluated as being breast cancer of the subtype thus compared when having an expression profile equivalent to the expression profile of the sample thus compared, or is evaluated as not being breast cancer of the subtype thus compared when having an expression profile of genes different from the expression profile of the sample thus compared.

**4.** The method of differentiating or classifying according to claim 2, wherein
comparison with the expression profile of the corresponding differentiation marker gene set in the sample derived from a breast cancer patient having the desired subtype to be differentiated or classified in the step (b) is performed by cluster analysis.

**5.** The method of differentiating or classifying according to claim 2, wherein
in the step (b), the differentiating or classifying is performed by comparing the expression profile thus acquired with a predetermined threshold value.

**6.** The method of differentiating or classifying according to claim 2, wherein
the step (b) is a step of differentiating or classifying whether the test sample is the desired subtype to be differentiated by calculating a subtype differentiation score from the expression levels of the genes included in the gene set thus measured.

**7.** The method of differentiating or classifying according to claim 6, wherein
the subtype differentiation score in the step (b) is determined on the basis of the expression levels of genes included in each gene group selected in accordance with the desired subtype to be differentiated, or an average value thereof.

**8.** The method of differentiating or classifying according to claim 1, wherein
the desired subtype is a subtype selected from a group composed of luminal A, luminal B (HER2 positive), luminal B (HER2 negative), HER2 positive, HER2 positive-like, triple negative, phyllodes tumor, squamous cell carcinoma, normal-like, normal, and undeterminable.

**9.** The method of differentiating or classifying according to claim 1, wherein
the at least one gene group selected in accordance with the desired subtype to be differentiated in the step (a) is

(i) the group 1 and the group m for calculating a hormone sensitivity score, the group o for calculating a cell cycle score, and the group j and the group k for calculating a HER2 amplification score when the desired subtype is luminal A,
(ii) the group j and the group k for calculating the HER2 amplification score, and the group 1 and the group m for calculating the hormone sensitivity score when the desired subtype is luminal B (HER2 positive),
(iii) the group j and the group k for calculating the HER2 amplification score, the group 1 and the group m for calculating the hormone sensitivity score, and the group o for calculating the cell cycle score when the desired subtype is luminal B (HER2 negative),
(iv) the group j and the group k for calculating the HER2 amplification score, the group 1 and the group m for calculating the hormone sensitivity score, or the group i for calculating a HER2-like score when the desired subtype is HER2 positive,
(v) the group i for calculating the HER2-like score, and the group j and the group k for calculating the HER2 amplification score when the desired subtype is HER2 positive-like,
(vi) the group f, the group g, the group h, and the group n for calculating a triple negative score when the desired subtype is triple negative,
(vii) the group b for calculating a phyllodes tumor score when the desired subtype is phyllodes tumor,
(viii) the group a for calculating a squamous cell score when the desired subtype is squamous cell carcinoma,
(ix) the group a to the group o for calculating a cancer score and all other scores when the desired subtype is undeterminable,
(x) the group e for calculating a normal-like score, the group o for calculating the cell cycle score, and the group c and the group d for calculating the cancer score when the desired subtype is normal-like, or
(xi) the group c and the group d for calculating the cancer score when the desired subtype is normal.

**10.** The method of differentiating or classifying according to claim 1, wherein

the desired subtypes are luminal A and B, HER2 positive-like, HER2 positive, and triple negative, and
the differentiation marker gene set includes a combination of genes obtained by selecting at least one gene from each gene group of the group f, the group g, the group i, the group j, the group k, the group 1, the group m, the group n, and the group o.

**11.** The method of differentiating or classifying according to claim 1, wherein
the differentiation marker gene set includes all genes included in each gene group of a plurality of the gene groups thus selected.

12. The method of differentiating or classifying according to claim 1, wherein
the differentiation marker gene set further includes at least one gene selected from a control group composed of ABCF3, FBXW5, MLLT1, FAM234A, PITPNM1, WDR1, NDUFS7, and AP2A1.

13. A differentiation marker gene set for differentiating or classifying a subtype of breast cancer, the differentiation marker gene set comprising:

a combination of genes obtained by selecting at least one gene from each gene group of at least one gene group selected from gene groups composed of groups a to o shown in the tables below,
the at least one gene group being selected in accordance with a desired subtype to be differentiated or classified.

[Table 2A]

| Gene group | Gene symbol | Gene group | Gene symbol | Gene group | Gene symbol |
|---|---|---|---|---|---|
| Group a | KRTLAP SERPINB3 SPRR2A SPRR1B KLK13 | Group e | KRT14 DST WIF1 SYNM KIT | Group g | ART3 EN1 SPHK1 TRIM47 COL27A1 |
| | KRT1 LGALS7 PI3 | Group f | GABRP SFRP1 ELF5 | | RFLNA RASD2 A2ML1 |
| Group b | SERPINH1 SNAI2 GPR173 | | MIA MMP7 FDCSP | | MARCO TSPYL5 TM4SF1 |
| | HAS2 PTH1R PAGES ITLN1 SH3PXD2B | Group g | CRABP1 PROM1 KRT23 S100A1 WIPF3 CYYR1 TFCP2L1 DSC2 | | FABP5 |
| Group c | TAP1 FN1 CTHRC1 MMP9 | | | Group h | SPIB BCL2A1 MZB1 KCNK5 LM04 RNF150 LYZ |
| Group d | ADIPOQ CD36 GOS2 GPD1 LEP LIPE PLIN1 CAVIN2 LIFR TGFBR3 | | MFGE8 KLK7 KLK5 DSG3 TTYH1 SCRGI S100B ETV6 OGFRL1 MELTF HORMAD1 | Group i | C21orf58 ATP13A5 NUDT8 HSD17B2 ABCA12 ENPP3 WNT5A MPP3 VPS13D PXMP4 GGT1 |
| Group e | CAPN6 PIGR KRT15 KRT5 | | PKP1 FOXC1 ITGB8 VGLLI | | TRPV6 MAB21L4 CLDN8 LBP |

[Table 2B]

| Gene group | Gene symbol | Gene group | Gene symbol | Gene group | Gene symbol |
|---|---|---|---|---|---|
| Group i | SRD5A3 | Group l | THSD4 | Group n | GATA3 |
| | PAPSS2 | | MAPT | | ZG16B |
| | TMEM45B | | LONRF2 | | KIAA0040 |
| | CLCA2 | | TCEAL3 | | TMC4 |
| | FASN | | DBNDD2 | | AGR2 |
| | MPHOSPH6 | | FGD3 | | TFF3 |
| | NXPH4 | | GFRA1 | | SCGB2A2 |
| | HPGD | | PARD6B | | MUCL1 |
| | KYNU | | STC2 | Group o | DDX11 |
| | GLYATL2 | | SLC39A6 | | ATAD2 |
| | KMO | | ENPP5 | | GGH |
| | SRPK3 | | ZNF703 | | CDCA3 |
| | THRSP | | EVL | | CCNA2 |
| | PLA2G2A | | TBC1D9 | | CCNB2 |
| | TFAP2B | | CHAD | | ANLN |
| | FABP7 | | GREB1 | | UBE2C |
| | SLPI | | HPN | | CKS2 |
| | SERHL2 | | IL6ST | | YKI67 |
| | S100A9 | | GASK1B | | FOXM1 |
| | KRT7 | | CA12 | | UBE2T |
| | TMEM86A | | KCNE4 | | MCM4 |
| | MBOAT1 | | NAT1 | | CKAP2 |
| Group j | PGAP3 | | CYP2B6 (CYP2B7P) | | JPT1 |
| | STARD3 | | ARMT1 | | KPNA2 |
| | ERBB2 | | MAGED2 | | H2AFX |
| | MIEN1 | | CELSR1 | | H2AFZ |
| | GRB7 | | INPP5J | | CDK1 |
| Group k | GSDMB | | PADI2 | | PTTG1 |
| | ORMDL3 | | PPP1R1B | | CDC20 |
| | MED24 | Group m | ESR1 | | MYBL2 |
| | MSL1 | Group n | MLPH | | RRM2 |
| | CASC3 | | FOXA1 | | |
| | WIPF2 | | XBP1 | | |

14. The differentiation marker gene set according to claim 13, wherein
the desired subtype is a subtype selected from a group composed of luminal A, luminal B (HER2 positive), luminal B (HER2 negative), HER2 positive, HER2 positive-like, triple negative, phyllodes tumor, squamous cell carcinoma, normal-like, normal, and undeterminable.

15. The differentiation marker gene set according to claim 13, wherein
the at least one gene group is

(i) the group 1 and the group m for calculating a hormone sensitivity score, the group o for calculating a cell cycle score, and the group j and the group k for calculating a HER2 amplification score when the desired subtype is luminal A,
(ii) the group j and the group k for calculating the HER2 amplification score, and the group 1 and the group m for calculating the hormone sensitivity score when the desired subtype is luminal B (HER2 positive),

(iii) the group j and the group k for calculating the HER2 amplification score, the group 1 and the group m for calculating the hormone sensitivity score, and the group o for calculating the cell cycle score when the desired subtype is luminal B (HER2 negative),

(iv) the group j and the group k for calculating the HER2 amplification score, the group 1 and the group m for calculating the hormone sensitivity score, or the group i for calculating a HER2-like score when the desired subtype is HER2 positive,

(v) the group i for calculating a HER2-like score, and the group j and the group k for calculating the HER2 amplification score when the desired subtype is HER2 positive-like,

(vi) the group f, the group g, the group h, and the group n for calculating a triple negative score when the desired subtype is triple negative,

(vii) the group b for calculating a phyllodes tumor score when the desired subtype is phyllodes tumor,

(viii) the group a for calculating a squamous cell score when the desired subtype is squamous cell carcinoma,

(ix) the group a to the group o for calculating a cancer score and all other scores when the desired subtype is undeterminable,

(x) the group e for calculating a normal-like score, the group o for calculating the cell cycle score, and the group c and the group d for calculating the cancer score when the desired subtype is normal-like, or

(xi) the group c and the group d for calculating the cancer score when the desired subtype is normal.

16. The differentiation marker gene set according to claim 13, the differentiation marker gene set comprising:
a combination of genes obtained by selecting at least one gene from each gene group of nine gene groups composed of the group f, the group g, the group i, the group j, the group k, the group 1, the group m, the group n, and the group o.

17. The differentiation marker gene set according to claim 13, the differentiation marker gene set comprising:
a combination of genes obtained by selecting at least one gene from each gene group of 15 gene groups composed of the groups a to o.

18. The differentiation marker gene set according to claim 13, the differentiation marker gene set further comprising:
at least one gene selected from a control group composed of ABCF3, FBXW5, MLLT1, FAM234A, PITPNM1, WDR1, NDUFS7, and AP2A1.

19. A kit for differentiating or classifying a subtype of breast cancer in a test sample, the kit comprising:
means for measuring expression levels of genes included in the differentiation marker gene set for differentiating or classifying a subtype of breast cancer described in claim 13.

20. The kit according to claim 19, wherein
the means for measuring expression levels of genes is at least one means selected from a group composed of a primer or a probe for the genes or markers thereof.

21. The kit according to claim 20, wherein
the kit is for a PCR, a microarray, or an RNA sequence.

Fig. 1

Fig. 2

Fig. 3

EP 4 001 431 A1

52

Fig. 4

Fig. 5

Fig. 6

Score
100%
0%
-100%

Triple negative score
HER2-like score
HER2 amplification score
Hormone sensitivity score
Cell cycle score

Determination by 153 genes

Determination by 199 genes

Normal-like　Luminal A　Luminal B (HER2-)　Luminal B (HER2+)　HER2+　HER2+-like　TNBC　Phyllodes tumor　Undeterminable

## INTERNATIONAL SEARCH REPORT

International application No.

PCT/JP2020/027649

**A. CLASSIFICATION OF SUBJECT MATTER**
C12Q 1/6837(2018.01)i; C12Q 1/686(2018.01)i; C12Q 1/6876(2018.01)i
FI: C12Q1/6837 Z ZNA; C12Q1/686 Z; C12Q1/6876 Z
According to International Patent Classification (IPC) or to both national classification and IPC

**B. FIELDS SEARCHED**

Minimum documentation searched (classification system followed by classification symbols)
C12Q1/6837; C12Q1/686; C12Q1/6876

| Documentation searched other than minimum documentation to the extent that such documents are included in the fields searched | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2012-85555 A (WATANABE, Shinya) 10.05.2012 (2012-05-10) claims, examples | 1–9, 13–15, 19–21 |
| Y | | 12, 18 |
| A | | 10, 11, 16, 17 |
| X | JP 2012-85554 A (WATANABE, Shinya) 10.05.2012 (2012-05-10) claims, examples | 1–9, 13–15, 19–21 |
| Y | | 12, 18 |
| A | | 10, 11, 16, 17 |
| X | JP 2019-516754 A (INSTITUT NATIONAL DE LA SANTÉ ET DE LA RECHERCHE MÉDICALE) 20.06.2019 (2019-06-20) example 1 | 1–9, 12–15, 18–21 |
| Y | | 12, 18 |
| A | | 10, 11, 16, 17 |

☒ Further documents are listed in the continuation of Box C.    ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 17 September 2020 (17.09.2020) | 06 October 2020 (06.10.2020) |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

International application No.

PCT/JP2020/027649

C (Continuation).   DOCUMENTS CONSIDERED TO BE RELEVANT

| Category* | Citation of document, with indication, where appropriate, of the relevant passages | Relevant to claim No. |
|---|---|---|
| X | JP 2017-508469 A (THE COUNCIL OF THE QUEENSLAND INSTITUTE OF MEDICAL RESEARCH) 30.03.2017 (2017- | 1-9, 12-15, 18-21 |
| A | 03-30) claims, examples, tables 16-21 | 10, 11, 16, 17 |
| X | JP 2015-519043 A (IMMUNOVIA AB) 09.07.2015 (2015-07-09) claims, examples, tables 1-6 | 1-9, 12-15, 18-21 |
| A | | 10, 11, 16, 17 |
| X | JP 2012-525818 A (NATIONAL RESEARCH COUNCIL OF CANADA) 25.10.2012 (2012-10-25) claims, examples, | 1-9, 12-15, 18-21 |
| A | tables 1, 3 | 10, 11, 16, 17 |

Form PCT/ISA/210 (continuation of second sheet) (January 2015)

| International application No. |
| --- |
| PCT/JP2020/027649 |

| Patent Documents referred in the Report | Publication Date | Patent Family | Publication Date |
| --- | --- | --- | --- |
| JP 2012-85555 A | 10 May 2012 | (Family: none) | |
| JP 2012-85554 A | 10 May 2012 | (Family: none) | |
| JP 2019-516754 A | 20 Jun. 2019 | WO 2017/202949 A1 example 1 EP 3463433 A1 KR 10-2019-0032295 A CN 109689089 A | |
| JP 2017-508469 A | 30 Mar. 2017 | US 2017/0107577 A1 claims, examples, tables 16-21 WO 2015/135035 A2 EP 3119908 A2 KR 10-2016-0132067 A CN 106661614 A | |
| JP 2015-519043 A | 09 Jul. 2015 | US 2015/0057177 A1 claims, examples, tables 1-6 WO 2013/153524 A1 EP 2836838 A1 KR 10-2014-0143457 A CN 104508486 A | |
| JP 2012-525818 A | 25 Oct. 2012 | US 2012/0040863 A1 claims, examples, tables 1, 3 WO 2010/118520 A1 EP 2419533 A1 CN 102421920 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016013081 A

**Non-patent literature cited in the description**

- **PEROU CM et al.** *Nature,* 2000, vol. 406, 747-752
- **SORLIE T et al.** *Proc Natl Acad Sci,* 2001, vol. 98 (19), 10869-74
- **GREEN ; SAMBROOK.** Molecular Cloning. Cold Spring Harbor Laboratory Press, 2012
- **J. CHRISTOPHER LOVE et al.** Self-Assembled Monolayers of a Form of Nanotechnology. *Chemical Review,* 2005, vol. 105, 1103-1169
- **TOYOSAKA MORIIZUMI ; TAKAMICHI NAKAMO-TO.** Sensa Kougaku (Sensor Engineering). Shokodo Co., Ltd, 1997